# EUROPEAN PATENT SPECIFICATION

(11) **EP 1 235 847 B1**
(45) Date of publication and mention of the grant of the patent: **20.01.2016**
(21) Application number: 00983778.2
(22) Date of filing: 28.11.2000
(51) Int. Cl.: C07H 21/04, C07K 14/00, C07K 16/00, G01N 33/53, A61K 39/395, C07K 14/705

(54) **ISOLATION OF FIVE NOVEL GENES CODING FOR NEW Fc RECEPTORS-TYPE MELANOMA INVOLVED IN THE PATHOGENESIS OF LYMPHOMA/MELANOMA**
ISOLIERUNG VON FüNF GENEN, WELCHE FüR FC-REZEPTOREN DES MELANOM-TYPS KODIEREN, DIE IN DER LYMPHOM/MELANOM-PATHOGENESE EINE ROLLE SPIELEN
ISOLEMENT DE CINQ NOUVEAUX GENES CODANT POUR DES NOUVEAUX RECEPTEURS Fc DE TYPE MELANOME INTERVENANT DANS LA PATHOGENESE DU LYMPHOME MALIN ET DU MELANOME

(30) Priority: 29.11.1999 US 168151 P
(43) Date of publication of application: 04.09.2002
(60) Divisional application: 10181794.8
(73) Proprietor: The Trustees of Columbia University in the City of New York, New York, New York 10027 (US)
(72) Inventor: DALLA-FAVERA, Riccardo, New York, NY 10025 (US)
(74) Representative: Vossius & Partner Patentanwälte Rechtsanwälte mbB
(86) International application number: PCT/US2000/032403
(87) International publication number: WO 2001/038490

(56) References cited:
- EP-A- 0 330 191
- DATABASE EMBL [Online] EBI; Homo sapiens cDNA clone IMAGE:1333716 3', mRNA, 16 February 1998 (1998-02-16) NCI-CGAP: "National Cancer Institute, Cancer Genome Anatomy Project (CGAP)" Database accession no. AA811806 XP002241080
- MILLER ET AL.: 'IRTAs: a new family of Fc-related cell surface receptors expressed in B cells and implicated in lymphomagenesis' BLOOD vol. 96, no. 11, 16 November 2000, page 499A, XP002944801

## Description

The invention disclosed was herein made in the course of work under NCI Grant No. CA 44029 from the National Cancer Institute. Accordingly, the U.S. Government has certain rights in this invention.

Throughout this application, various references are referred to in parentheses.

Full bibliographic citation for these references may be found at the end of this application, preceding the claims.

### BACKGROUND OF THE INVENTION

Abnormalities of chromosome 1q21 are common in B cell malignancies, including B cell lymphoma and myeloma, but the genes targeted by these aberrations are largely unknown. By cloning the breakpoints of a t(1;14)(q21;q32) chromosomal translocation in a myeloma cell line, we have identified two novel genes, *IRTA1* and *IRTA2,* encoding cell surface receptors with homologies to the Fc and Inhibitory Receptor families. Both genes are normally expressed in mature B cells, but with different distributions in peripheral lymphoid organs: IRTA1 is expressed in marginal zone B cells, while *IRTA2* is also expressed in germinal center centrocytes and in immunoblasts. As the result of the t(1;14) translocation, the IRTA1 signal peptide is fused to the Immunoglobulin Cα domain to produce a chimaeric IRTA1/Ca fusion protein. In Multiple Myeloma and Burkitt lymphoma cell lines with 1q21 abnormalities, *IRTA2* expression is deregulated. Thus, *IRTA1* and *IRTA2* are novel immunoreceptors with a potentially important role in B cell development and lymphomagenesis.

B-cell Non-Hodgkin's Lymphoma (B-NHL) and Multiple Myeloma (MM) represent a heterogeneous group of malignancies derived from mature B cells with phenotypes corresponding to pre-Germinal Center (GC) (mantle cell), GC (follicular, diffuse large cell, Burkitt's), or post-GC B cells (MM) (for review, Gaidano and Dalla-Favera, 1997; Kuppers et al., 1999). Insights into the pathogenesis of these malignancies have been gained by the identification of recurrent clonal chromosomal abnormalities characteristic for specific disease subtypes. The common consequence of these translocations is the transcriptional deregulation of protooncogenes by their juxtaposition to heterologous transcriptional regulatory elements located in the partner chromosome (Gaidano and Dalla-Favera, 1997). These heterologous transcriptional regulatory elements can be derived from the Immunoglobulin (IG) locus or from other partner chromosomal loci. Examples include *MYC* in t(8;14)(q24;q32) in Burkitt's lymphoma (BL) (Dalla-Favera et al., 1982; Taub et al., 1982), the *CCND1* gene deregulated by the t(11;14)(q13;q32) in mantle cell lymphoma (MCL) (Rosenberg et al., 1991) and multiple myeloma (MM) (Ronchetti et al., 1999), *BCL2* involved in the t(14;18)(q32;q21) in follicular lymphoma (FL) (Bakhshi et al., 1985), *BCL6* in t(3;14)(q27;q32) in diffuse large B cell lymphoma (DLCL) (Ye et al., 1993), as well as *FGFR3* in t(4;14)(p16;q32) (Chesi et al., 1997), *MAF* in t(14;16) (q32;q23) (Chesi et al., 1998) and *MUM1*/*IRF4* in t(6;14)(p25;q32) (Iida et al., 1997) in multiple myeloma (MM). The identification of these oncogenes has offered valuable insights into the pathogenesis and diagnosis of their corresponding malignancies.

Chromosomal abnormalities involving band 1q21-q23 are among the most frequent genetic lesions in both B-NHL and MM. Among NHL subtypes, translocation breakpoints at 1q21-q23, including translocations and duplications, have been reported, often as the single chromosomal abnormality, in 17-20% of follicular and diffuse large B-cell lymphoma (DLCL), in 39% of marginal-zone B cell lymphoma (Offit et al., 1991; Whang-Peng et al., 1995; Cigudosa et al., 1999) and in 27-38% of Burkitt lymphoma, where they represent the second most common cytogenetic abnormality after translocations involving the *MYC* proto-oncogene (Berger and Bernheim, 1985; Kornblau et al., 1991). Comparative genome hybridization (CGH) has also identified 1q21-q23 as a recurring site for high-level amplification in 10% of DLCL cases (Rao et al., 1998). In MM, trisomy of the 1q21-q32 region has been reported in 20-31% of cases (Sawyer et al., 1995), amplification of the 1q12-qter region in 80% of cell lines and 40% of primary tumors (Avet-Loiseau et al., 1997), and nonrandom unbalanced whole-arm translocations of 1q, associated with the multiduplication of the adjacent 1q21-22 region, were found in 23% of patients with abnormal karyotypes (Sawyer et al., 1998).

The high frequency of involvement of 1q21 structural rearrangements in B-cell malignancies suggests that this locus may harbor genes critical to the pathogenesis of these diseases. Cloning of a t(1;14)(q21;q32) in a pre-B cell acute lymphoblastic leukemia cell line previously identified a novel gene, *BCL9* deregulated in this single case (Willis et al., 1998), but not involved in other cases. A recent report characterized the t(1;22)(q22;q11) in a follicular lymphoma (FL) cell line and found that the *FCGR2B* locus, encoding the low affinity IgG Fc receptor FCGRIIB, was targeted in this cell line and in two additional FL cases (Callanan et al., 2000). Finally, the *MUCl* locus has been identified in proximity of the breakpoint of a t(1;14)(q21;q32) in NHL (Dyomin et al., 2000; Gilles et al., 2000), and *MUC1* locus rearrangements have been found in 6% of NHL with 1q21 abnormalities (Dyomin et al., 2000). These results highlight the heterogeneity of the 1q21 breakpoints and the need to identify additional candidate oncogenes situated in this locus, since the large majority of these alterations remain unexplained.

EP-A2 0 330 191 discusses a method for cloning cDNAs from mammalian expression libraries based on transient expression in mammalian host cells.

EMBL Database clone IMAGE:1333716, Database accession no. AA811806 discloses a 475 base pair human messenger ribonucleic acid (mRNA) sequence (AA811806.1). EMBL Database clone IMAGE:2301418, Database accession no. AI699363 (human EST) discloses a 429 base pair human messenger ribonucleic acid (mRNA) sequence. EMBL Database clone IMAGE:1327317, Database accession no. AA724600 discloses a 422 base pair human messenger ribonucleic acid (mRNA) sequence.

The aim of this study was to further explore the architecture of 1q21 chromosomal rearrangements in B cell malignancy. To that end, we have employed a molecular cloning approach of the t(1;14)(q21;q32) present in the myeloma cell line FR4. We have identified two novel genes that are differentially targeted by 1q21 abnormalities. These genes code for five novel members of the immunoglobulin receptor family, *IRTA1, IRTA2, IRTA3, IRTA4 and IRTA5* (Immunoglobulin superfamily Receptor Translocation Associated genes 1, 2, 3, 4, and 5), which may be important for normal lymphocyte function and B cell malignancy.

### SUMMARY OF THE INVENTION

This invention provides an isolated nucleic acid molecule which encodes immunoglobulin receptor, Immunoglobulin superfamily Receptor Translocation Associated, IRTA2, protein.

Disclosed is a method of producing an IRTA polypeptide (protein) which comprises: (a) introducing a vector comprising an isolated nucleic acid which encodes an immunoglobulin receptor, Immunoglobulin superfamily Receptor Translocation Associated, IRTA, protein into a suitable host cell; and (b) culturing the resulting cell so as to produce the polypeptide.

This invention provides an isolated nucleic acid molecule comprising at least 15 contiguous nucleotides capable of specifically hybridizing with a unique sequence included within the sequence of the isolated nucleic acid molecule encoding IRTA2 protein, or fragment(s) thereof, having the amino acid sequence set forth in any of Figure 18B-1-18B-3.

This invention provides a method for detecting a B cell malignancy or a type of B cell malignancy in a sample from a subject wherein the B cell malignancy comprises a 1q21 chromosomal rearrangement which comprises:
a) contacting the RNA of a sample obtained from the subject with a nucleic acid molecule of at least 15 contiguous nucleotides capable of specifically hybridizing with a unique sequence included within the sequence of an isolated RNA encoding human IRTA2 protein under conditions permitting hybridization of the RNA of step (a) with the nucleic acid molecule capable of specifically hybridizing with a unique sequence included within the sequence of an isolated RNA encoding human IRTA2 protein, wherein the nucleic acid molecule is labeled with a detectable marker; and b) detecting any hybridization in step (a), wherein detecion of hybridization indicates presence of B cell malignancy or a type of B cell malignancy in the sample.

This invention provides an antisense oligonucleotide having a sequence capable of specifically hybridizing to an mRNA molecule encoding a human IRTA2 protein so as to prevent overexpression of the mRNA molecule.

This invention provides a purified IRTA2 protein comprising the amino acid sequence set forth in Figures 18B-1-18B-3 (SEQ ID NO:3).

This invention provides an antibody/antibodies directed to an epitope of a purified IRTA2 protein, or fragment(s) thereof, having the amino acid sequence set forth in any of Figures 18B-1-18B-3.

This invention provides an antibody directed to a purified IRTA2 protein.

This invention provides a pharmaceutical composition comprising an amount of the antibody directed to an IRTA2 protein effective to bind to cancer cells expressing a human IRTA2 protein. so as to prevent growth of the cancer cells and a pharmaceutically acceptable carrier.

This invention provides a pharmaceutical composition comprising an amount of any of the oligonucleotides of nucleic acid molecules encoding IRTA2 proteins described herein effective to prevent overexpression of a human IRTA2 protein and a pharmaceutically acceptable carrier capable. This invention provides a method of diagnosing B cell malignancy which comprises a 1q21 chromosomal rearrangement in a sample from a subject which comprises:
a) contacting the sample obtained from the subject with an antibody directed to a purified IRTA2 protein capable of specifically binding with a human IRTA2protein on a cell surface of a cancer cell under conditions permitting binding of the antibody with human IRTA2 protein on the cell surface of the cancer cell, wherein the antibody is labeled with a detectable marker; and b) detecting any binding in step (a), wherein detecion of binding indicates a diagnosis of B cell malignancy in the sample.

Disclosed is a method of detecting human IRTA protein in a sample which comprises: a) contacting the sample with any of any of the above-described anti-IRTA antibodies under conditions permitting the formation of a complex between the antibody and the IRTA in the sample; and b) detecting the complex formed in step (a), thereby detecting the presence of human IRTA in the sample.

This invention provides a use for treating a subject having a B cell cancer which comprises administering to the subject an amount of anti-IRTA2 antibody effective to bind to cancer cells expressing an IRTA2 protein so as to prevent growth of the cancer cells and a pharmaceutically acceptable carrier, thereby treating the subject.

This invention provides a use for treating a subject having a B cell cancer which comprises administering to the subject an amount of an antisense oligonucleotide having a sequence capable of specifically hybridizing to an mRNA molecule encoding a human IRTA2 protein so as to prevent overexpression of the humanIRTA2 protein, so as to arrest cell growth or induce cell death of cancer cells expressing IRTA2 protein and a pharmaceutically acceptable carrier, thereby treating the subject.

The invention also provides a pharmaceutical composition comprising either an effective amount of any of the oligonucleotides described herein and a pharmaceutically acceptable carrier.

The invention also provides a pharmaceutical composition comprising either an effective amount of an antibody directed against an epitope of IRTA2 protein described herein and a pharmaceutically acceptable carrier.

### BRIEF DESCRIPTION OF THE FIGURES

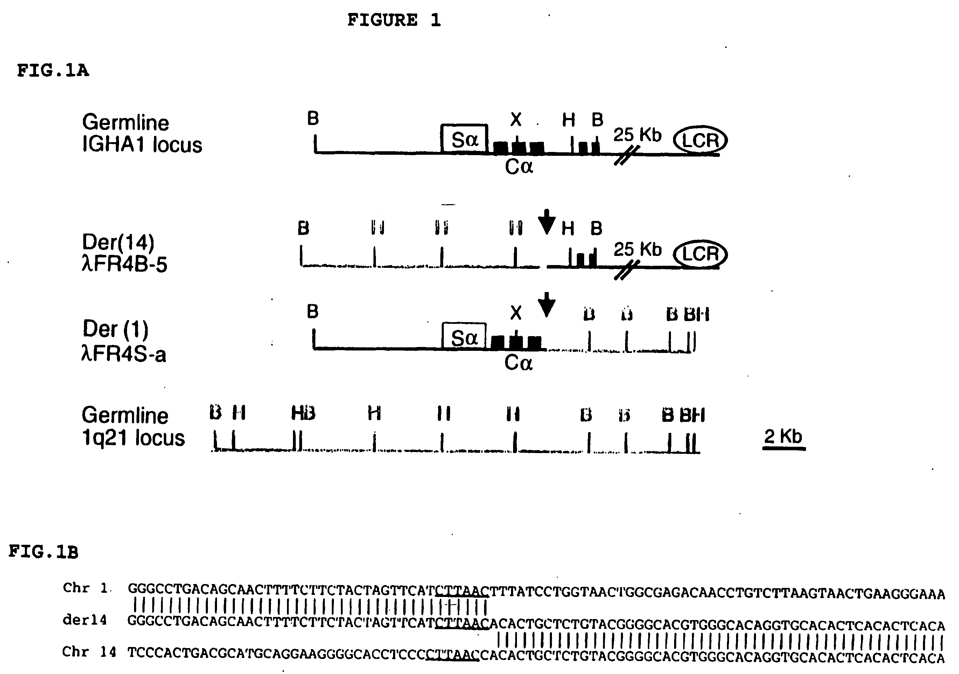

- **Figures 1A-1B.**: Molecular cloning of the translocation t(1;14)(q21;q32) in the FR4 multiple myeloma cell line. Fig. 1A) Schematic representation of the λFR4B-5 and λFR4S-a clones, representing der(14) and der(1) breakpoints, and of the germline IgH and 1q21 loci. Fig. 1B) Nucleotide sequence of the breakpoint junction and its alignment to the corresponding germline regions of chromosome 14. Sα, IgA switch region; LCR: 3' IgH locus control region; B, *BamHI;* H, *HindIII;* X, *XhoI.*

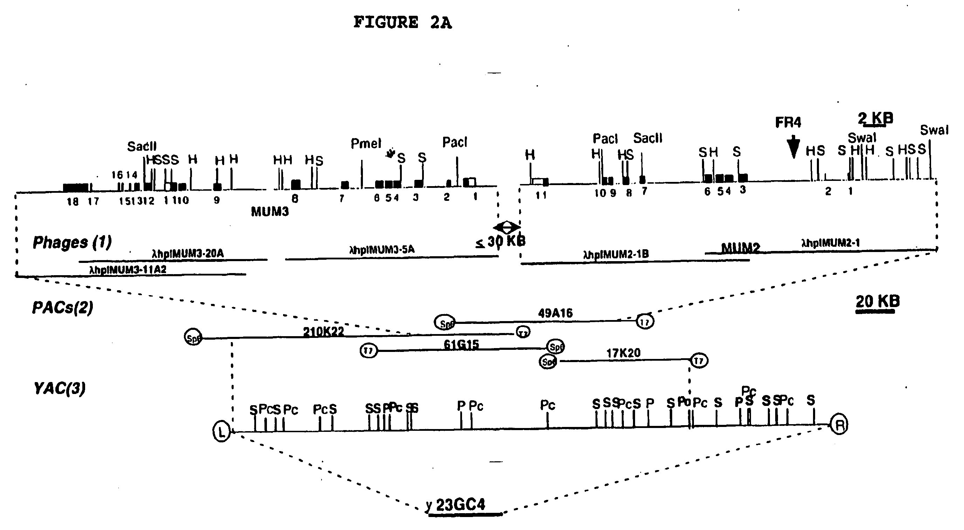

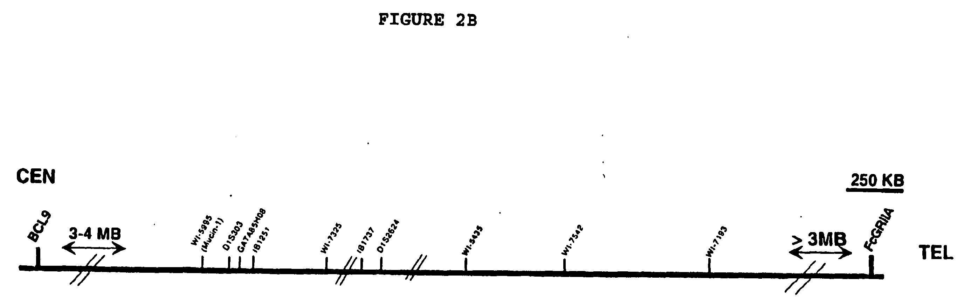

- **Figures 2A-2B.**: Genomic map of the 1q21 locus in the vicinity of the FR4 breakpoint. Fig. 2A) Restriction endonuclease map and schematic representation of genomic clones, i.e. bacteriophages (1), P1 artificial chromosomes (PACs) (2), and yeast artificial chromosome (YAC) (3), spanning the germline 1q21 locus at the FR4 breakpoint region (arrowhead). The name of each clone is placed directly on top of its representation. End fragments derived from the PAC and YAC inserts are depicted as circles, with either an SP6/T7 vector orientation (PAC), or left/right arm vector orientation (YAC). The top panel in Fig. 1A depicts the genomic organization of two genes surrounding the FR4 breakpoint. The two genes were identified by exon trapping of PAC 49A16. They are closely spaced in the genome, within s 30 Kb of each other and are named MUM2 and MUM3 (multiple myeloma-2 and 3). In the scheme of their genomic loci, black boxes indicate coding exons, whereas white and light or medium grey boxes indicate non-coding exons. Connecting introns are lines. MUM3 (left) gives rise to three alternatively spliced mRNAs, all sharing a common 5' untranslated region (UTR) but diverse 3' UTRs (marked by different shades). Numbers underneath the boxes identify the order of exons in the cDNA. Exons less than 100 bp are depicted as thin vertical lines. The position and size of each exon was determined by sequencing of genomic PAC and phage clones and by hybridization of cDNA probes to endonuclease-digested clone DNA. PAC and YAC mapping was performed by partial digestion with rare cutting enzymes followed by Pulse-Field-Gel-Electrophoresis and hybridization to internal and end-derived probes. Dashed lines align regions of overlap. S, SacI; H, HindIII; S, Swal; Pc, PacI; P, PmeI; Fig. 2B) Genethon genetic linkage map of 1q21 in the region of the MUM2/MUM3 locus. Sequence-tagged sites (STS) are ordered in approximate distance previously determined by Dib, C., et al. (1996) Nature, 380:162-164. STS WI-5435 (in bold) is contained within YAC 23GC4 and PAC 49A16. Parallel vertical lines represent interrupted segments, whose approximate size is depicted above in megabases (MB). Sizing was estimated by the size of nonchimeric YAC contigs between two markers. The BCL9 gene at the centromere was cloned from a different t(1;14) (q21;q32) breakpoint by Willis T.G. et al., (1998) Blood 91, 6:1873-1881. The FcGRIIA gene is at the 1q21-q22 chromosomal band border.

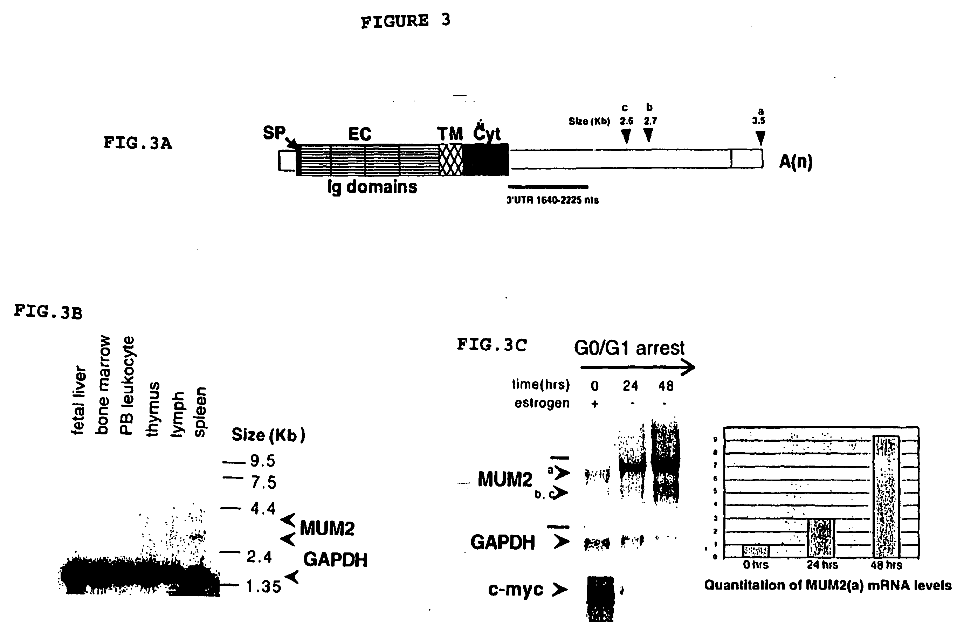

- **Figures 3A-3C.**: MUM2 mRNA structure and expression pattern. Fig. 3A) Schematic representation of MUM2 mRNA. Pattern-filled, wide boxes represent coding domains and narrow empty boxes represent untranslated regions. SP, signal peptide; EC, extracellular domain; TM, transmembrane domain; CYT, cytoplasmic domain; A(n), polyA tail. The extracellular region is composed of four immunoglobulin-like domains as depicted. Alternative polyadenylation signals (arrows) generate three MUM2 mRNA species (a, b, c) whose length (in Kb) ranges from 2.6-3.5. Fig. 3B) Northern blot analysis of MUM2 mRNA expresion in human tissues of the immune system. The cDNA probe used for the analysis is shown as a solid bar underneath the mRNA scheme in Fig. 3A). Each lane contains 2µg mRNA of the corresponding tissue. On the right side of the blot, the position of RNA molecular weight markers is depicted. The position of MUM2 and GAPDH mRNA transcripts is shown by arrows. (A GAPDH probe was included in the hybridization as an internal control-0.15 ng labelled +50 ng unlabelled probe-). The rsults of this analysis show weak expression of MUM2 in lymph node and spleen. MUM2 expression was not detected in a variety of other human tissues (data not shown). Fig. 3C) Northern blot analysis of MUM2 expression in total RNA from EREB, a conditional EBV-transformed B lymphoblastoid cell line. EREB carries the EBV genome with an EBNA2-estrogen receptor fusion protein, active only in the presence of estrogen. For this experiments, cells were grown in the presence of estrogen (1µg/ml) followed by estrogen withdrawal for the indicated times. Upon estrogen withdrawal, EREB cells undergo G0/G1 arrest, determined by the loss of c-myc expression. In Fig. 3C, a Northern blot of EREB total RNA (10µg per lane) was hybridized with the MUM2 cDNA probe shown in Fig. 3A and the GAPDH internal control probe, as in Fig. 3B. Arrows indicate the position of the corresponding mRNAs on the EREB blot. a, band c correspond to the MUM2 species in panel Fig 3A. The same blot was then stripped and reprobed with a c-myc cDNA probe (exon 2) to verify cellular G0/G1 arrest. Quantitation of MUM2 mRNA by the use of a phosphorimager densitometric analysis demonstrates a 10-fold increase in their levels within 48 hrs of estrogen withdrawal, suggesting that MUM2 expression is elevated as the cells enter a resting phase.

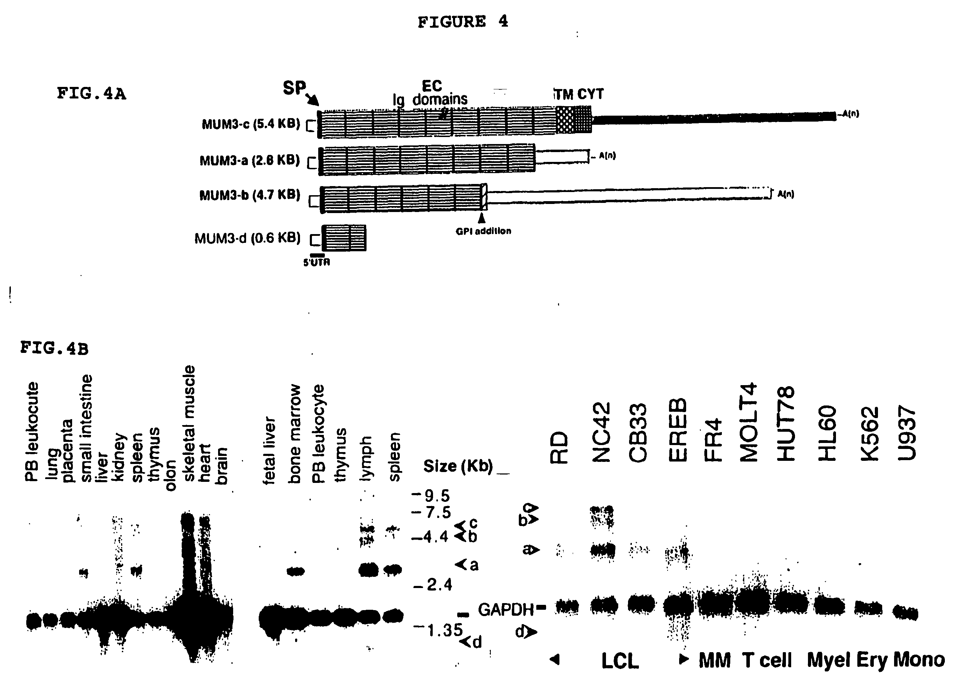

- **Figures 4A-4B.**: MUM3 mRNA structure and expression pattern. Fig. 4A) Schematic representation of MUM3 mRNA Pattern-filled, wide boxes represent coding domains and narrow empty or gray boxes represent untranslated regions. SP, signal peptide; EC, extracellular domain; TM, transmembrane domain; CYT, cytoplasmic domain; A(n), polyA tail. The extracellular region is composed of immunoglobulin-like domains, as depicted. Alternative splicing generates four mRNA species with diverse subcellular localization. MUM3-a and -d proteins are secreted, whereas MUM3-b contains a hydrophobic stretch of amino acids at its C-terminus which may serve as a signal for addition of a glycophosphatidyl-inositol anchor (GPI-anchor), as shown. MUM3-c spans the plasma membrane. Sequence identity among species is indicated by identical filling. Fig. 4B) Northern blot analysis of MUM3 mRNA expression in multiple human tissues (left) and in various lymphoid and non-lymphoid cell lines (right). The cDNA probe used is shown as a solid bar below the cDNA scheme in Fig. 4A. Each lane contains 2µg mRNA of the corresponding tissue or cell line. The position of MUM3 and GAPDH mRNA transcripts is shown by arrows. (A GAPDH probe was included in the hybridization as an internal control as described in Fig. 3) a, b, c and d correspond to the MUM3 mRNA species shown in Fig. 4A. RD, NC42 and CB33, Epstein-Barr virus transformed B lymphoblastoid cell lines; EREB, conditional EBV-transformed B lymphoblastoid cell line; FR4, plasma cell line; MOLT4 and HUT78, T cell lines; HL60 and U937, myelomonocytic cell lines; K562, erythroid cell line. The results suggest that MUM3 is expressed solely in the immune system tissues of bone marrow, lymph and spleen and in particular in B cells with a lymphoblastoid phenotype.

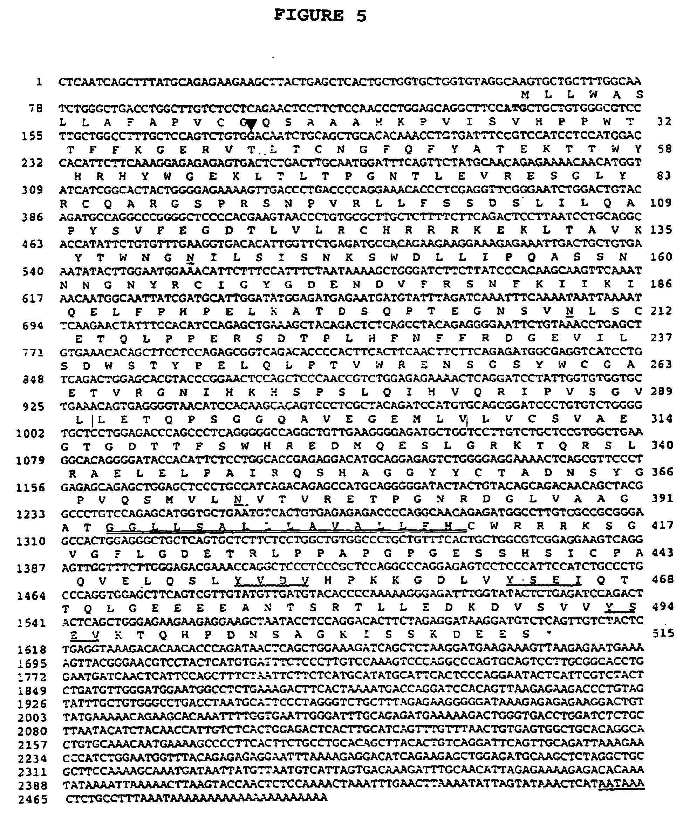

- **Figure 5.**: Nucleotide and amino acid sequence of human MUM2. The deduced amino acid sequence is shown above the nucleotide sequence in one-letter code and is numbered on the right, with position 1 set to the first codon of the signal peptide. The predicted signal peptidase site was derived by a computer algorithm described in Nielsen et al., Protein Engineering 10, 1-6 (1997) and is marked by an arrowhead. The polyadenylation signal AATAAA is underlined. Potential sites for N-glycosylation are also underlined in the amino acid sequence. A hydrophobic stretch of 16 amino acids predicted to span the plasma membrane is doubly underlined. Consensus SH2-binding sites are highlighted by a wavy underline.

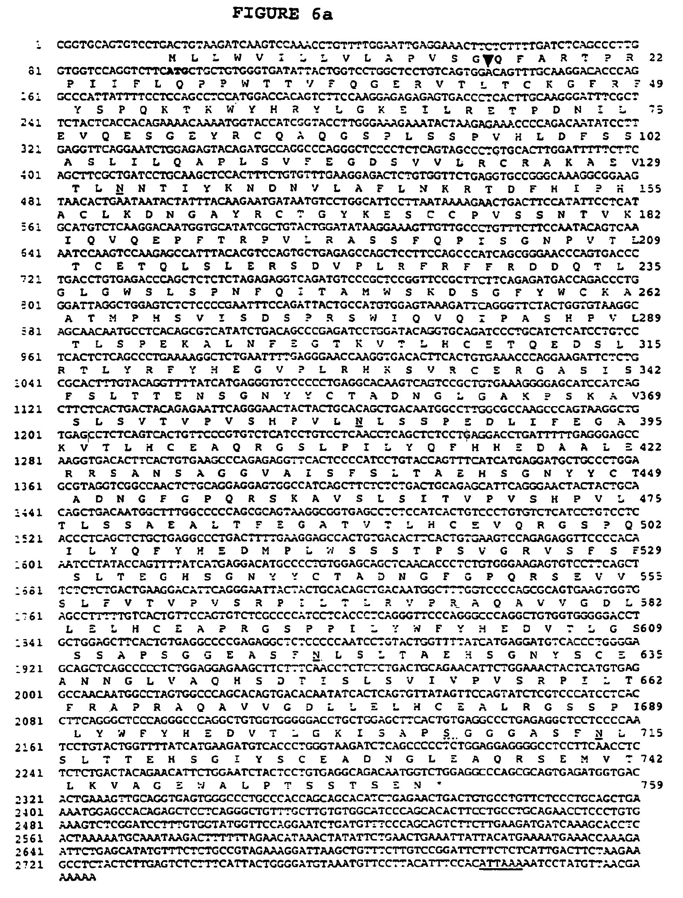

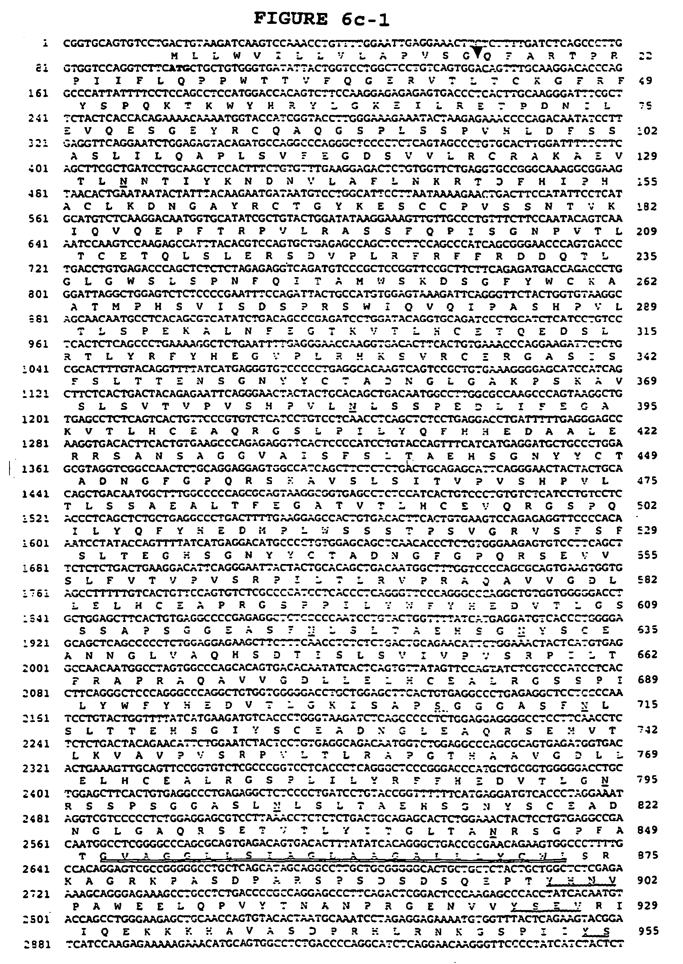

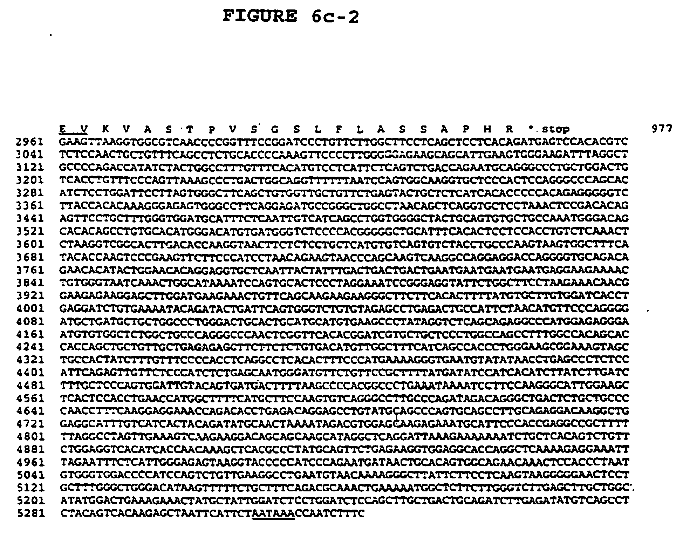

- **Figure 6A.**: Nucleotide and amino acid sequence of human MUM3-a. The deduced amino acid sequence is shown above the nucleotide sequence in one-letter code and is numbered on the right, with position 1 set to the first codon of the signal peptide. The predicted site for signal peptidase cleavage was derived as previously stated above and is.marked by an arrowhead. The polyadenylation signal ATTAAA is underlined. Potential sites for N-glycosylation are also underlined in the amino acid sequence. The protein lacks a transmembrane domain and is predicted to be secreted.

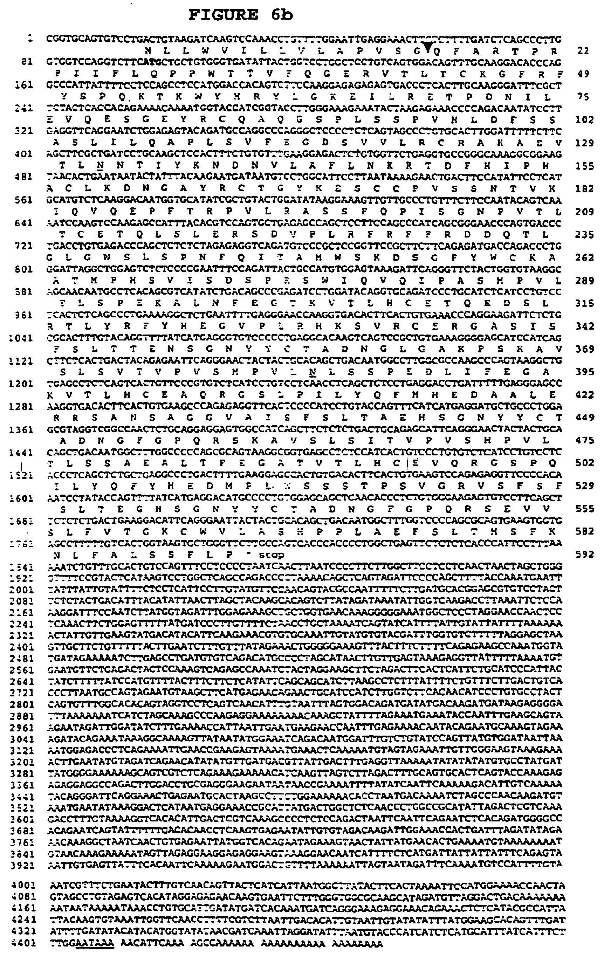

- **Figure 6B.**: Nucleotide and amino acid sequence of human MUM3-b. The deduced amino acid sequence is shown above the nucleotide sequence in one-letter code and is numbered on the right, with position 1 set to the first codon of the signal peptide. The predicted site for signal peptidase cleavage was derived as previously stated above and is marked by an arrowhead. The polyadenylation signal AATAAA is underlined. Potential sites for N-glycosylation are underlined in the amino acid sequence.
- **Figure 6C-1-6C-2.**: Nucleotide and amino acid sequence of human MUM3-c. The deduced amino acid sequence is shown above the nucleotide sequence in one-letter code and is numbered.on the right, with position 1 set to the first codon of the signal peptide. The predicted site for signal peptidase cleavage was derived as previously stated above and is marked by an arrowhead. The polyadenylation signal AATAAA is underlined. Potential sites for N-glycosylation are underlined in the amino acid sequence. A hydrophobic stretch of 23 amino acids predicted to span the plasma membrane is doubly underlined. Consensus SH2-binding sites are highlighted by a wavy underline.

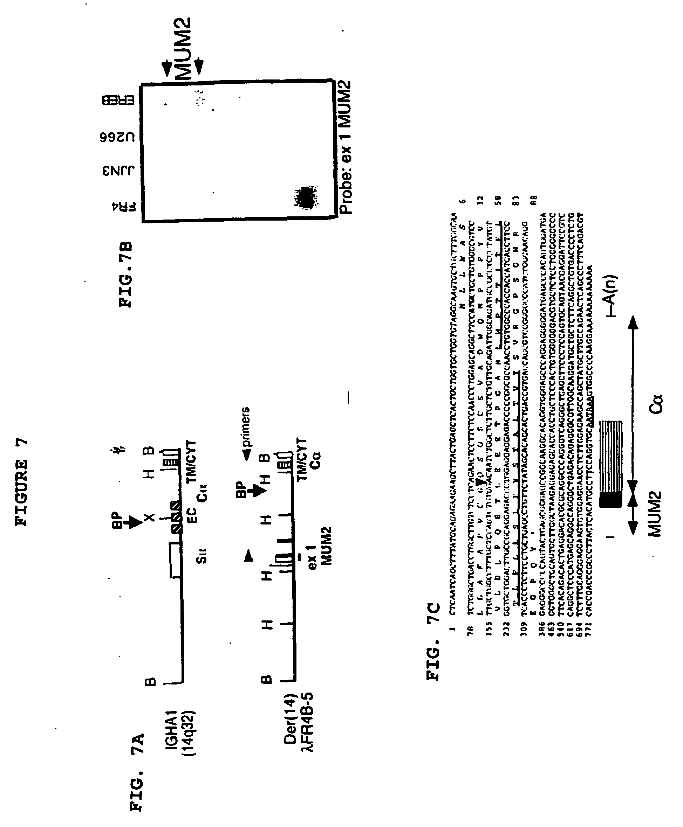

- **Figures 7A-7C.**: t(1;14)(q21;32) in FR4 generates a MUM2/Ca fusion transcript. Fig. 7A) Schematic representation of ther der(14) genomic clone λFR4B-5 and of the germline IgHA1 locus. The FR4 breakpoint is marked by an arrow. Filled and open boxes represent the MUM2 and Calpha coding and non-coding exons respectively. The position of the MUM2 exon 1 probe used for Northern blot analysis is shown by a bar. Fig. 7B) Northern blot analysis with a MUM2 exon 1 probe on FR4 and additional cell lines detects an abnormal message of 0.8 Kb, selectively in FR4. Arrowheads point to the location of normal MUM2 message in EREB mRNA. JJN3 and U266, myeloma cell lines; EREB, conditional EBV-transformed B lymphoblastoid cell line. Two µg of polyA+ RNA were loaded per lane. Fig. 7C) Nucleotide ans amino acid sequence of the MUM2-Ca fusion cDNA in FR4. The cDNA was amplified by RT-PCR from FR4 total RNA using the primers shown in Fig. 7A, and was subsequently subcloned and sequenced. The deduced amino acid sequence is shown above the nucleotide sequence in one-letter code and is numbered on the right with position 1 set to the first codon of the signal peptide. The predicted site for signal peptidase cleavage was derived as previously stated above and is marked by an arrowhead. The polyadenylation signal AATAAA is underlined. The Calpha transmembrane domain is underlined. The MUM2 portion of the cDNA is shown on italics. H, HindIII; B, BamHI; X, XhoI; Sα, IgA switch region; EC, extracellular region; TM, transmembrane; CYT, cytoplasmic domain.

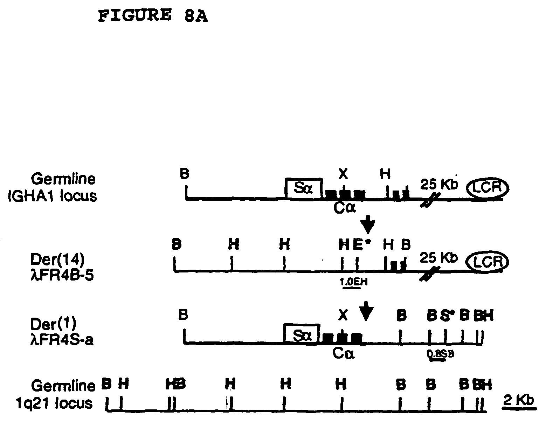

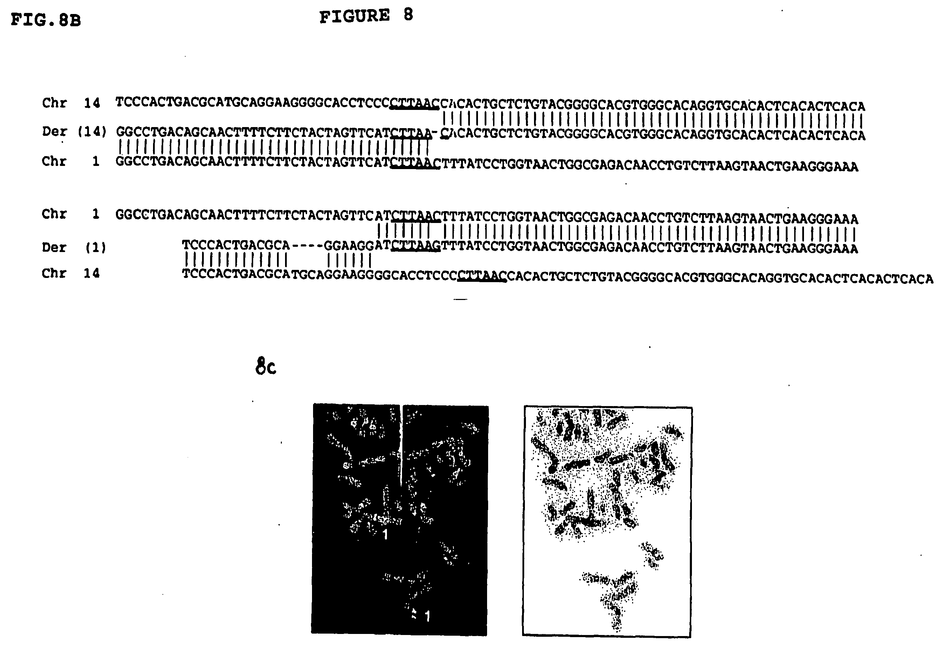

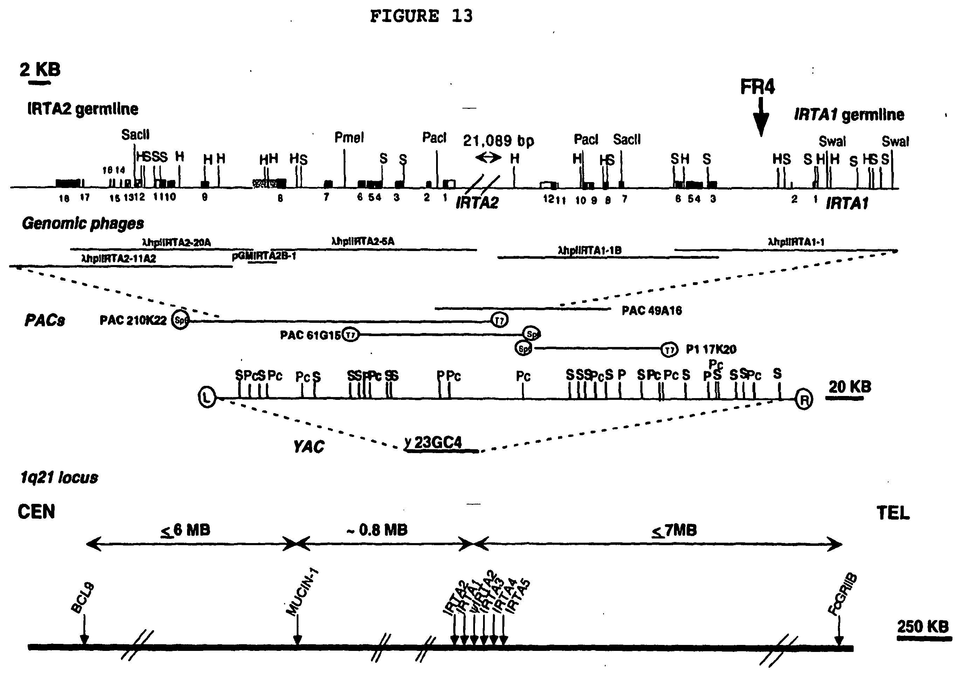

- **Figures 8A-8C.**: Molecular cloning of the translocation t(1;14)(q21;q32) in the FR4 multiple myeloma cell line. Fig. 8A) Schematic representation of the phage clones representing der(14) and der(1) breakpoints and the germline IGH and 1q21 loci. Chromosome 14 sequences are indicated by a solid black line with black boxes representing Ca1 exons. Chromosome 1 sequences are shown as a grey line. The probes used for chromosomal mapping are indicated below the map. Restriction enzyme codes are: B, BamHI; H, HindIII; X, XhoI; S, SacI; E, EcoRI. For enzymes marked by (*) only sites delineating the probes are shown. Sa: IgA switch region; LCR: 3'IgH locus control region. Fig. 8B) Nucleotide sequence of the breakpoint junctions and their alignment to the corresponding germline regions of chromosomes 14 and 1. Fig. 8C) Left, fluorescence in situ hybridization (FISH) analysis on human normal metaphase spreads with the PAC clone 49A16 (Fig. 13) spanning the germline1q21 region at the FR4 breakpoint. Right, DAPI stained image from the same metaphase spread.

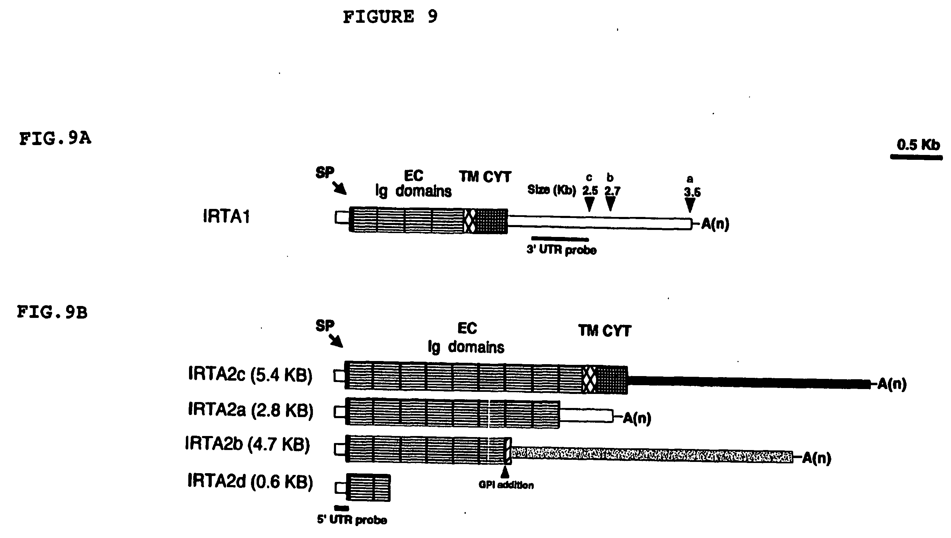

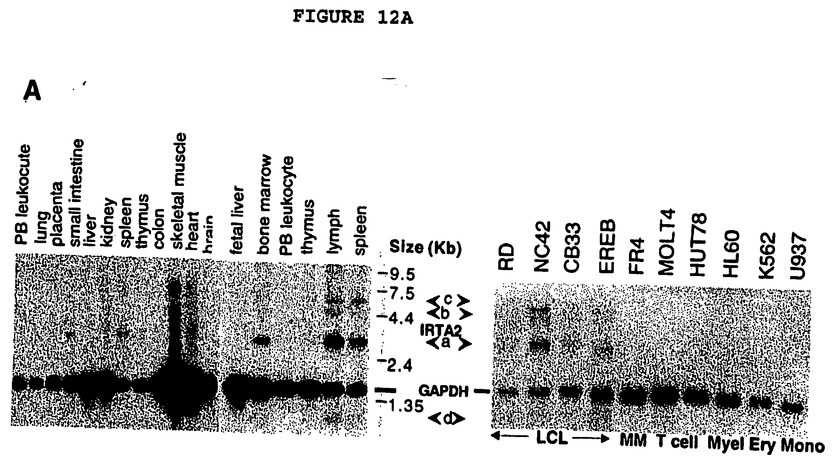

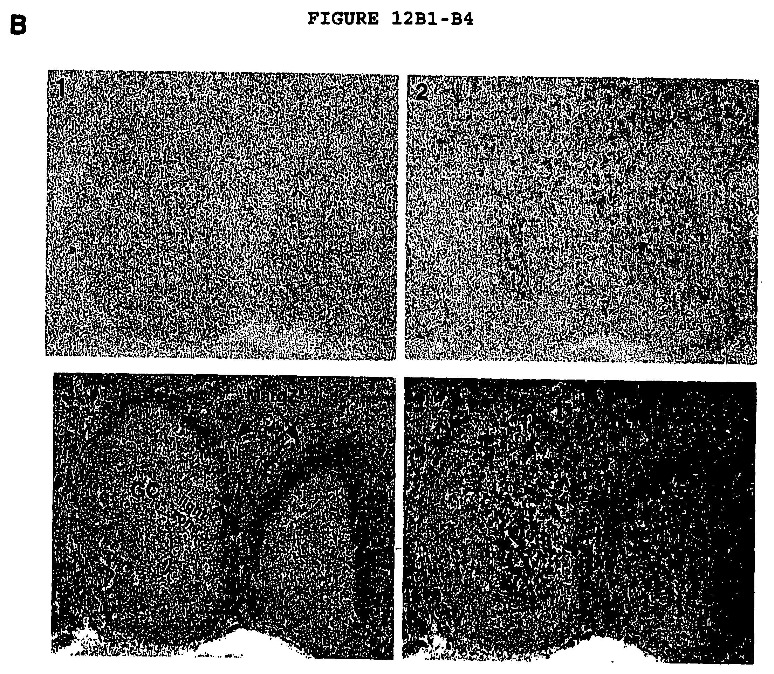

- **Figures 9A-9B.**: Structure of *IRTA1* and *IRTA2* cDNAs. Figs. 9A;9B) Schematic representation of the full-length *IRTA1* (Fig. 9A) and *IRTA2* (Fig. 9B) cDNAs. Pattern-filled, wide boxes represent coding domains and narrow boxes represent untranslated regions (UTR). The predicted site for signal peptidase cleavage is marked by an arrowhead and was derived according to the SignalIP World Wide Web server at http://www.cbs.dtu.dk/service/SignalIP. The transmembrane domain prediction algorithm is described in Tusnady et al, 1998. SP, signal peptide; EC, extracellular domain; Ig, immuno-globulin-type; TM, transmembrane domain; CYT, cytoplasmic domain; A(n), polyA tail; GPI, glycophosphatidyl inositol. In (Fig. 9A), arrows in the 3' UTR indicate different polyadenylation addition sites utilized in the *IRTA1* cDNA. In (Fig. 9B), different 3'UTR regions in *IRTA2* isoforms are differentially shaded. Bars underneath the UTR regions in (Fig. 9A) and (Fig. 9B) identify probes used for Northern blot analysis in Figure 12.

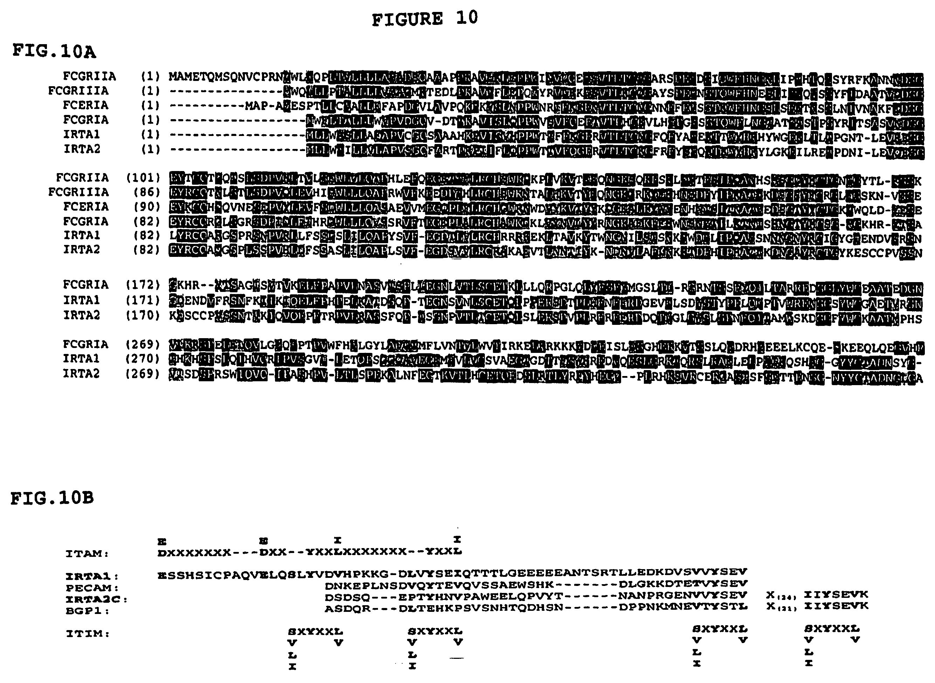

- **Figures 10A-10B.**: Comparison of the amino acid sequences of IRTA1 and IRTA2 with members of the Fc Receptor family Fig. 10A) Multiple sequence alignment of the first two (top) and the third (bottom) extracellular Ig-domains of IRTA1 and IRTA2 to Fc receptor family members. The sequences were compared using the ClustalW program (Thompson et al., 1994). Black-shaded boxes indicate conserved aminoacids among all sequences; dark-grey shaded boxes indicate conserved aminoacids among at least half of the sequences; light-shaded boxes indicate conservative substitutions. Fig. 10B) Alignment of the SH2-binding domains of IRTA1 and IRTA2 with the ITAM and ITIM consensus motifs. Conserved aminoacid positions are in bold. Symbol X represents any aminoacid.

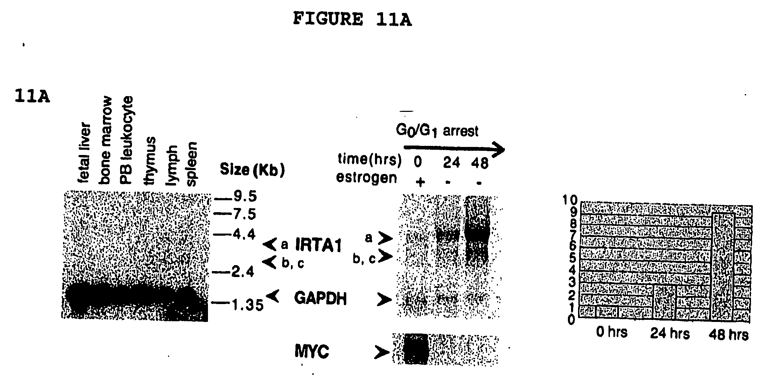

- **Figures 11A-11B-4.**: *IRTA1* expression pattern. Fig. 11A) Left panel. Northern blot analysis of *IRTA1* mRNA expression in tissues of the human immune system. Each lane contains 2mg mRNA. The position of RNA molecular weight markers is depicted on the right side of the blot. The positions of the *IRTA1* and *GAPDH* mRNA transcripts are shown by arrows. (A GAPDH probe was included in the hybridization as an internal control-0.15 ng labelled + 50 ng unlabelled probe-). Right Panel. Northern blot analysis of *IRTA1* expression in total RNA from the ER/EB cell line (10 mg per lane). For this experiment, cells were grown in the presence of estrogen (1mg/ml), followed by estrogen withdrawal for the indicated times. Arrows indicate the positions of the corresponding mRNAs. a, b and c correspond to the *IRTA1* differentially polyadenylated species. The same blot was stripped and reprobed with a *MYC* cDNA probe (exon 2) to verify cellular G₀/G₁ arrest. Densitometric analysis of *IRTA1* mRNA levels is plotted in the adjacent column graph. The cDNA probe used is shown as a solid bar underneath the *IRTA1* mRNA scheme in Figure 9A. Fig. 11B-1-11B-4) *In situ* hybridization analysis of *IRTA1* expression in serial sections of human tonsil. 1. Sense *IRTA1* probe 2. Antisense *IRTA1* probe 3. H&E staining 4. Antisense *IRTA1* signal superimposed over an H&E stained section. GC, germinal center, MargZ, marginal zone
- **Figure 12A-12B-4.**: *IRTA2* expression pattern. Fig. 12A) Northern blot analysis of *IRTA2* mRNA expression in multiple human tissues (left panel) and in various lymphoid and non-lymphoid cell lines (right panel). Each lane contains 2mg mRNA. The positions of the *IRTA2* and *GAPDH* transcripts are shown by arrows. a, b, c and d correspond to the alternatively spliced *IRTA2* mRNA isoforms. RD, NC42 and CB33, Epstein-Barr virus transformed B lymphoblastoid cell lines; EREB, conditional EBV-transformed B lymphoblastoid cell line; FR4, plasma cell line; MOLT4 and HUT78, T cell lines; HL60 and U937, myelomonocytic cell lines; K562, erythroid cell line. The cDNA probe used is shown as a solid bar underneath the *IRTA2* mRNA scheme in Figure 9B. Figs. 12B-1-12B-4) *In situ* hybridization analysis of *IRTA2* mRNA expression in human tonsil. Fig. 12B-1. Sense *IRTA2* cDNA probe, Fig. 12B-2. Antisense *IRTA2* cDNA probe, Fig. 12B-3. H&E staining, Fig. 12B-4. Antisense *IRTA2* cDNA probe signal superimposed over H&E stained section. GC, germinal center, MargZ, marginal zone
- **Figure 13.**: Map of the germline 1q21 region spanning the FR4 breakpoint and genomic organization of *IRTA1* and *IRTA2.* Primers used to amplify *IRTA1* exons from spleen cDNA are marked by arrowheads on top panel. Black and light boxes indicate coding and non-coding exons respectively. Arrows indicate position of *BCL9, MUC1, IRTA* family and *FCGRIIB.* loci. S, SacI; H, HindIII; S, SwaI; Pc, PacI; P, PmeI; Mb, Megabases

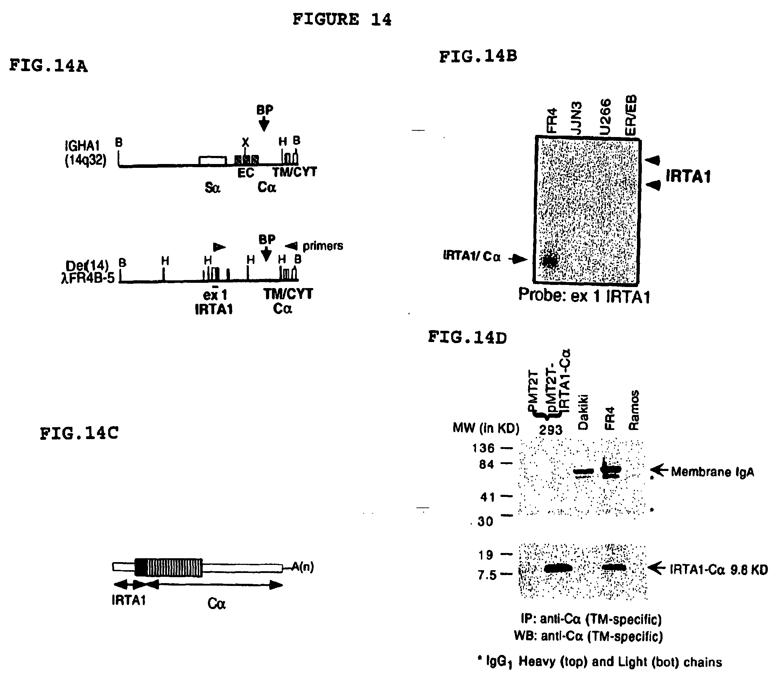

- **Figures 14A-14D.**: t(1;14)(q21;q32) in FR4 generates an *IRTA1*/*Cα* fusion transcript. Fig. 14A) Schematic representation of the der(14) genomic clone 1FR4B-5 and of the germline *IgCα₁* locus. The FR4 breakpoint is marked by an arrow. Filled and open boxes represent the *IRTA1* and *Ca₁* coding and non-coding exons respectively. Fig. 14B) Northern blot analysis with an *IRTA1* exon 1 probe (shown by a bar in Fig. 14A) on FR4 and additional cell lines detects an abnormal message in FR4. Arrowheads point to the location of normal *IRTA1* message in ER/EB mRNA. JJN3 and U266, myeloma cell lines. Two mg of polyA+ RNA loaded per lane. Fig. 14C) Schematic representation of the *IRTA1*/*Cα* fusion cDNA in FR4. The cDNA was amplified by RT-PCR from FR4 total RNA using the primers shown in (Fig. 14A), and sequenced after subcloning. Fig. 14D) SDS/PAGE analysis of immunoprecipitates obtained from vector control transfected and *IRTA1*/*Cα* transient expression construct transfected 293-T cells (lanes 1 & 2), or the following cell lines: mIgA positive lymphoblastoid cell line-Dakiki (lane 3), FR4 (lane 4), mIgM positive NHL cell line-Ramos (lane 5). H, HindIII; B, BamHI; X,XhoI; Sa, IgA switch region; EC, extracellular region; TM, transmembrane; CYT, cytoplasmic

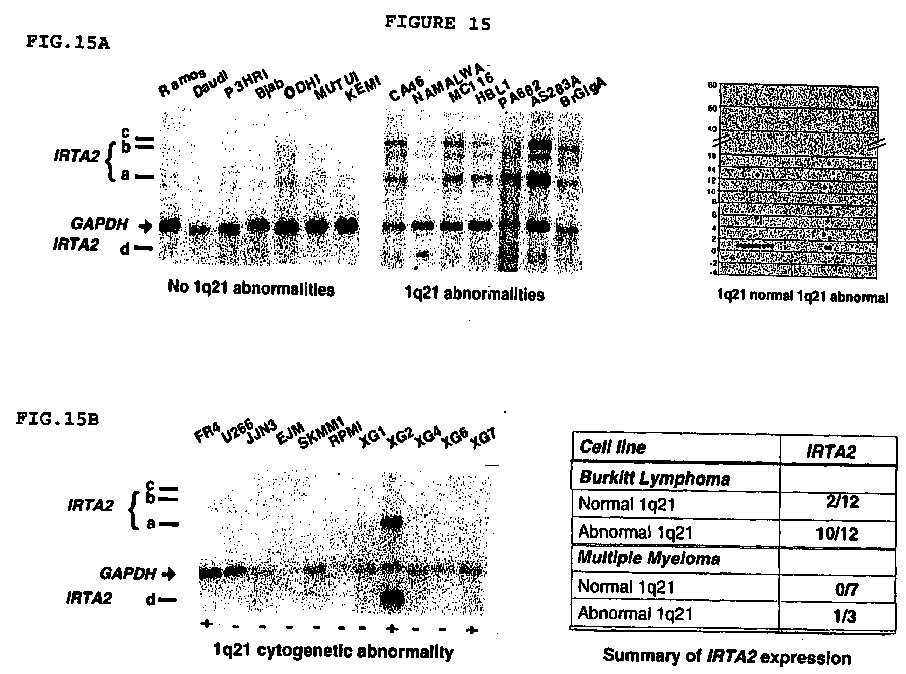

- **Figures 15A-15B.**: *IRTA2* expression is deregulated in cell lines carrying 1q21 abnormalities. Figs. 15A, 15B) Northern blot analysis of *IRTA2* mRNA expression in Burkitt lymphoma (Fig. 15A) and Multiple Myeloma (Fig. 15B) cell lines. The cDNA probe used is the same as in Fig. 12. Each lane contains 2mg mRNA. The positions of the *IRTA2* and *GAPDH* mRNA transcripts are shown by dashes and arrows, respectively. The relative levels of *IRTA2* mRNA expression in the left panel (Fig. 15A) were plotted on the right panel (Fig. 15A) after densitometric analysis and normalization versus the *GAPDH* levels. The right panel of (Fig. 15B) is a summary of the Northern blot analysis results.

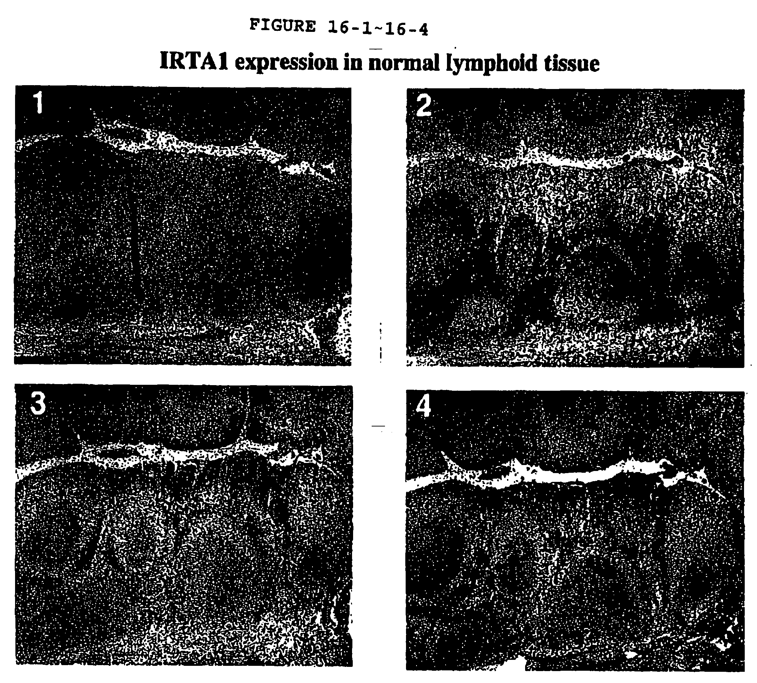

- **Figures 16-1-16-4**: IRTA1 expression in normal lymphoid tissue. Paraffin-embedded sections from normal human tonsil were stained with the following antibodies: Fig. 16-1) Negative control; Fig. 16-2) anti-CD3 mouse monoclonal to detect T cells; Fig. 16-3) anti-IRTA1 (mIRTA) mouse monoclonal; Fig. 16-4) anti-IRTA1 (J92884K) rabbit polyclonal. IRTA1 positive cells are located in the perifollicular and intraepithelial region of the tonsil, the equivalent of the marginal zone in the spleen.

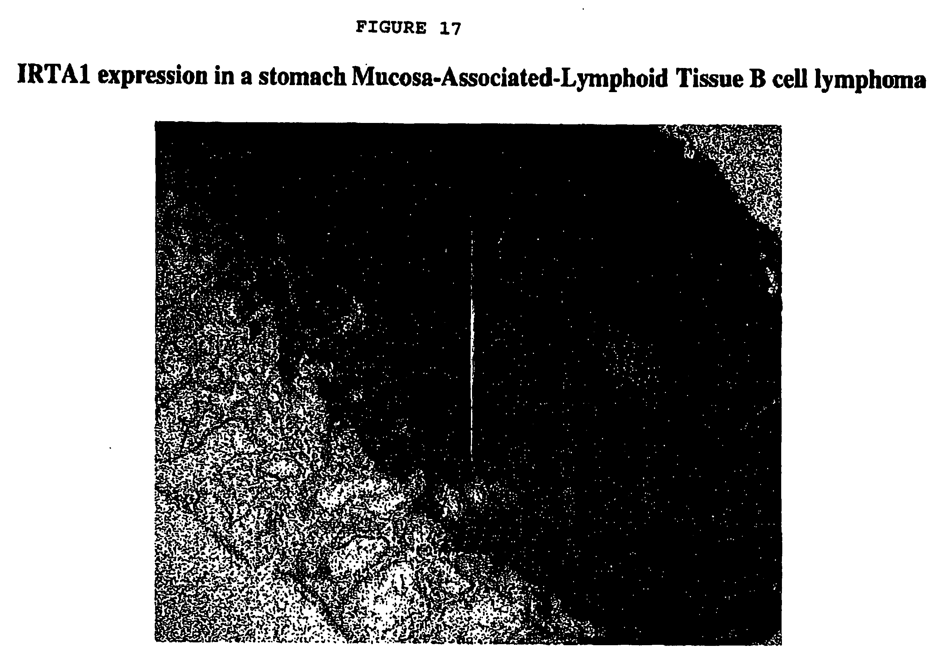

- **Figure 17**: IRTA1 expression in a stomach Mucosa-Associated-Lymphoid Tissue (MALT) B cell lymphoma. A paraffin-embedded section from a stomach MALT B cell lymphoma was stained with the anti-IRTA1 (mIRTA) mouse monoclonal antibody and counterstained with H&E. The majority of MALT lymphomas analyzed were IRTA1 positive. This antibody therefore can be an effective tool in the differential diagnosis of MALT lymphoma. The mIRTA1 antibody may also be proven useful in the therapy of this B cell tumor, similarly to the use of the anti-CD20 antibody (Rituximab) in the therapy of relapsed CD20-positive lymphomas (Foon K., Cancer J. 6: p273).

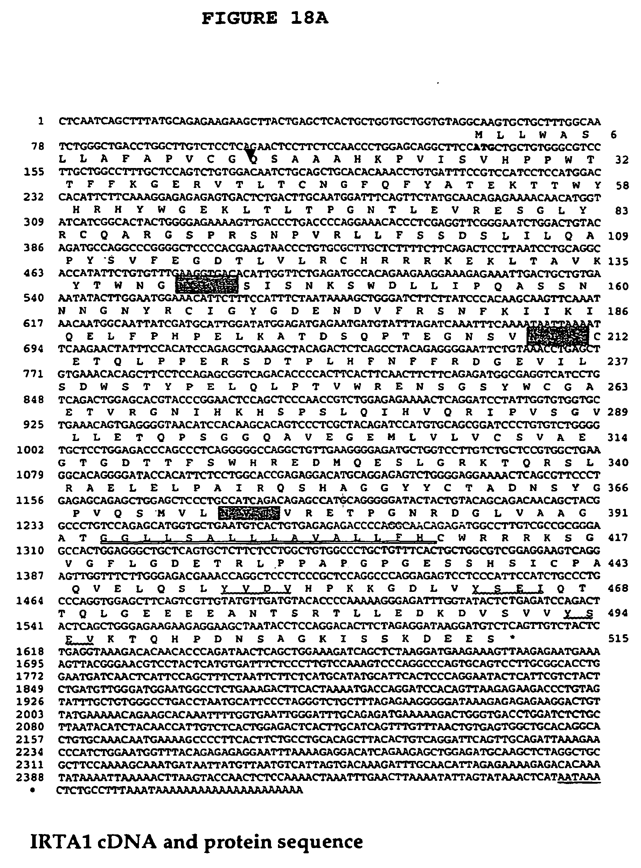

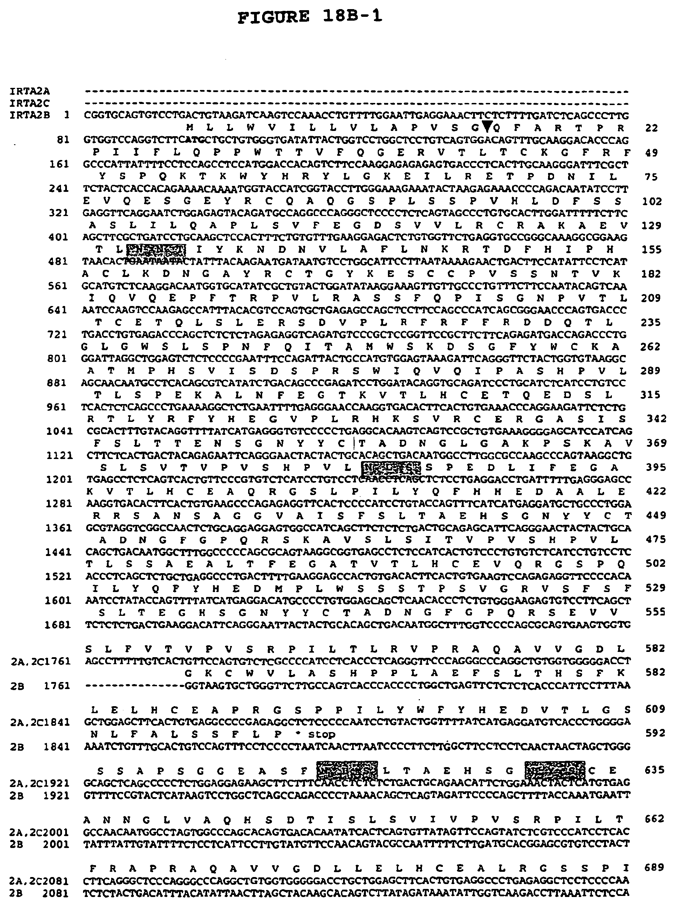

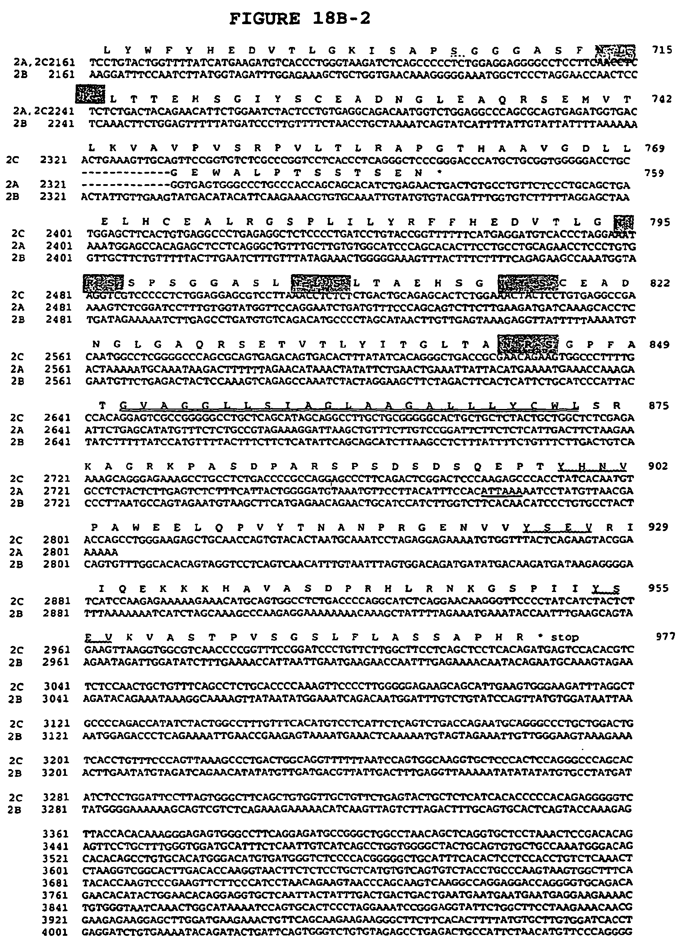

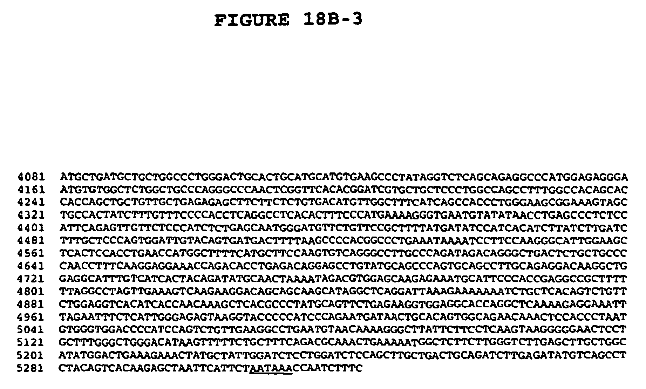

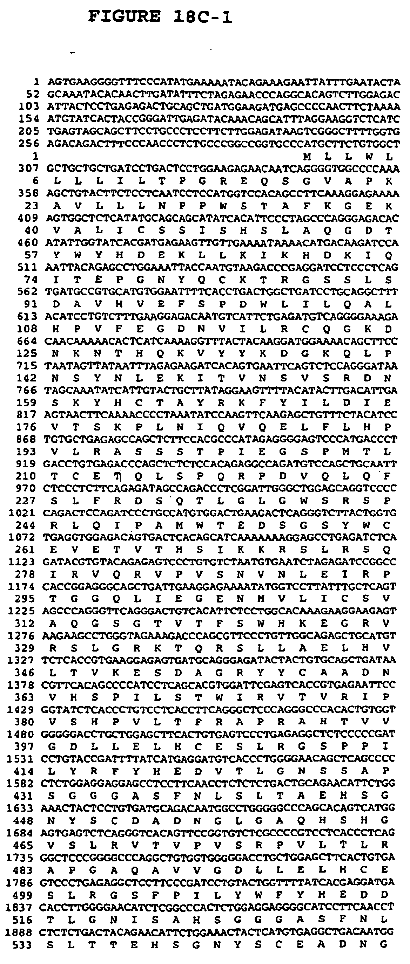

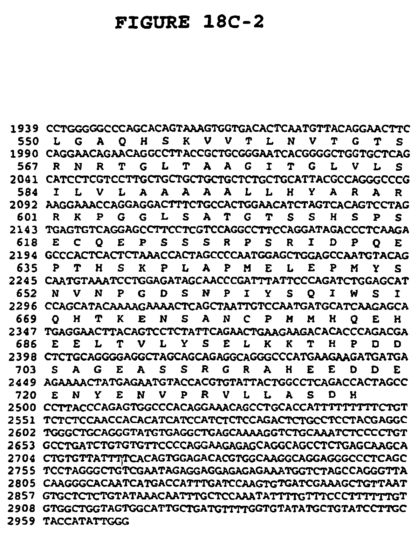

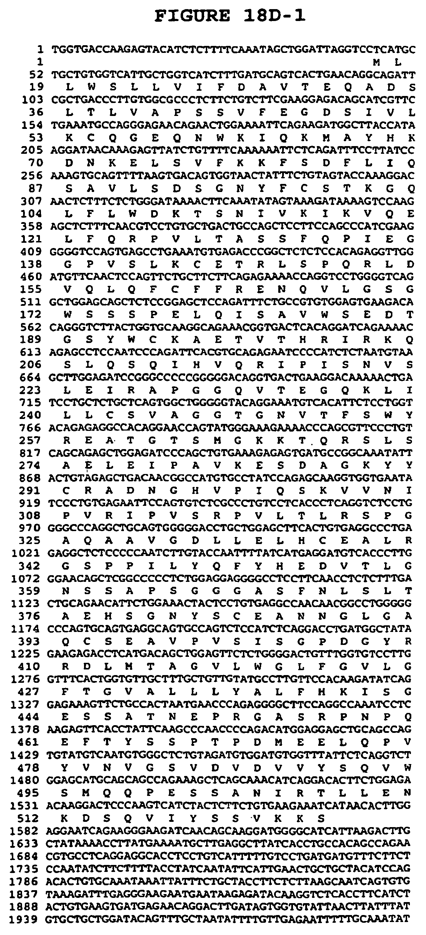

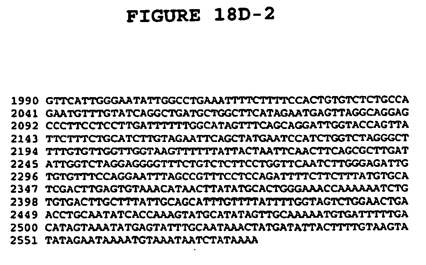

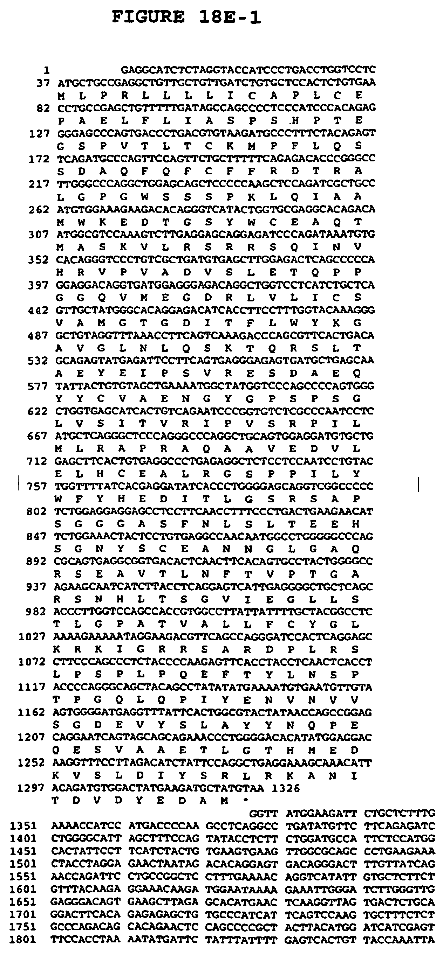

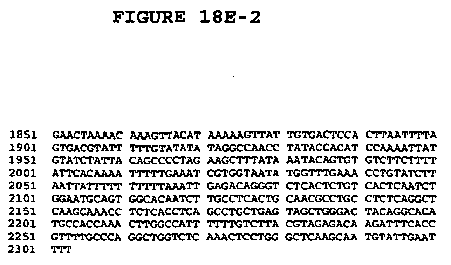

- **Figure 18A.**: IRTA1 cDNA and the amino acid sequence of the encoded IRTA1 protein.
- **Figures 18B-1-18B-3.**: IRTA2 cDNA and the amino acid sequence of the encoded IRTA2 protein.
- **Figures 18C-1-18C-2.**: IRTA3 cDNA and the amino acid sequence of the encoded IRTA3 protein.
- **Figures 18D-1-18D-2.**: IRTA4 cDNA and the amino acid sequence of the encoded IRTA4 protein.
- **Figures 18E-1-18E-2.**: IRTA5 cDNA and the amino acid sequence of the encoded IRTA5 protein.

### DETAILED DESCRIPTION OF THE INVENTION

The following standard abbreviations are used throughout the specification to indicate specific nucleotides: C=cytosine; A=adenosine; T=thymidine and G=guanosine.

This invention provides an isolated nucleic acid molecule which encodes immunoglobulin receptor, Immunoglobulin superfamily Receptor Translocation Associated, IRTA2, protein.

As used herein "Immunoglobulin Receptor Translocation Associated" genes, "IRTA" are nucleic acid molecules which encode novel immunoglobulin superfamily cell surface receptors in B cells which are important in B cell development, and whose abnormal expression, e.g. deregulated expression, perturbs cell surface B cell immunological responses and thus is involved in B cell malignancy, including lymphomagenesis.

Nucleic acid molecules encoding proteins designate "MUM-2" and "MUM-3" proteins in the First Series of Experiments are now called "*IRTA-1*" and *"IRTA-2"* genes, i.e. nucleic acid molecules which encode IRTA-1 and IRTA-2 proteins respectively. IRTA-3, -4 and -5 proteins are members of the same the immunoglobulin gene superfamily as are the IRTA-1 and IRTA-2 proteins.

In an embodiment of the above-described isolated nucleic acid molecule, the encoded IRTA protein is IRTA2 protein comprising the amino acid sequence set forth in Figures 18B-1-18B-3 (SEQ ID NO:3).

In another embodiment of any of the above-described isolated nucleic acid molecules, the nucleic acid molecule is DNA. In further embodiments, the DNA is cDNA. In additional embodiments, the DNA is genomic DNA. In another embodiment, the nucleic acid molecule is an RNA molecule.

In preferred embodiments of the isolated nucleic acid molecule, wherein the nucleic acid molecules encode human IRTA2, protein.

In another preferred embodiment, the isolated nucleic acid molecules are operatively linked to a promoter of DNA transcription. In yet another preferred embodiment of the isolated nucleic acid molecule, the promoter comprises a bacterial, yeast, insect, plant or mammalian promoter.

This invention provides a vector comprising any of the above-described isolated nucleic acid molecule encoding IRTA2 protein, including but not limited to mammalian IRTA2 protein , of which human and murine are preferred.

In an embodiment, the vector is a plasmid.

This invention provides a host cell comprising the above-described vector comprising any of the above-described isolated nucleic acid molecule encoding IRTA2 protein. Preferably, the isolated nucleic acid molecules in such vectors are operatively linked to a promoter of DNA transcription. In another embodiment of the host cell, the cell is selected from a group consisting of a bacterial cell, a plant cell, and insect cell and a mammalian cell.

Disclosed is a method of producing an IRTA polypeptide (protein) which comprises: (a) introducing a vector comprising an isolated nucleic acid which encodes an immunoglobulin receptor, Immunoglobulin superfamily Receptor Translocation Associated, IRTA, protein into a suitable host cell; and (b) culturing the resulting cell so as to produce the polypeptide.

The IRTA protein produced by the above-described method may be recovered and in a still further embodiment, may be purified either wholly or partially. In an embodiment the IRTA protein is IRTA2,

In a further embodiment, the IRTA2 proteins may be a mammalian protein. In still further embodiments, the mammalian protein may be a human or mouse IRTA2 protein.

IRTA genes (nucleic acid molecules encoding IRTA proteins IRTA1, IRTA2, IRTA3, IRTA4 and IRTA5) are useful for the production of the IRTA proteins encoded thereby. IRTA proteins are useful for production of antibodies; such antibodies are used as reagents for differential diagnosis of lymphoma subtypes in hematopathology. Antibodies directed against IRTA proteins and which bind specifically to IRTA proteins also have therapeutic uses, i.e. to specifically target tumor cells, which may be used and administered similarly to "Rituximab" (an anti-CD20 antibody), which is an antibody approved by the FDA for therapy of relapsed CD20-positive lymphomas (Foon K., Cancer J. 6(5):273). Anti-IRTA1, anti-IRTA2, anti-IRTA3, anti-IRTA4 and anti-IRTA5 antibodies are also useful markers for isolation of specific subsets of B cells in research studies of normal and tumor B cell biology. Moreover; anti-IRTA1, anti-IRTA2, anti-IRTA3, anti-IRTA4 and anti-IRTA5 antibodies are useful research reagents to experimentally study the biology of signaling in normal and tumor B cells.

Methods of introducing nucleic acid molecules into cells are well known to those of skill in the art. Such methods include, for example, the use of viral vectors and calcium phosphate co-precipitation. Accordingly, nucleic acid molecules encoding IRTA proteins IRTA1, IRTA2, IRTA3, IRTA4 and IRTA5 may be introduced into cells for the production of these IRTA proteins.

Numerous vectors for expressing the inventive protein IRTA2 may be employed. Such vectors, including plasmid vectors, cosmid vectors, bacteriophage vectors and other viruses, are well known in the art. For example, one class of vectors utilizes DNA elements which are derived from animal viruses such as bovine papilloma virus, polyoma virus, adenovirus, vaccinia virus, baculovirus, retroviruses (RSV, MMTV or MoMLV), Semliki Forest virus or SV40 virus. Additionally, cells which have stably integrated the DNA into their chromosomes may be selected by introducing one or more markers which allow for the selection of transfected host cells. The markers may provide, for example, prototrophy to an auxotrophic host, biocide resistance or resistance to heavy metals such as copper. The selectable marker gene can be either directly linked to the DNA sequences to be expressed, or introduced into the same cell by cotransformation.

Regulatory elements required for expression include promoter sequences to bind RNA polymerase and transcription initiation sequences for ribosome binding. Additional elements may also be needed for optimal synthesis of mRNA. These additional elements may include splice signals, as well as enhancers and termination signals. For example, a bacterial expression vector includes a promoter such as the lac promoter and for transcription initiation the Shine-Dalgarno sequence and the start codon AUG. Similarly, a eukaryotic expression vector includes a heterologous or homologous promoter for RNA polymerase II, a downstream polyadenylation signal, the start codon AUG, and a termination codon for detachment of the ribosome. Such vectors may be obtained commercially or assembled from the sequences described by methods well known in the art, for example the methods described above for constructing vectors in general.

These vectors may be introduced into a suitable host cell to form a host vector system for producing the inventive proteins. Methods of making host vector systems are well known to those skilled in the art.

Suitable host cells include, but are not limited to, bacterial cells (including gram positive cells), yeast cells, fungal cells, insect cells and animal cells. Suitable animal cells include, but are not limited to HeLa cells, Cos cells, CV1 cells and various primary mammalian cells. Numerous mammalian cells may be used as hosts, including, but not limited to, the mouse fibroblast cell NIH-3T3 cells, CHO cells, HeLa cells, Ltk- cells and COS cells. Mammalian cells may be transfected by methods well known in the art such as calcium phosphate precipitation, electroporation and microinjection.

This invention provides an isolated nucleic acid molecule comprising at least 15 contiguous nucleotides capable of specifically hybridizing with a unique sequence included within the sequence of the isolated nucleic acid molecule encoding IRTA2 protein, or fragment(s) thereof, having the amino acid sequence set forth in Figures 18B-1-18B-3. In other embodiments, the isolated nucleic acid molecules are labeled with a detectable marker. In still other embodiments of the isolated nucleic acid molecules, the detectable marker is selected from the group consisting of a radioactive isotope, enzyme, dye, biotin, a fluorescent label or a chemiluminescent label.

This invention provides a method for detecting a B cell malignancy or a type of B cell malignancy in a sample from a subject wherein the B cell malignancy comprises a 1q21 chromosomal rearrangement which comprises:
a) contacting the RNA sample obtained from the subject with a nucleic acid molecule of at least 15 contiguous nucleotides capable of specifically hybridizing with a unique sequence included within the sequence of an isolated RNA encoding human IRTA2protein , under conditions permitting hybridization of the RNA of step (a) with the nucleic acid molecule capable of specifically hybridizing with a unique sequence included within the sequence of an isolated RNA encoding human IRTA2 protein, wherein the nucleic acid molecule is labeled with a detectable marker; and b) detecting any hybridization in step (a), wherein detecion of hybridization indicates presence of B cell malignancy or a type of B cell malignancy in the sample.

Detection of hybridization of RNA encoding IRTA proteins will indicate that a malignancy is a B cell malignancy. More specifically, detection of hybridization of RNA encoding IRTA1 protein indicates that the B cell malignancy is a Mucosa-Associated-Lymphoid Tissue (MALT) B cell lymphoma. Detection of hybridization of RNA encoding IRTA4 and IRTA5 proteins indicate that the B cell malignancy is a mantle cell lymphoma. In an embodiment of the above-described method, the B cell malignancy comprises a 1q21 chromosomal rearrangement. One of skill will use the above-described method as a diagnostic aid in conjunction with other standard methods of detecting/diagnosing malignancies, e.g. pathology of a tumor sample, which may indicate lymphoma and the above-described method will then narrow the malignancy to a B cell lymphoma or more specifically to MALT) B cell lymphoma or a mantle cell lymphoma as discussed supra.

One of skill is familiar with known methods of detecting of hybridization nucleic acid molecules to nucleic acid oligonucleotides, i.e. nucleic acid probes encoding a protein of interest for diagnostic methods. The nucleic acid molecules encoding the IRTA proteins of the subject invention are useful for detecting B cell malignancy. One of skill will recognize that variations of the above-described method for detecting a B cell malignancy in a sample include, but are not limited to, digesting nucleic acid from the sample with restrictio enzymes and separating the nucleic acid molecule fragments so oobtained by size fractionation before hybridization.

In an embodiment of the above-described method for detecting a B cell malignancy in a sample from a subject, wherein the detectable marker is radioactive isotope, enzyme, dye, biotin, a fluorescent label or a chemiluminescent label. In a preferred embodiment, the B cell malignancy is selected from the group consisting of B cell lymphoma, multiple myeloma, Burkitt's lymphoma, marginal zone lymphoma, diffuse large cell lymphoma and follicular lymphoma cells. In a further embodiemnt, the B cell lymphoma is Mucosa-Associated-Lymphoid Tissue B cell lymphoma (MALT). In another preferred embodiment, the B cell lymphoma is non-Hodgkin's lymphoma.

This invention provides an antisense oligonucleotide having a sequence capable of specifically hybridizing to an mRNA molecule encoding a human IRTA2 protein so as to prevent overexpression of the mRNA molecule.

In a preferred embodiment of the antisense oligonucleotide, the ITRA protein is human IRTA2. In a further embodiment of any of the above-described oligonucleotides of nucleic acid molecules encoding the IRTA2 protein, the nucleic acid may be genomic DNA or cDNA.

One of skill is familiar with conventional techniques for nucleic acid hybridization of oligonucleotides, e.g. Ausubel, F.M. et al. Current Protocols in Molecular Biology, (John Wiley & Sons, New York, 1998), for example stringent conditions of 65°C in the presence of an elevated salt concentration. Such conditions are used for completely complementary nucleic acid hybridization, whereas conditions that are not stringent are used for hybridization of nucleic acids which are not totally complementary.

As used herein, the phrase "specifically hybridizing" means the ability of a nucleic acid molecule to recognize a nucleic acid sequence complementary to its own and to form double-helical segments through hydrogen bonding between complementary base pairs. As used herein, a "unique sequence" is a sequence specific to only the nucleic acid molecules encoding the IRTA2 , protein. Nucleic acid probe technology is well known to those skilled in the art who will readily appreciate that such probes may vary greatly in length and may be labeled with a detectable label, such as a radioisotope or fluorescent dye, to facilitate detection of the probe. Detection of nucleic acid molecules encoding the IRTA2 protein is useful as a diagnostic test for any disease process in which levels of expression of the corresponding IRTA2 protein is altered. DNA probe molecules are produced by insertion of a DNA molecule which encodes mammalian IRTA2 protein or fragments thereof into suitable vectors, such as plasmids or bacteriophages, followed by insertion into suitable bacterial host cells and replication and harvesting of the DNA probes, all using methods well known in the art. For example, the DNA may be extracted from a cell lysate using phenol and ethanol, digested with restriction enzymes corresponding to the insertion sites of the DNA into the vector (discussed herein), electrophoresed, and cut out of the resulting gel. The oligonucleotide probes are useful for 'in situ' hybridization or in order to locate tissues which express this IRTA gene family, and for other hybridization assays for the presence of these genes (nucleic acid molecules encoding the IRTA2 protein) or their mRNA in various biological tissues. In addition, synthesized oligonucleotides (produced by a DNA synthesizer) complementary to the sequence of a DNA molecule which encodes an IRTA2 protein are useful as probes for these genes, for their associated mRNA, or for the isolation of related genes by homology screening of genomic or cDNA libraries, or by the use of amplification techniques such as the Polymerase Chain Reaction.

This invention provides a purified. IRTA2 protein comprising the amino acid sequence set forth in Figures 18B-1-18B-3 (SEQ ID NO:3). In an embodiment of the purified IRTA2 protein, the IRTA2 protein is human IRTA2.

In order to facilitate an understanding of the Experimental Details section which follows, certain frequently occurring methods and/or terms are best described in Sambrook, et al.(1989) and Harlow & Lane, Antibodies: A Laboratory Manual, Cold Spring Harbor Laboratories, Cold Spring Harbor, NY: 1988.

This invention provides an antibody/antibodies directed to an epitope of a purified IRTA2 protein, or fragment(s) thereof, having the amino acid sequence set forth in Figures 18B-1-18B-3.

As used herein, the term "antibody" includes, but is not limited to, both naturally occurring and non-naturally occurring antibodies. Specifically, the term "antibody" includes polyclonal and monoclonal antibodies, and binding fragments thereof. Furthermore, the term "antibody" includes chimeric antibodies and wholly synthetic antibodies, and fragments thereof. The polyclonal and monoclonal antibodies may be "purified" which means the polyclonal and monoclonal antibodies are free of any other antibodies. As used herein, partially purified antibody means an antibody composition which comprises antibodies which specifically bind to the IRTA protein of the subject invention, and consists of fewer protein impurities than does the serum from which the antibodies are derived. A protein impurity is a protein other than the antibodies specific for the IRTA protein of the subject invention. For example, the partially purified antibodies may be an IgG preparation.

Polyclonal antibodies (anti-IRTA antibodies) may be produced by injecting a host animal such as rabbit, rat, goat, mouse or other animal with the immunogen(s) of this invention, e.g. a purified human IRTA2 described infra. The sera are extracted from the host animal and are screened to obtain polyclonal antibodies which are specific to the immunogen. Methods of screening for polyclonal antibodies are well known to those of ordinary skill in the art such as those disclosed in Harlow & Lane, Antibodies: A Laboratory Manual, (Cold Spring Harbor Laboratories, Cold Spring Harbor, NY: 1988).

The anti-IRTA monoclonal antibodies of the subject invention may be produced by immunizing for example, mice with an immunogen (the IRTA polypeptides or fragments thereof as described herein). The mice are inoculated intraperitoneally with an immunogenic amount of the above-described immunogen and then boosted with similar amounts of the immunogen. Spleens are collected from the immunized mice a few days after the final boost and a cell suspension is prepared from the spleens for use in the fusion.

Hybridomas may be prepared from the splenocytes and a murine tumor partner using the general somatic cell hybridization technique of Kohler, B. and Milstein, C., Nature (1975) 256: 495-497. Available murine myeloma lines, such as those from the American Type Culture Collection (ATCC), 10801 University Boulevard, Manassas, VA 20110-2209, USA, may be used in the hybridization. Basically, the technique involves fusing the tumor cells and splenocytes using a fusogen such as polyethylene glycol. After the fusion the cells are separated from the fusion medium and grown in a selective growth medium, such as HAT medium, to eliminate unhybridized parent cells. The hybridomas may be expanded, if desired, and supernatants may be assayed by conventional immunoassay procedures, for example radioimmunoassay, using the immunizing agent as antigen. Positive clones may be characterized further to determine whether they meet the criteria of the invention antibodies.

Hybridomas that produce such antibodies may be grown in vitro or in vivo using known procedures. The monoclonal antibodies may be isolated from the culture media or body fluids, as the case may be, by conventional immunoglobulin purification procedures such as ammonium sulfate precipitation, gel electrophoresis, dialysis, chromatography, and ultrafiltration, if desired.

In the practice of the subject invention any of the above-described antibodies may be labeled with a detectable marker. In one embodiment, the labeled antibody is a purified labeled antibody. The term "antibody" includes, by way of example, both naturally occurring and non-naturally occurring antibodies. Specifically, the term "antibody" includes polyclonal and monoclonal antibodies, and fragments thereof. Furthermore, the term "antibody" includes chimeric antibodies and wholly synthetic antibodies, and fragments thereof. A "detectable moiety" which functions as detectable labels are well known to those of ordinary skill in the art and include, but are not limited to, a fluorescent label, a radioactive atom, a paramagnetic ion, biotin, a chemiluminescent label or a label which may be detected through a secondary enzymatic or binding step. The secondary enzymatic or binding step may comprise the use of digoxigenin, alkaline phosphatase, horseradish peroxidase, ß-galactosidase, fluorescein or steptavidin/biotin. Methods of labeling antibodies are well known in the art.

Methods of recovering serum from a subject are well known to those skilled in the art. Methods of partially purifying antibodies are also well known to those skilled in the art, and include, by way of example, filtration, ion exchange chromatography, and precipitation.

The polyclonal and monoclonal antibodies of the invention may be labeled with a detectable marker. In one embodiment, the labeled antibody is a purified labeled antibody. The detectable marker may be, for example, a radioactive or fluorescent marker. Methods of labeling antibodies are well known in the art.

Determining whether the polyclonal and monoclonal antibodies of the subject invention bind to cells, e.g. cancer cells, expressing an IRTA protein and form a complex with one or more of the IRTA protein(s) described herein, or fragments thereof, on the surface of said cells, may be accomplished according to methods well known to those skilled in the art. In the preferred embodiment, the determining is accomplished according to flow cytometry methods.

The antibodies of the subject invention may be bound to an insoluble matrix such as that used in affinity chromatography. Cells which form a complex, i.e. bind, with the immobilized polyclonal or monoclonal antibody may be isolated by standard methods well known to those skilled in the art. For example, isolation may comprise affinity chromatography using immobilized antibody.

Alternatively, the antibody may be a free antibody. In this case, isolation may comprise cell sorting using free, labeled primary or secondary antibodies. Such cell sorting methods are standard and are well known to those skilled in the art.

This invention provides an antibody directed to a purified IRTA2 protein. In a preferred embodiment of the anti-IRTA antibody the IRTA protein is human IRTA protein. The IRTA protein may be any mammalian IRTA protein, including a murine IRTA protein. In a further embodiment of any the above-described antibodies, the antibody is a monoclonal antibody. In another embodiment, the monoclonal antibody is a murine monoclonal antibody or a humanized monoclonal antibody. As used herein, "humanized" means an antibody having characteristics of a human antibody, such antibody being non-naturally occurring, but created using hybridoma techniques wherein the antibody is of human origin except for the antigen determinant portion, which is murine. In yet another embodiment, the antibody is a polyoclonal antibody.

In preferred embodiments, any of the antibodies of the subject invention may be conjugated to a therapeutic agent. In further preferred embodiments, the therapeutic agent is a radioisotope, toxin, toxoid, or chemotherapeutic agent. The conjugated antibodies of the subject invention may be administered to a subject having a B cell cancer in any of the methods provided below.

This invention provides a pharmaceutical composition comprising an amount of the antibody directed to an IRTA2 protein effective to bind to cancer cells expressing an IRTA2 protein selected from the group consisting of human so as to prevent growth of the cancer cells and a pharmaceutically acceptable carrier. The anti-IRTA antibody may be directed to an epitope of an IRTA2 protein. The IRTA proteins may be human or mouse IRTA proteins.

In preferred embodiments of the above-described pharmaceutical composition, the cancer cells are selected from the group consisting of B cell lymphoma, multiple myeloma, a mantle cell lymphoma, Burkitt's lymphoma, marginal zone lymphoma, diffuse large cell lymphoma and follicular lymphoma cells. In another preferred embodiment of the pharmaceutical composition, the B cell lymphoma cells are Mucosa-Associated-Lymphoid Tissue B cell lymphoma (MALT) cells. In another preferred embodiment of the pharmaceutical composition, the B cell lymphoma cells are non-Hodgkin's lymphoma cells. This invention provides as defined in the claims a pharmaceutical composition comprising an amount of the above-described oligonucleotides effective to prevent overexpression of a human IRTA2 protein and a pharmaceutically acceptable carrier capable. In a preferred embodiment of the pharmaceutical composition the oligonucleotide is a nucleic acid molecule which encodes an IRTA2 protein.

The IRTA protein may be a human or mouse IRTA protein.

As used herein, "malignant" means capable of metastasizing. As used herein, "tumor cells" are cells which originate from a tumor, i.e., from a new growth of different or abnormal tissue..The tumor cells and cancer cells may exist as part of the tumor mass, or may exist as free-floating cells detached from the tumor mass from which they originate.

As used herein, malignant cells include, but are in no way limited to, B cell lymphoma, multiple myeloma, Burkitt's lymphoma, mantle cell lymphoma, marginal zone lymphoma, diffuse large cell lymphoma and follicular lymphoma. The B cell lymphoma is Mucosa-Associated-Lymphoid Tissue B cell lymphoma (MALT) or is non-Hodgkin's lymphoma.

As used herein, "subject" is any animal or artificially modified animal. Artificially modified animals include, I I but are not limited to, SCID mice with human immune systems. In a preferred embodiment, the subject is a human.

This invention provides a method of diagnosing B cell malignancy which comprises a 1q21 chromosomal rearrangement in a sample from a subject which comprises:
a) contacting the sample obtained from the subject with an antibody directed to a purified IRTA2 protein capable of specifically binding with a human IRTA2 .protein on a cell surface of a cancer cell under conditions permitting binding of the antibody with humanIRTA2 protein on the cell surface of the cancer cell, wherein the antibody is labeled with a detectable marker; and b) detecting any binding in step (a), wherein detecion of binding indicates a diagnosis of B cell malignancy in the sample.

In an embodiment of the above-described method of diagnosing B cell malignancy, the IRTA protein is IRTA2.

In another embodiment of the method the IRTA protein is human or mouse IRTA protein. In a further embodiment IRTA protein is purified. In a preferred embodiment of this method, the B cell malignancy is selected from the group consisting of B cell lymphoma, multiple myeloma, Burkitt's lymphoma, marginal zone lymphoma, diffuse large cell lymphoma and follicular lymphoma. In yet another embodiment of this method, the B cell lymphoma is Mucosa-Associated-Lymphoid Tissue B cell lymphoma (MALT). In another preferred embodiment of this method, the B cell lymphoma is non-Hodgkin's lymphoma.

Disclosed is a method of detecting human IRTA protein in a sample which comprises: a) contacting the sample with any of any of the above-described anti-IRTA antibodies under conditions permitting the formation of a complex between the antibody and the IRTA in the sample; and b) detecting the complex formed in step (a), thereby detecting the presence of human IRTA in the sample. In an embodiment the IRTA protein detected may be an IRTA2, protein, having an amino acid sequence set forth in Figures 18B-1-18B-3. As described hereinabove detection of the complex formed may be achieved by using antibody labeled with a detectable marker and determining presence of labeled complex.

Detecting human IRTA protein in a sample from a subject is another method of diagnosing B cell malignancy in a subject. In an embodiment of this method of diagnosis, the B cell malignancy is selected from the group consisting of B cell lymphoma, multiple myeloma, Burkitt's lymphoma, marginal zone lymphoma, diffuse large cell lymphoma and follicular lymphoma. In yet another embodiment of this method, the B cell lymphoma is Mucosa-Associated-Lymphoid Tissue B cell lymphoma (MALT). In another preferred embodiment of this method, the B cell lymphoma is non-Hodgkin's lymphoma.

This invention provides a use for treating a subject having a B cell cancer which comprises administering to the subject an amount of anti-IRTA antibody effective to bind to cancer cells expressing an IRTA protein so as to prevent growth of the cancer cells and a pharmaceutically acceptable carrier, thereby treating the subject. Growth and proliferation of the cancer cells is thereby inhibited amd the cancer cells die. In an embodiment of the above-described use, the IRTA protein is human IRTA2.

In a preferred embodiment of the above-described method of treating a subject having a B cell cancer, the anti-IRTA antibody is a monoclonal antibody. In another embodiment of the use, the monoclonal antibody is a murine monoclonal antibody or a humanized monoclonal antibody. The antibody may be a chimeric antibody. In a further embodiment, the anti-IRTA antibody is a polyoclonal antibody. In an embodiment, the polyclonal antibody may be a murine or human polyclonal antibody. In a preferred embodiment, the B cell cancer is selected from the group consisting of B cell lymphoma, multiple myeloma, Burkitt's lymphoma, mantle cell lymphoma marginal zone lymphoma, diffuse large cell lymphoma and follicular lymphoma. In another preferred embodiment, the B cell lymphoma is Mucosa-Associated-Lymphoid Tissue B cell lymphoma (MALT). In a further preferred embodiment, the B cell lymphoma is non-Hodgkin's lymphoma. In a preferred embodiment of the above-described method of treating a subject having a B cell cancer, administration of the amount of anti-IRTA antibody effective to bind to cancer cells expressing an IRTA protein is intravenous, intraperitoneal, intrathecal, intralymphatical, intramuscular, intralesional, parenteral, epidural, subcutaneous; by infusion, liposome-mediated delivery, aerosol delivery; topical, oral, nasal, anal, ocular or otic delivery. In another preferred embodiment of the above-described methods, the anti-IRTA antibody may be conjugated to a therapeutic agent. In further preferred embodiments, the therapeutic agent is a radioisotope, toxin, toxoid, or chemotherapeutic agent.

This invention provides a use for treating a subject having a B cell cancer which comprises administering to the subject an amount of an antisense oligonucleotide having a sequence capable of specifically hybridizing to an mRNA molecule encoding a human IRTA2 protein so as to prevent overexpression of the human IRTA2 protein, so as to arrest cell growth or induce cell death of cancer cells expressing IRTA protein(s) and a pharmaceutically acceptable carrier.

In an embodiment of the above-described use for treating a subject having a B cell cancer, the IRTA protein is human IRTA2. In a preferred embodiment, B cell cancer is selected from the group consisting of B cell lymphoma, multiple myeloma, Burkitt's lymphoma, marginal zone lymphoma, diffuse large cell lymphoma and follicular lymphoma. In another preferred embodiment, the B cell lymphoma is Mucosa-Associated-Lymphoid Tissue B cell lymphoma (MALT). In a yet another preferred embodiment, the B cell lymphoma is non-Hodgkin's lymphoma. In embodiments of any of the above-described oligonucleotides of nucleic acid molecules encoding the IRTA2 protein, the nucleic acid may be genomic DNA or cDNA. In a further preferred embodiment of the above-described use for treating a subject having a B cell cancer, administration of the amount of oligonucleotide of effective to prevent overexpression of the human IRTA protein is intravenous, intraperitoneal, intrathecal, intralymphatical, intramuscular, intralesional, parenteral, epidural, subcutaneous; by infusion, liposome-mediated delivery, aerosol delivery; topical, oral, nasal, anal, ocular or otic delivery. In an embodiment of the above-described uses, the oligonucleotide may be conjugated to a therapeutic agent. In further preferred embodiments, the therapeutic agent is a radioisotope, toxin, toxoid, or chemotherapeutic agent.

The invention also provides a pharmaceutical composition comprising either an effective amount of the oligonucleotides or of the antibodies described above and a pharmaceutically acceptable carrier. In the subject invention an "effective amount" is any amount of an oligonucleotide or an antibody which, when administered to a subject suffering from a disease or abnormality against which the oligonucleotide or antibody are effective, causes reduction, remission, or regression of the disease or abnormality. In the practice of this invention the "pharmaceutically acceptable carrier" is any physiological carrier known to those of ordinary skill in the art useful in formulating pharmaceutical compositions.

Pharmaceutically acceptable carriers are well known to those skilled in the art and include, but are not limited to, 0.01-0.1M and preferably 0.05M phosphate buffer or 0.8% saline. Additionally, such pharmaceutically acceptable carriers may be aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Intravenous vehicles include fluid and nutrient replenishers, electrolyte replenishers such as those based on Ringer's dextrose, and the like. Preservatives and other additives may also be present, such as, for example, antimicrobials, antioxidants, chelating agents, inert gases and the like.

In one preferred embodiment the pharmaceutical carrier may be a liquid and the pharmaceutical composition would be in the form of a solution. In another equally preferred embodiment, the pharmaceutically acceptable carrier is a solid and the composition is in the form of a powder or tablet. In a further embodiment, the pharmaceutical carrier is a gel and the composition is in the form of a suppository or cream. In a further embodiment the compound may be formulated as a part of a pharmaceutically acceptable transdermal patch.

A solid carrier can include one or more substances which may also act as flavoring agents, lubricants, solubilizers, suspending agents, fillers, glidants, compression aids, binders or tablet-disintegrating agents; it can also be an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, polyvinylpyrrolidine, low melting waxes and ion exchange resins.

Liquid carriers are used in preparing solutions, suspensions, emulsions, syrups, elixirs and pressurized compositions. The active ingredient can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the carrier can also be an oily ester such as ethyl oleate and isopropyl myristate. Sterile liquid carriers are useful in sterile liquid form compositions for parenteral administration. The liquid carrier for pressurized compositions can be halogenated hydrocarbon or other pharmaceutically acceptable propellent.

Liquid pharmaceutical compositions which are sterile solutions or suspensions can be utilized by for example, intramuscular, intrathecal, epidural, intraperitoneal or subcutaneous infection. Sterile solution can also be administered intravenously. The compounds may be prepared as a sterile solid composition which may be dissolved or suspended at the time of administration using sterile water, saline, or other appropriate sterile injectable medium. Carriers are intended to include necessary and inert binders, suspending agents, lubricants, flavorants, sweeteners, preservatives, dyes, and coatings.

The pharmaceutical composition comprising the oligonucleotide or the antibody can be administered orally in the form of a sterile solution or suspension containing other solutes or suspending agents, for example, enough saline or glucose to make the solution isotonic, bile salts, acacia, gelatin, sorbitan monoleate, polysorbate 80 (oleate esters of sorbitol and its anhydrides copolymerized with ethylene oxide) and the like.

The pharmaceutical composition comprising the oligonucleotide or the antibody can also be administered orally either in liquid or solid composition form. Compositions suitable for oral administration include solid forms, such as pills, capsules, granules, tablets, and powders, and liquid forms, such as solutions, syrups, elixirs, and suspensions. Forms useful for parenteral administration include sterile solutions, emulsions, and suspensions.

Optimal dosages to be administered may be determined by those skilled in the art, and will vary with the particular inhibitor in use, the strength of the preparation, the mode of administration, and the advancement of the disease condition or abnormality. Additional factors depending on the particular subject being treated will result in a need to adjust dosages, including subject age, weight, gender, diet, and time of administration.

This invention will be better understood from the Experimental Details which follow. However, one skilled in the art will readily appreciate that the specific methods and results discussed are merely illustrative of the invention as described more fully in the claims which follow thereafter.

### EXPERIMENTAL DETAILS

### First Series of Experiments

Molecular analysis of chromosomal translocations associated with multiple myeloma (MM) has indicated that the pathogenesis of this malignancy may be heterogeneous, being associated with several distinct oncogenes including BCL-1, MUM-1 and FGFR3. Structural abnormalities of chromosome 1q21, including translocations with chromosome 14q32, represent frequent cytogenetic aberrations associated with multiple myeloma. In order to identify the genes involved in these translocations, the breakpoint regions corresponding to both derivatives of a t(1;14)(q21;q32) detectable in the FR4 human plasmacytoma cell line were cloned. Analysis of the breakpoint sequences showed that they involved a reciprocal recombination between the Immunoglobulin heavy chain (IgH) locus on 14q32 and unknown sequences on 1q21. The normal locus corresponding ot hte 1q21 region involved in the translocation was cloned and athe genes adjacent to the breakpoint region were identified by an exon-trapping strategy. Two genes were found, located within a 20 Kb distance from each other, in the region spanning the breakpoint on 1q21. The first gene, called MUM-2 (multiple myeloma-2) is expressed as a 2.5 Kb mRNA transcript detectable in spleen and lymph nodes. Cloning and sequencing of the full-length MUM-2cDNA predicts a 515 amino acid cell surface glycoprotein containing four extracellular Ig-type domains, a transmembrane and a cytoplasmic domain and sharing a 37% identity (51%homology) with Fc gamma receptor I over its first three extracellular domains. In FR4 cells, the translocation breakpoints interrupt the MUM-2 coding domain and juxtapose it to the IgH locus in the same transcriptional orientation. As a consequence, structurally abnormal FR4-specific MUM-2 transcripts (3.0, 5.2 and 6.0 Kb) in lymph nodes and spleen and encodes a protein with an extracellular domain containing six Ig-type domains homologous to members of the Fc gamma and Ig-type adhesion receptor families. The structure of the MUM-2 and MUM-3 genes and their direct involvement in a MM-associated translocation suggest that these genes code for novel cell surface receptors important for normal lymphocyte function and B cell malignancy.

### Second Series of Experiments

### Experimental Procedures

### Cell Lines

The MM cell lines used in this study (FR4, U266, JJN3, EJM, SKMM1, RPMI-8226, XG1, XG2, XG4, XG6, XG7) have been previously reported (Tagawa et al., 1990), (Jernberg et al., 1987), (Hamilton et al., 1990; Jackson et al., 1989), (Eton et al., 1989), (Zhang et al., 1994). The FR4 cell line was established in the laboratory of one of the authors (S.T). The U266, JJN3, and EJM cell lines were gifts from Dr. K. Nilsson (University of Uppsala, Uppsala, Sweden) and the SKMM-1 cell line was a gift of A.N. Houghton (Memorial Sloan Kettering Cancer Center, New York, NY). The five XG cell lines were obtained from Dr. Bernard Klein and cultured in the presence of 1 ng/ml human recombinant IL-6 as described previously (Zhang et al., 1994). The BL cell lines with 1q21 abnormalities have been previously described (Polito et al., 1995), (Magrath et al., 1980) and were grown in RPMI, 10% FCS.

### Genomic and cDNA library screening and DNA sequence analysis

Two genomic libraries were constructed from FR4 genomic DNA either by BamHI complete digestion or by Sau3AI partial digestion and subsequent ligation of gel-purified fractions into the 1DASH-II phage vector (Stratagene). The BamHI library was screened with a 4.2 kb XhoI-BamHI probe derived from the Ca locus and the Sau3AI library was screened with a 5'Sa probe previously described (Bergsagel et al., 1996). A human placental DNA library (Stratagene) was screened with probe 1.0EH (Figures 8A-8C) to obtain the germline 1q21 locus. Library screening and plaque isolation were preformed according to established procedures (Sambrook et al., 1989). *IRTA1* and *IRTA2* cDNA clones were isolated from an oligo-dT/random-primed cDNA library constructed from normal human spleen RNA (Clontech). The *IRTA1* cDNA probe used for library screening was obtained from RT-PCR of human spleen cDNA using primers flanking exons 1 and 3. DNA sequencing was preformed on an ABI 373 automated sequencer (Applied Biosystems). Sequence homology searches were carried out through the BLAST e-mail server at the National Center for Biotechnology Information, Bethesda, MD.

### PAC and YAC isolation and exon trapping

Human PAC clones were obtained by screening a human PAC library spotted onto nylon membranes (Research Genetics), with the 1.0 EH probe (Figures 8A-8C). The Zeneca (formerly ICI) human YAC library (Anand et al., 1990) obtained from the United Kingdom Human Genome Mapping Resource Center (UK-HGMP) was screened using a PCR-based pooling strategy. Exon trapping was performed using the exon trapping system (Gibco BRL), according to the manufacturer's instructions.

### Isolation of PAC/YAC end clones, pulsed-field gel electrophoresis (PFGE) and fluorescence in situ hybridization (FISH) analysis

PAC DNA extraction was performed according to standard alkaline lysis methods (Drakopoli N et al., 1996). A vectorette-PCR method was used to isolate PAC and YAC end probes (Riley et al., 1990), as previously described (Iida et al, 1996). PFGE analysis was performed according to standard protocols (Drakopoli N et al., 1996) using the CHEF Mapper system (BioRad, Hercules, CA). Biotin labeling of PAC DNA, chromosome preparation and FISH were performed as previously described (Rao et al., 1993).

### Southern and Northern blot analyses, RACE and RT-PCR

Southern and northern blot analyses were performed as described previously (Neri et al, 1991). For Northern blot analyses total RNA was prepared by the guanidium thiocyanate method and poly(A) RNA was selected using poly(T)-coated beads (Oligotex Kit by Quigen). For Northern blots, 2 mg of poly (A) RNA were loaded per lane. Multiple tissue Northern filters were obtained from Clontech. RACE was performed using the Marathon cDNA Amplification kit (Clontech) and Marathon-Ready spleen cDNA. First strand cDNA synthesis was performed using the Superscript RT-PCR system (Gibco BRL)

### In situ hybridization

Digoxigenin-containing antisense and sense cRNA probes were transcribed with T3 and T7 RNA polymerase, respectively, from linearized pBluescript KS+ plasmids containing coding region of cDNAs (nucleotides 62 to 1681 of IRTA1 and 18 to 2996 of IRTA2.) Hyperplastic human tonsillar tissue surgically resected from children in Babies' Hospital, Columbia Presbyterian Medical Center was snap frozen in powdered dry ice. Cryostat sections were stored for several days at -80 degrees C prior to processing. Non-radioactive *in situ* hybridization was performed essentially as described (Frank et al., 1999), except that fixation time in 4% paraformaldehyde was increased to 20 minutes, and proteinase K treatment was omitted. The stringency of hybridization was 68 degrees C, in 5X SSC, 50% formamide. Alkaline phosphatase-conjugated anti-digoxigenin antibody staining was developed with BCIP/NBT substrate.

### Transfection, immunoprecipitation and Western Blotting

293 cells (ATCC), grown in DMEM, 10% FCS were transiently transfected, according to the standard calcium phosphate method, with pMT2T and pMT2T-IRTA1/Ca transient expression constructs. The latter was generated using the *IRTA1*/*Ca* RT-PCR product from FR4. Cells (2x10⁶ of transfectants and 2x10⁷ of remaining cell lines) were solubilized in Triton X-100 lysis buffer (150 mM NaCl, 10 mM Tris-HCl [pH 7.4], 1% Tx-100, 0.1% BSA) in the presence of a protease inhibitors coctail (Roche Biochemicals).

Lysates were incubated at 4°C for 2 hours with 4 mg/ml of the monoclonal antibody #117-332-1 (Yu et al., 1990) (Tanox Biosystems, Inc, Houston, Texas) that was raised against the extracellular portion of the IgA membrane peptide. Immune complexes were isolated with protein G-Sepharose (Pharmacia) prior to electrophoresis on 10-20% Tris-HCl gradient gels (Biorad) and immunobloting, using 15 mg/ml of the #117-332-1 antibody. Results were visualized by ECL (Amersham).

### RESULTS

### Molecular Cloning of the t(1;14)(q21;q32)

Chromosomal translocations involving the Ig heavy-chain (IGH) locus often occur within or near IgH switch regions as a result of "illegitimate" switch recombination events (Dalla-Favera et al., 1983; Chesi et al., 1996; Chesi et al., 1998). The breakpoints can be detected by Southern-blot hybridization assays as rearranged alleles in which the IGH constant (C_{H}) region sequences have lost their syntenic association with IGH joining (J_{H}) and 5' switch region (S) sequences (Dalla-Favera et al., 1983; Neri et al., 1988; Neri et al., 1991; Bergsagel et al., 1996). This assay has led to the identification of several chromosomal partners for the IgH locus in B-NHL and MM (Taub et al., 1982; Dalla-Favera et al., 1983; Neri et al., 1988; Neri et al., 1991; Ye et al., 1993; Chesi et al., 1996; Richelda et al., 1997; Iida et al., 1997; Dyomin et al., 1997; Dyomin et al., 2000). We employed the same strategy in order to clone the 1q21 breakpoint region in FR4, a myeloma cell line carrying a t(1;14)(q21;q32), as determined by cytogenetic analysis (Tagawa et al., 1990; Taniwaki M, unpublished results). Two "illegitimately" rearranged fragments were identified within the Cα heavy-chain locus in FR4 by Southern blot hybridization analysis (data not shown), and were cloned from phage libraries constructed from FR4 genomic DNA. Restriction mapping, Southern blot hybridization and partial nucleotide sequencing of two genomic phages (clones λ FR4B-5 and λ FR4S-a, Figure 8A) demonstrated that they contained the chromosomal breakpoints of a reciprocal balanced translocation between the *Cα*₁ locus on 14q32 and non-IGH sequences. A probe (1.0EH) representing these non-IgH sequences (Figure 8A) was then used to clone the corresponding normal genomic locus from phage, P1 artificial chromosome (PAC), and yeast artificial chromosome (YAC) human genomic libraries. Fluorescence *in situ* hybridization (FISH) analysis of normal human metaphase spreads using the 100-kb non-chimaeric PAC clone 49A16 which spans the breakpoint region (see below, Figure 13), identified the partner chromosomal locus as derived from band 1q21 (Figure 8C). Mapping to a single locus within chromosome 1 was confirmed by hybridization of two non-repetitive probes to DNA from a somatic-cell hybrid panel representative of individual human chromosomes (data not shown). These results were consistent with the cloning of sequences spanning the t(1;14)(q21;q32) in FR4.

Sequence analysis of the breakpoint regions on the derivative chromosomes and alignment with the germline 14q32 and 1q21 loci revealed that the breakpoint had occurred in the intron between the CH3 and the transmembrane exon of *Cα*₁ on chromosome 14. Although the breakpoint region was devoid of recombination signal sequences (RSS) or switch signal sequences (Kuppers et al., 1999), the sequence CTTAAC (underlined on Figure 8B) was present in both germline chromosomes 14 and 1 at the breakpoint junction. One copy of this sequence was present in each of the derivative chromosomes, with a slight modification in the der(1) copy (point mutation in the last nucleotide: C to G). The nucleotides AT preceding CTTAAC on chromosome 1 were also present in both derivative chromosomes (Figure 8B). The translocation did not result in any loss of chromosome 1 sequences. On the other hand, in the chromosome 14 portion of der(1) we observed two deletions upstream of the breakpoint junction: a 16 nucleotide deletion (GGCACCTCCCCTTAAC) and a 4 nucleotide deletion (TGCA) 6 nucleotides upstream (Figure 8B). These observations indicate that the t(1;14)(q21;q32) in FR4 cells represents a balanced reciprocal translocation possibly facilitated by the presence of homologous sequences (CTTAAC) on both chromosomes.

### The 1q21 breakpoint region contains genes coding for novel members of the Immunoglobulin Receptor Superfamily

We next investigated whether the region of chromosome 1q21 spanning the translocation breakpoint in FR4 contains a transcriptional unit. DNA from partially overlapping PAC clones 49A16 and 210K22 (Figure 13) was "shotgun" cloned in plasmids, sequenced and analyzed for homology to known genes in human genome databases. In parallel, candidate genes on the 49A16 PAC were sought by an exon trapping strategy (Church et al., 1994).

Mapping of the candidate exons on the 1q21 genomic clones revealed that the FR4 breakpoint had occurred between two trapped exons (see below, Figure 13), which belonged to the same transcript since they could be linked by RT-PCR using spleen RNA. This RT-PCR product was then used as a probe to screen a spleen cDNA library in order to isolate full-length clones corresponding to this transcript. Two sets of cDNA clones were identified, belonging to two distinct transcripts and sharing a 76% mRNA sequence identity within the 443 bp probe region. Full length cDNA clones for both transcripts were obtained by rapid amplification of cDNA ends (RACE) on human spleen cDNA that generated 5' and 3' extension products.

The schematic structure of the cDNA representing the first transcript is depicted in Figure 9A. Alternate usage of three potential polyadenylation sites in its 3' untranslated region gives rise to three mRNA species of 2.6, 2.7 and 3.5 kb, encoding the same putative 515-amino acid protein (Figure 9A). The predicted features of this protein include a signal peptide, in accordance with the [-3, -1] rule (von Heijne, 1986), four extracellular Ig-type domains carrying three potential asparagine (N)-linked glycosylation sites (Figure 9A), a 16 amino acid transmembrane and a 106 amino acid cytoplasmic domain with three putative consensus Src-homology 2 (SH2)-binding domains (Unkeless and Jin, 1997) (Figure 10B). These (SH2)-binding domains exhibit features of both ITAM (Immune-receptor Tyrosine-based Activation Motif -D/EX₇D/EX₂YXXL/IX₆₋₈YXXL/I; where X denotes non-conserved residues) (Reth, 1989) and ITIM motifs (Immune-receptor Tyrosine-based Inhibition Motif - S/V/L/IYXXL/V where *X* denotes non-conserved residues) (Unkeless and Jin, 1997). As shown in Figure 10B, the first two SH2-binding domains are spaced 8 aminoacids apart, consistent with the consensus ITAM motif. Diverging from the consensus, the glutamate residue (E) is positioned four rather than two aminoacids before the first tyrosine (Y) (Figure 10B), and the +3 position relative to tyrosine (Y) is occupied by valine (V) rather than leucine (L) or isoleucine (I) (Cambier, 1995). All three domains conform to the ITIM consensus and each is encoded by a separate exon, as is the case for ITIM. Thus their arrangement may give rise to three ITIM or possibly to one ITAM and one ITIM. The overall structure of this protein suggests that it represents a novel transmembrane receptor of the Ig superfamily and it was therefore name IRTA1 (Immune Receptor Translocation Associated gene 1).

The second cDNA shares homology to *IRTA1* (68% nucleotide identity for the length of the *IRTA1* message encoding its extracellular domain) and was named *IRTA2.* The *IRTA2* locus is more complex than *IRTA1* and is transcribed into three major mRNA isoforms (*IRTA2a, IRTA2b, IRTA2c)* of different molecular weight (2.8, 4.7 and 5.4 kb respectively), each with its own unique 3' untranslated region (Figure 9B). In addition, a 0.6 kb transcript (Figure 12A) arises from the usage of an early polyadenylation signal at nucleotide 536 of *IRTA2.* The three predicted IRTA2 protein isoforms encoded by these transcripts share a common aminoacid sequence until residue 560, featuring a common signal peptide and six extracellular Ig-type domains (Figure 9B). IRTA2a encodes for a 759 aa secreted glycoprotein with eight Ig-type domains followed by 13 unique, predominantly polar aminoacids at its C-terminus. IRTA2b diverges from IRTA2a at amino acid residue 560, and extends for a short stretch of 32 additional residues, whose hydrophobicity is compatible with its docking to the plasma membrane via a GPI-anchor (Ferguson and Williams, 1988). IRTA2c is the longest isoform whose sequence deviates from IRTA2a at aminoacid 746. It encodes a 977 aa type I transmembrane glycoprotein with nine extracellular Ig-type domains, harboring eight potential N-linked glycosylation sites, a 23 aminoacid transmembrane and a 104 aminoacid cytoplasmic domain with three consensus SH2-binding motifs (Figure 10B). Each of the SH2-binding sites in IRTA2c agrees with the ITIM consensus (Figure 10B) and is encoded by a separate exon. These features suggest that IRTA2c is a novel transmembrane receptor of the Ig superfamily with secreted and GPI-linked isoforms.

### Homology between the IRTA proteins and Immunoglobulin Superfamily Receptors

Amino acid alignment of the entire extracellular domains of the IRTA1 and IRTA2 proteins to each other and to other Ig superfamily members revealed a remarkable homology between them (47% identity and 51% similarity) and a lower, but striking homology to the Fc gamma receptor family of proteins. This homology was stronger in the aminoacid positions conserved among the different classes of Fc receptors. Among Fc receptors, the high affinity IgG receptor FCGRI (CD64) shared the highest levels of homology with the first three Ig-domains of IRTA1 and IRTA2 (37% identity and 50% similarity) throughout its entire extracellular portion (Figure 10A). Lower levels of homology were observed between the IRTA proteins and the extracellular domains of other cell surface molecules, including human platelet endothelial cell adhesion molecule (PECAM1), B-lymphocyte cell adhesion molecule (CD22) and Biliary Glycoprotein 1 (BGP1) (22-25% identity, 38-41% homology).

No homology is apparent between the IRTAs and members of the Fc receptor family in their cytoplasmic domains. In contrast, significant aminoacid homology is present between IRTA1 and PECAM1 (31% aminoacid identity and 45% homology), IRTA2c and BGP1 (30% identity, 35% homology) and IRTA2c and PECAM1 (28% identity, 50% homology) (Figure 10B). These homologies suggest employment of similar downstream signaling pathways by these different proteins.

### IRTA1 and IRTA2 are normally expressed in specific subpopulations of B cells

The normal expression pattern of the *IRTA1* and *IRTA2* mRNAs was first analyzed by Northern blot hybridization of RNA derived from different normal human tissues and from human cell lines representing different hematopoietic lineages and stages of B-cell development.

*IRTA1* expression was detected at a very low level in human spleen and lymph node RNA (Figure 11A, left panel) and was undetectable in all other human tissues analyzed, including fetal liver, bone marrow, lung, placenta, small intestine, kidney, liver, colon, skeletal muscle, heart and brain (data not shown). Among B cell lines, *IRTA1* expression was absent in cell lines representing pre-B and germinal center B-cells, plasma cells and cells of erythroid, T-cell and myeloid origin (data not shown, see Materials and Methods). Expression was detectable at very low levels only in EBV-immortalized lymphoblastoid cell lines (LCL), which represent a subpopulation (immunoblasts) positioned downstream of germinal center B cells in B-cell differentiation. However, expression was induced in estrogen-deprived ER/EB cells which, being immortalized by a recombinant EBV genome in which the EBNA2 gene is fused to the estrogen receptor, proliferate in the presence of estrogen while they arrest in the G₀/G₁ phase upon estrogen deprivation (Kempkes et al., 1995). *IRTA1* expression was barely detectable in these cells in the presence of estrogen, but was induced (10-fold) upon their G₀/G₁ arrest following estrogen withdrawal (Figure 11A, right panel). Taken together, these results suggest that *IRTA1* is expressed in a lymphoid subpopulation present in spleen and lymph nodes and presumably represented by resting B cells.

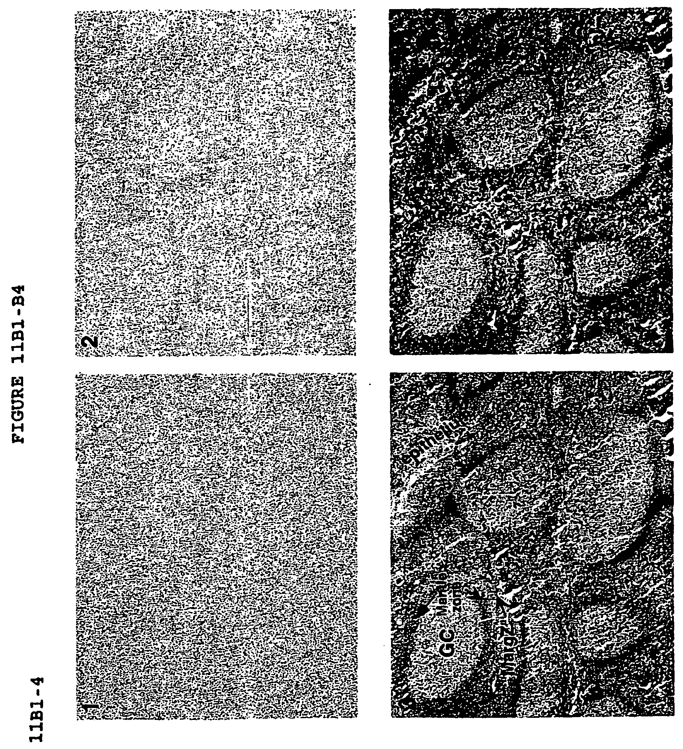

To further investigate the phenotype and tissue distribution of the cells expressing *IRTA1,* we performed *in situ* hybridization on human tonsillar tissue using a *IRTA1* antisense cDNA probe (Figure 11B). Serial sections were processed for *in situ* hybridization with a control sense cDNA probe (Panel # 1 in Figure 11B), an antisense cDNA probe (Panel # 2) and hematoxylin and eosin (H&E) staining (Panel # 3) to outline the architecture of the lymphoid tissue. The *IRTA1* hybridization signal was excluded from the germinal center and the mantle zone of the follicles and was characteristically concentrated in the perifollicular zone with infiltration in the intraepithelial region (Figures 11B-2, 11B-4). In this region, only B cells were positive as documented by staining with B cell specific markers (IgD, not shown), and by immnunohistochemical analysis with anti-IRTA1 and anti-B (CD20, PAX5), anti-T (CD3), and anti-monocyte (CD68) antibodies (not shown; G. Cattoretti et al., manuscript in preparation). This perifollicular area is the "marginal zone" equivalent of the tonsil, representing a functionally distinct B-cell compartment that contains mostly memory B-cells and monocytoid B-cells (de Wolf-Peeters et al., 1997). Together with the Northern blot analysis of normal tissues and cell lines, these results indicate that *IRTA1* is expressed in a subpopulation of resting mature B-cells topographically located in the perifollicular and intraepithelial region, sites rich in memory B cells.

In the case of *IRTA2,* Northern blot analysis detected all alternatively spliced species in human lymph node, spleen, bone marrow and small intestine mRNA, with relative preponderance of the *IRTA2a* isoform (Figure 12A, left panel). Among the hematopoietic cell lines of lymphoid and non-lymphoid origin tested, *IRTA2* expression was restricted to B-cell lines with an immunoblastic, post-germinal center phenotype (Figure 12A, right panel). Similarly to *IRTA1,* it was absent from cell lines derived from pre-B cells, germinal center centroblasts, plasma cells, T-cells, erythroid cells and myeloid cells (Figure 12A, right panel).

*In situ* hybridization analysis of human tonsillar tissue, using the *IRTA2c* cDNA as a probe, was consistent with the results of the Northern blot analysis. The *IRTA2* mRNA was largely excluded from the mantle zone of the germinal center, with the exception of a few positive cells (Figures 12B-2, 12B4). Within the germinal center, the dark zone, represented by centroblasts, appeared negative for *IRTA2,* while the light zone, rich in centrocytes, was strongly positive (Figures 12B-2, 12B-4). Finally, *IRTA2* mRNA was detected in the "marginal zone" equivalent region outside germinal center follicles and in the intraepithelial and interfollicular regions of the tonsil. This pattern is consistent with specificity of *IRTA2* for centrocytes and post-germinal center B cells. Comparing their expression patterns, we conclude that both are specific for mature B cells, but IRTA2 has a broader pattern of expression that includes centrocytes and interfollicular B cells, while *IRTA1* is restricted to marginal zone B cells, most likely memory cells.

### Genomic organization of the IRTA1 and IRTA2 genes

To understand the consequences of 1q21 abnormalities on *IRTA1* and *IRTA2* gene structure and expression, we first determined the organization of their genomic loci. The *IRTA1* gene contains 11 exons with a total genomic size of 24.5 kb (Figure 13). The *IRTA2* locus was found to span a genomic region of approximately 40 kb (Figure 13). The three *IRTA2* alternatively spliced products share their first 8 exons, at which point *IRTA2b* does not utilize the next splicing site, and terminates by entering its 3'UTR region. *IRTA2a* and 2c isoforms splice into exon 9, with *IRTA2a* entering into its 3'UTR after exon 11 and *IRTA2c* splicing into exon 12 and extending until exon 18 (Figure 13).

Based on sequencing data, we determined that the *IRTA1* and *IRTA2* genes are located 21 kb distant from each other, juxtaposed in the same transcriptional orientation (Figure 13) that extends from the telomere (5') towards the centromere (3'). At the 1q21 locus, they are tightly linked to each other as well as to three additional genes we recently cloned through their homology to the *IRTAs* (I.M, manuscript in preparation). All five genes are contiguous, covering a -300 kb region at 1q21. This region is located at the interval between previously reported 1q21 breakpoints. Based on the distance between genomic clones harboring the respective genes on the Whitehead Institute Radiation Hybrid map, the *IRTA1-2* locus is estimated to lie approximately 0.8 Mb away from the *MUC1* locus towards the telomere (N.P, unpublished data; Dyomin et al., 2000; Gilles et al., 2000) and less than or equal to 7 Mb away from the *FCGRIIB* locus towards the centromere (N.P, unpublished data).

### The t(1;14)(q21;q32) translocation generates an IRTA1/Ca₁ fusion protein in the FR4 myeloma cell line

Comparative restriction and nucleotide sequence analysis of germline versus rearranged sequences from the *Cα*₁ and *IRTA1* loci showed that the translocation had fused sequences within intron 2 of the *IRTA1* gene to the intronic sequences between the *CH3* and the transmembrane exon of Ca₁ in the same transcriptional orientation (Figure 14A). This suggested that, if *IRTA1* sequences were expressed in the translocated locus, the intact donor site at the 3' border of the *IRTA1* exon and the intact acceptor site at the 5' of Ca₁ could be used to generate a fusion *IRTA1*/*Ca₁* mRNA, and possibly a IRTA1/Ca₁ fusion protein.

In order to test this prediction, we analyzed *IRTA1* mRNA expression in FR4 by Northern blot analysis using an *IRTA1* cDNA probe derived from exon 1 (Figure 14A). This probe detected a 0.8 kb message in FR4 that was absent from other B-cell lines, and was shorter than the normal 2.5 kb message detectable in ER/EB cells (Figure 14B). We cloned this transcript by RT-PCR of FR4 mRNA using primers derived from sequences at the 5' border of *IRTA1* exon 1 and the 3' border of the *C*α cytoplasmic exon (Figure 14A). An RT-PCR product was obtained from FR4, but not from the DAKIKI cell line expressing wild-type surface IgA, or other cell lines lacking a t(1;14) translocation (data not shown). Direct sequencing analysis of the PCR product indicated that splicing had precisely linked *IRTA1* and *Ca*₁ at canonical splicing sites and determined that the fusion transcript was 820 bp long.

Analysis of the predicted protein product indicated that the *IRTA1*/*Cα*₁ splicing had resulted in a fusion between the IRTA1 signal peptide and first two extracellular aminoacids, with the 32-amino acid long extracellular spacer, transmembrane domain and cytoplasmic tail of the membrane IgA₁ (mIgA₁) receptor (Figure 14C). To assay for the expression of this fusion protein in FR4 protein extracts, we used an antibody directed against extracellular aminoacid residues specific for the transmembrane isoform of Cα₁ (Yu et al., 1990) for immunoprecipitation, followed by Western blotting. Our results demonstrated that FR4 cells, but not a control cell line (DAKIKI) expressing wild-type surface IgA, express a 9.8kDa protein consistent with the predicted size of IRTA1/Cα₁ fusion protein (Figure 14D). These results show that the translocated allele encodes a fusion protein, composed of the signal peptide and first two extracellular residues of IRTA1 (17 aminoacids) fused to the Cα₁ encoded transmembrane and cytoplasmic domains (71 aminoacids). In contrast to *IRTA1*/*Ca*₁ overexpression on der(14), no expression was detected in FR4 for the reciprocal *Ca₁* /*IRTA1* transcript or for the intact *IRTA2* gene on der(1).

With the exception of FR4, *IRTA1* mRNA expression was not detected in any other myeloma or lymphoma cell line, regardless of the status of its chromosomal band 1q21 (data not shown). Thus, the IRTA1/Ca fusion represents a rare event in 1q21 aberrations.

### Frequent deregulation of IRTA2 expression in cell lines carrying 1q21 abnormalities

In order to establish the physical relationship between other 1q21 breakpoints and the *IRTA1*/*2* locus, we performed FISH analysis with the PAC 49A16 on our panel of BL and MM cell lines. Among ten BL cell lines analyzed, seven with dup(1)(q21q32) and three with 1q21 translocations (AS283A, BL104, BL136), we detected three signals corresponding to the *IRTA1*/*IRTA2* locus in seven of the former and two of the latter, consistent with dup(1)(q21q32) in the first case and dup(1)(q21q32) followed by a translocation breakpoint at 1q21 in the second. (Table 1). FISH analysis of AS283A and BL136, using probes spanning the *IRTA* locus and with neighboring genomic clones, placed the breakpoint of the derivative chromosomes outside the *IRTA* locus in both cell lines, at a distance of >800 kb towards the centromere in AS283A and >800 kb towards the telomere in BL136 (N.P, unpublished results). Consistent with this finding, analysis of 30 cases of MM primary tumors by interphase FISH with the 300-kb YAC 23GC4 (Figure 13), showed that 15 cases (50% of total analyzed) had more than two interphase FISH signals (data not shown), while double color FISH with two PAC clones flanking the YAC centromeric and telomeric borders detected no split of these two probes in any of the cases. These results indicate that, with the exception of FR4, the breakpoints of 1q21 aberrations in BL or MM are not within or in close proximity to the genomic region defined by *IRTA1* and *IRTA2.* However, the consistent outcome of either dup(1)(q21q32) (see Table 1) or dup(1)(q21q32) followed by unbalanced translocations (AS283A, BL136, XG2, XG7 in Table 1) is partial trisomy or tetrasomy of the region of 1q21 containing the IRTA genes.

**Table 1. Summary of karyotypic and FISH data on IRTA1/IRTA2 locus**

| Tumour type | Cytogenetics | PAC 49A16 | Copy number of IRTA locus by FISH | *IRTA2* mRNA expression |
|---|---|---|---|---|
| Burkitt Lymphoma | | | | |
| **AS283A** | der(4) t (1;4)(q21;q35) | der(4), normal 1 | 3 | ++++++ |
| **MC116** | dup1q21 | dup1q21 | 3 | +++ |
| **CA46** | dup1q21 | duplq21 | 3 | +++ |
| **PA682** | dup1q21 | duplq21 | 3 | ++ |
| **BrgIgA** | dup1q21 | dup1q21 | 3 | ++ |
| **BL32** | dup1q21 | dup1q21 | 3 | - |
| **BL92** | dup1q21 | dup1q21 | 3 | ++ |
| **BL103** | invdup1q21 | dup1q21 | 3 | + |
| **BL104** | t(1;3)(q21;p25) | der(1) | 2 | + |
| **BL136** | der(1)(qpter1q21::q21) | der(1) | 3 | ++ |

| Multiple Myeloma | | | | |
|---|---|---|---|---|
| **XG2** | der(1) t(1;?)(q21;?) | der(1), normal 1 | 3 | ++++ |
| | der 19 t(1;19)(q12;?) | der(19) | | |
| **XG7** | der(9) t (1;9)(q12;?) | der(9) | 4 | - |
| | der(19) t (1;19)(q12;?) | der(19) | | |
| | der(1)t(1;?)(q21;?)x2 | der(1)x2 | | |

We then investigated whether these aberrations had an effect on *IRTA2* mRNA expression. To this end, we used a cDNA probe corresponding to the *IRTA2* 5' untranslated region to screen a Northern blot with a panel of B-NHL and MM cell lines lacking or displaying 1q21 chromosomal abnormalities. The results show that most (ten out of twelve) BL lines with normal 1q21 chromosomes essentially lack *IRTA2* expression, consistent with the fact that BL derive from GC centroblasts which normally lack *IRTA2* expression (Figure 15A, left panel). In contrast, most BL lines carrying 1q21 abnormalities (ten out of twelve) clearly display *IRTA2* mRNA upregulation (Figure 15A, right panel), ranging from 2 to 50 fold over baseline levels detected in BL with normal 1q21. Among myeloma cell lines, *IRTA2* was overexpressed in one out of three lines displaying 1q21 abnormalities (XG2), while it was expressed in none out of seven with normal 1q21 (Figure 15B).

These results show a strong correlation between the presence of 1q21 chromosomal aberrations and deregulation of *IRTA2* mRNA expression in BL and suggest that trisomies of the *IRTA2* locus may deregulate its expression in this lymphoma subtype (see Discussion).

### Discussion

Efforts described herein to identify genes involved in chromosomal aberrations affecting band 1q21 in Multiple Myeloma and B cell lymphoma, led to the discovery of *IRTA1* and *IRTA2,* two founding members of a novel subfamily of related receptors within the immunoreceptor family; full length nucleic acid sequences encoding IRTA1 and IRTA2 proteins are provided herein, as are the amino acid sequences of the encoded IRTA1 and IRTA2 proteins. Subsequently three additional genes of members of this subfamily of related receptors were isolated, *IRTA3, IRTA4,* and *IRTA5,* the full length nucleic acid sequences of which are provided herein, as are the amino acid sequences of the encoded IRTA3, IRTA4, and *IRTA5* proteins. These results have implications for the normal biology of B cells as well as for the role of 1q21 aberrations in lymphomagenesis.

### IRTA1 and IRTA2 are founding members of a new subfamily within the Ig superfamily

Several features shared between the two IRTA genes and their encoded proteins suggest that they form a new subfamily within the immunoreceptor superfamily. First, they share a higher degree of homology with each other in their extracellular domains than with other superfamily members both in their mRNA (68% identity) and protein (47% identity) sequence. Second, they share homology in their cytoplasmic domains, marked by the presence of ITAM-like and ITIM signaling motifs in the context of homologous aminoacid sequences. Third, IRTA1 and IRTA2 belong to a larger subfamily of five genes displaying higher intrafamily homology and tight clustering within a -300 kb region at 1q21 (I.M. et al., manuscript in preparation). Their genomic organization suggests that a common ancestral gene may have given rise to this subfamily, by a process of duplication and sequence divergence, similar to the mechanism proposed for the Fc receptor family (Qiu et al., 1990).

In their extracellular domain, the *IRTA* proteins are closely related to the Fc receptor subfamily based on the high degree of aminoacid homology shared especially with the high affinity FCGRI receptor (37-45% aminoacid identity). A common evolutionary origin with Fc receptors is also suggested by the position of the *IRTA* family locus in the interval between the *FCGRI* locus on 1q21 and the *FCERI* and *FCGRII-III* loci on 1q21-q23. Finally, the *IRTA* and *FCR* genes share a similar exon/intron organization of the gene portion that encodes their signal peptide, in particular the two 5' leader exons with the sequences encoding the signal peptidase site located within the second 21-bp exon.

Based on their cytoplasmic ITIM-like motifs, the IRTAproteins can be considered members of the Inhibitory Receptor Superfamily (IRS), a group of receptors that block activation of many cell types in the immune system (Lanier, 1998). Such members include FCGRIIB and CD22 in the human (DeLisser et al., 1994) and PIR-B in the mouse (Kubagawa et al., 1997). Analogous to IRS members, the ITIM of IRTA1 and IRTA2 are encoded by individual exons. A feature, that many IRS members share is the existence of corresponding activating receptor isoforms whose cytoplasmic domains are devoid of ITIM (reviewed in Ravetch and Lanier, 1998). It is possible that the secreted isoform of IRTA2, which lacks ITIM-like motifs, fulfills an analogous role by counteracting the effect of the transmembrane isoform.

Significant homology in the sequence and overall organization of their extracellular portion is shared among the IRTA1 and IRTA2 proteins and the Cell Adhesion Molecule (CAM) subfamily members PECAM1, CD22 and BGP1. In addition, the ability of IRTA2 to generate three protein isoforms with distinct subcellular localization (a transmembrane, a GPI-linked or a secreted protein) by differential splicing is shared by *NCAM,* another member of the CAM subfamily (Dickson et al., 1987; Gower et al., 1988). Thus, the IRTA family is also related to the CAM family, as has been previously suggested for a member of the Fc receptor family (murine FCGRII) because of its homology to PECAM1 (CAM, IRS family) (Daeron, 1991; Newman et al., 1990; Stockinger et al., 1990).

In conclusion, the IRTA family may represent an intersection among the Fc, IRS and CAM families, combining features from all three. Accordingly, IRTA proteins may have a role in the regulation of signal transduction during an immune response (like Fc receptors), intercellular communication (like members of the IRS and CAM families) and cell migration (like CAM family members) (DeLisser et al., 1994; Ravetch and Lanier, 2000). Initial experiments indicate that IRTA1 can weakly bind heat aggregated IgA, while IRTA2c can specifically bind heat aggregated human serum IgG (with higher affinity for IgG₁ and IgG₂), but not monomeric human IgG, IgA, IgM and IgE (data not shown). These initial data lend support to a functional relationship between the IRTA and the Fc receptor families, but do not exclude functions dependent on other ligands for the IRTA proteins.

### Differential pattern of expression of IRTA genes in mature B cells

The *IRTA* genes display a specific pattern of expression in various normal B cell compartments. *IRTA1* is topographically restricted to B cells within the perifollicular region, which was originally named marginal zone in the spleen, but is also detectable in most lymphoid organs (de Wolf-Peeters et al., 1997). The *in situ* hybridization data presented here have been confirmed by immunohistochemical analysis using anti-IRTA1 antibodies which show that the IRTA1 protein is selectively expressed in marginal zone B cells, and, among NHL, in marginal zone lymphoma, the tumors deriving from these cells (G. Cattoretti et al., manuscript in preparation). On the other hand, *IRTA2* has a broader pattern of expression that includes GC centrocytes, as well as a broad spectrum of perifollicular cells, which may include immunoblasts and memory cells. Initial data suggest that the pattern of expression of *IRTA3* is analogous to *IRTA2,* while *IRTA4* and *IRTA5* are selectively expressed in mantle zone B cells (I. Miller et al., manuscript in preparation), the pre-GC compartment of mature B cells (MacLennan, I. C., 1994). This topographic restriction of IRTA gene expression in lymphoid organs suggests that the IRTA molecules may play a role in the migration or activity of various B cell subpopulations in specific functional B cell compartments. In addition, IRTA expression should be useful for the differential diagnosis of NHL subtypes deriving from various B cell compartments, particularly IRTA1 in the diagnosis of marginal zone lymphoma.

### IRTA1 locus and 1q21 abnormalities in MM

In the FR4 cell line, the consequence of the t(1;14) translocation is the formation of an *IRTA1*/*Cα*₁ fusion gene. Despite the fact that this gene is driven by the *IRTA1* promoter region, which is normally silent in plasma cells, its expression is high in FR4, presumably due to the influence of the *Cα₁* 3' LCR, which is retained downstream of the *Cα₁* locus. The fusion gene encodes a IRTA1/Cα₁ fusion protein which contains only the signal peptide and first two amino acids of IRTA1 linked to the surface IgA receptor. The latter has been almost completely deprived of its extracellular domain, but retains all its transmembrane and intracellular domains. This structure indicates that the IRTA1/Cα₁ fusion protein, though probably unable to bind any ligand, may retain the potential for dimerization and signaling. In particular, the membrane (m) IgA-derived extracellular portion contains a cysteine residue, which can be involved in disulphide bonds between two α-chains or between a-chains and associated proteins, such as the auxilliary surface receptor CD19 (Leduc et al., 1997). The fusion protein also carries the intact, 14 amino acid mIgA cytoplasmic domain, which is highly conserved in evolution (Reth, 1992) and may play an essential role in the proliferation, survival and differentiation of mature B-cells, analogous to the role of mIgG and mIgE (Kaisho et al., 1997). Thus, the emergence of the IRTA1/Ca₁ protein in FR4 may have provided the cells with a proliferative and survival advantage during tumor development through ligand (antigen)-independent activation of the BCR pathway. This fusion event however, appears to be rare in B-cell malignancy, since so far we were able to detect it only in FR4 cells.

### IRTA2 locus and 1q21 abnormalities in MM and BL

Abnormal expression of *IRTA2* is a frequent consequence of 1q21 abnormalities. Although this gene is not expressed normally either in centroblasts, the presumed normal counterparts of BL (Kuppers et al., 1999), or in BL with normal 1q21, its levels are upregulated on average by 10-fold in BL cell lines with 1q21 abnormalities. This deregulation appears to be specific for *IRTA2* since all the other 4 *IRTA* genes present within 300 kb on 1q21 are either not expressed in BL (*IRTA1*), or their pattern of expression does not correlate with the presence of 1q21 abnormalities (*IRTA3*, 4, 5, not shown). The mechanism by which this deregulation occurs is difficult to ascertain in the absence of structural lesions within or adjacent to the *IRTA2* gene. Since the heterogeneous aberrations that affect 1q21 all cause an excess copy number of the *IRTA* locus, it is possible that this may lead to regulatory disturbances, as is the case for low level amplification of *BCL2* in FL lacking (14;18) translocations (Monni et al., 1997), *REL* in diffuse large cell lymphoma (Houldsworth et al., 1996; Rao et al., 1998) and deregulation of Cyclin D1 in some MM cases with trisomy 11 (Pruneri et al., 2000). On the other hand, 1q21 abnormalities, including translocations and duplications, change the genomic context of the *IRTA* locus and may lead to deregulation of *IRTA2* by distant cis-acting enhancer chromatin organizing elements acting on its promoter as is the case for *MYC* in endemic BL (Pelicci et al., 1986) and MM (Shou et al., 2000) and for *CCND1* in mantle cell lymphoma (Bosch et al., 1994; Swerdlow et al., 1995) and MM (Pruneri et al., 2000).

The biological consequences of deregulated *IRTA2* expression are difficult to predict at this stage. The observation that IRTA2 has homology with CAM adhesion receptors, together with its specific distribution in the light zone of the GC suggest that its ectopic expression in centroblasts may cause a disruption in the GC development and architecture. On the other hand, our initial observations that IRTA2 can bind IgG immune complexes comparably to bona fide Fc receptors suggest that its inappropriate expression may perturb the dynamics of cell surface regulation of B cell immunological responses, possibly leading to clonal expansion. Deregulated expression of *FCGR2B* as a result of the t(1;14) (q21;q32) in follicular lymphoma has been proposed to contribute to lymphomagenesis in this tumor type (Callanan et al., 2000), by a mechanism involving escape by tumor cells of anti-tumor immune surveillance through their Fc binding and inactivation of tumor specific IgG. Similar evasion mechanisms have been observed in cells infected by Fc-encoding herpesvisures (Dubin et al., 1991). The role of IRTA2 deregulation needs to be tested in "gain of function" transgenic mice constitutively expressing IRTA2 in the GC.

### Preferences for Second Series of Experiments

Anand, R., Riley, J. H., Butler, R., Smith, J. C., and Markham, A. F. (1990). A 3.5 genome equivalent multi access YAC library: construction, characterisation, screening and storage. Nucleic Acids Res 18, 1951-6.
Avet-Loiseau, H., Andree-Ashley, L. E., Moore, D., 2nd, Mellerin, M. P., Feusner, J., Bataille, R., and Pallavicini, M. G. (1997). Molecular cytogenetic abnormalities in multiple myeloma and plasma cell leukemia measured using comparative genomic hybridization. Genes Chromosomes Cancer 19, 124-33.
Bakhshi, A., Jensen, J. P., Goldman, P., Wright, J. J., McBride, O. W., Epstein, A. L., and Korsmeyer, S. J. (1985). Cloning the chromosomal breakpoint of t(14;18) human lymphomas: clustering around JH on chromosome 14 and near a transcriptional unit on 18. Cell 41, 899-906.
Berger, R., Bernheim, A. (1985). Cytogenetics of Burkitt's lymphoma-leukaemia: a review. IARC Sci Publ 60, 65-80.
Bergsagel, P. L., Chesi, M., Nardini, E., Brents, L. A., Kirby, S. L., and Kuehl, W. M. (1996). Promiscuous translocations into immunoglobulin heavy chain switch regions in multiple myeloma. Proc Natl Acad Sci U S A 93, 13931-6.
Bosch, F., Jares, P., Campo, E., Lopez-Guillermo, A., Piris, M. A., Villamor, N., Tassies, D., Jaffe, E. S., Montserrat, E., Rozman, C.et al. (1994). PRAD-1/cyclin D1 gene overexpression in chronic lymphoproliferative disorders: a highly specific marker of mantle cell lymphoma. Blood 84, 2726-32.
Callanan, M. B., Le Baccon, P., Mossuz, P., Duley, S., Bastard, C., Hamoudi, R., Dyer, M. J., Klobeck, G., Rimokh, R., Sotto, J. J., and Leroux, D. (2000). The IgG Fc receptor, FcgammaRIIB, is a target for deregulation by chromosomal translocation in malignant lymphoma. Proc Natl Acad Sci U S A 97, 309-14.
Cambier, J. C. (1995). Antigen and Fc receptor signaling. The awesome power of the immunoreceptor tyrosine-based activation motif (ITAM). J Immunol 155, 3281-5.
Chesi, M., Bergsagel, P. L., Brents, L. A., Smith, C. M., Gerhard, D. S., and Kuehl, W. M. (1996). Dysregulation of cyclin D1 by translocation into an IgH gamma switch region in two multiple myeloma cell lines [see comments]. Blood 88, 674-81.
Chesi, M., Bergsagel, P. L., Shonukan, O. O., Martelli, M. L., Brents, L. A., Chen, T., Schrock, E., Ried, T., and Kuehl, W. M. (1998). Frequent dysregulation of the c-maf proto-oncogene at 16q23 by translocation to an Ig locus in multiple myeloma. Blood 91, 4457-63.
Chesi, M., Nardini, E., Brents, L. A., Schrock, E., Ried, T., Kuehl, W. M., and Bergsagel, P. L. (1997). Frequent translocation t(4;14)(p16.3;q32.3) in multiple myeloma is associated with increased expression and activating mutations of fibroblast growth factor receptor 3. Nat Genet 16, 260-4.
Church, D. M., Stotler, C. J., Rutter, J. L., Murrell, J. R., Trofatter, J. A., and Buckler, A. J. (1994). Isolation of genes from complex sources of mammalian genomic DNA using exon amplification. Nat Genet 6, 98-105.
Cigudosa, J. C., Parsa, N. Z., Louie, D. C., Filippa, D. A., Jhanwar, S. C., Johansson, B., Mitelman, F., and Chaganti, R. S. (1999). Cytogenetic analysis of 363 consecutively ascertained diffuse large B- cell lymphomas. Genes Chromosomes Cancer 25, 123-33.
Daeron, M. (1991). Fc receptors, or the elective affinities of adhesion molecules. Immunol Lett 27, 175-81.
Dalla-Favera, R., Bregni, M., Erikson, J., Patterson, D., Gallo, R.C., and Croce, C.M.: The human c-myc onc-gene is located on the region of chromosome 8 which is translocated in Burkitt lymphoma cells. Proc. Nat. Acad. Sci. USA 79:7824-7827, 1982.
Dalla-Favera, R., Martinotti, S., Gallo, R. C., Erikson, J., and Croce, C. M. (1983). Translocation and rearrangements of the c-myc oncogene locus in human undifferentiated B-cell lymphomas. Science 219, 963-7.
de Wolf-Peeters, C., Pittaluga, S., Dierlamm, J., Wlodarska, I., and Van Den Berghe, H. (1997). Marginal zone B-cell lymphomas including mucosa-associated lymphoid tissue type lymphoma (MALT), monocytoid B-cell lymphoma and splenic marginal zone cell lymphoma and their reflation to the reactive marginal zone. Leuk Lymphoma 26, 467-78.
DeLisser, H. M., Newman, P. J., and Albelda, S. M. (1994). Molecular and functional aspects of PECAM-1/CD31. Immunol Today 15, 490-5.
Dickson, G., Gower, H. J., Barton, C. H., Prentice, H. M., Elsom, V. L., Moore, S. E., Cox, R. D., Quinn, C., Putt, W., and Walsh, F. S. (1987). Human muscle neural cell adhesion molecule (N-CAM): identification of a muscle-specific sequence in the extracellular domain. Cell 50, 1119-30.
Dierlamm, J., Pittaluga, S., Wlodarska, I., Stul, M., Thomas, J., Boogaerts, M., Michaux, L., Driessen, A., Mecucci, C., Cassiman, J. J., and et al. (1996). Marginal zone B-cell lymphomas of different sites share similar cytogenetic and morphologic features [see comments]. Blood 87, 299-307.
Dracopoli, C. N., Haines, J. L., Korf, B. R., Morton, C. C., Seidman, C. E., Seidman, J.G., Smith, D. R. (1997). Current Protocols in Human Genetics (New York: Wiley & Sons)
Dubin, G., Socolof, E., Frank, I., Friedman, H. M. (1991). Herpes simplex virus type 1 Fc receptor protects infected cells from antibody-dependent cellular cytotoxicity. Journal of Virology 65, 7046-50.
Dyomin, V. G., Palanisamy, N., Lloyd, K. O., Dyomina, K., Jhanwar, S. C., Houldsworth, J., and Chaganti, R. S. (2000). MUC1 is activated in a B-cell lymphoma by the t(1;14)(q21;q32) translocation and is rearranged and amplified in B-cell lymphoma subsets. Blood 95, 2666-71.
Dyomin, V.G., Rao, P.H., Dalla-Favera, R., Chaganti, R.S.K. (1997). BCL8, a novel gene involved in translocations affecting band 15q11-13 in diffuse large-cell lymphoma. Proc Natl Acad Sci USA 94, 5728-32.
Eton, O., Scheinberg, D. A., and Houghton, A. N. (1989). Establishment and characterization of two human myeloma cell lines secreting kappa light chains. Leukemia 3, 729-35.
Ferguson, M. A., and Williams, A. F. (1988). Cell-surface anchoring of proteins via glycosyl-phosphatidylinositol structures. Annu Rev Biochem 57, 285-320.
Frank, D., Mendelsohn, C. L., Ciccone, E., Svensson, K., Ohlsson, R., and Tycko, B. (1999). A novel pleckstrin homology-related gene family defined by Ipl/Tssc3, TDAG51, and Tihl: tissue-specific expression, chromosomal location, and parental imprinting. Mamm Genome 10, 1150-1159.
Gaidano, G., and Dalla-Favera, R. (1997). Molecular Biology of Lymphomas. In: Principles and Practice of Oncology, Fifth Ed, DeVita, VT, Hellman, S., Rosenberg SA (eds) JB Lippincott Co (publ.), 2131-2145.
Gilles, F., Goy, A., Remache, Y., Shue, P., and Zelenetz, A. D. (2000). MUC1 dysregulation as the consequence of a tt(1;14)(q21;q32) translocation in an extranodal lymphoma. Blood 95, 2930-2936.
Gower, H. J., Barton, C. H., Elsom, V. L., Thompson, J., Moore, S. E., Dickson, G., and Walsh, F. S. (1988). Alternative splicing generates a secreted form of N-CAM in muscle and brain. Cell 55, 955-64.
Hamilton, M. S., Ball, J., Bromidge, E., Lowe, J., and Franklin, I. M. (1990). Characterization of new IgG lambda myeloma plasma cell line (EJM): a further tool in the investigation of the biology of multiple myeloma. Br J Haematol 75, 378-84.
Houldsworth, J., Mathew, S., Rao, P. H., Dyomina, K., Louie, D. C., Parsa, N., Offit, K., Chaganti, R. S. (1996). REL proto-oncogene is frequently amplified in extranodal diffuse large cell lymphoma. Blood 87, 25-9.
Iida, S., Rao, P. H., Butler, M., Corradini, P., Boccadoro, M., Klein, B., Chaganti, R. S., and Dalla-Favera, R. (1997). Deregulation of MUM1/IRF4 by chromosomal translocation in multiple myeloma. Nat Genet 17, 226-30.
Jackson, N., Lowe, J., Ball, J., Bromidge, E., Ling, N. R., Larkins, S., Griffith, M. J., and Franklin, I. M. (1989). Two new IgA1-kappa plasma cell leukaemia cell lines (JJN-1 & JJN-2) which proliferate in response to B cell stimulatory factor 2. Clin Exp Immunol 75, 93-9.
Jernberg, H., Zech, L., and Nilsson, K. (1987). Cytogenetic studies on human myeloma cell lines. Int J Cancer 40, 811-7.
Juliusson, G., Oscier, D. G., Fitchett, M., Ross, F. M., Stockdill, G., Mackie, M. J., Parker, A. C., Castoldi, G. L., Guneo, A., Knuutila, S., and et al. (1990). Prognostic subgroups in B-cell chronic lymphocytic leukemia defined by specific chromosomal abnormalities. N Engl J Med 323, 720-4.
Kaisho, T., Schwenk, F., and Rajewsky, K. (1997). The roles of gamma 1 heavy chain membrane expression and cytoplasmic tail in IgGl responses. Science 276, 412-5.
Kempkes, B., Spitkovsky, D., Jansen-Durr, P., Ellwart, J. W., Kremmer, E., Delecluse, H. J., Rottenberger, C., Bornkamm, G. W., and Hammerschmidt, W. (1995). B-cell proliferation and induction of early G1-regulating proteins by Epstein-Barr virus mutants conditional for EBNA2. Embo J 14, 88-96.
Kornblau, S. M., Goodacre, A., Cabanillas, F. (1991). Chromosomal abnormalities in adult non-endemic Burkitt's lymphoma and leukemia: 22 new reports and a review of 148 cases from the literature. Hematol Oncol 9, 63-78.
Kubagawa, H., Burrows, P. D., and Cooper, M. D. (1997). A novel pair of immunoglobulin-like receptors expressed by B cells and myeloid cells [see comments]. Proc Natl Acad Sci U S A 94, 5261-6.
Kuppers, R., Klein, U., Hansmann, M. L., and Rajewsky, K. (1999). Cellular origin of human B-cell lymphomas. N Engl J Med 341, 1520-9.
Lanier, L. L. (1998). NK cell receptors. Annu Rev Immunol 16, 359-93.
Leduc, I., Drouet, M., Bodinier, M. C., Helal, A., and Cogne, M. (1997). Membrane isoforms of human immunoglobulins of the A1 and A2 isotypes: structural and functional study. Immunology 90, 330-6.
MacLennan, I. C. (1994). Germinal Centers. Annu Rev Immunol 12, 117-39.
Magrath, I. T., Pizzo, P. A., Whang-Peng, J., Douglass, E. C., Alabaster, O., Gerber, P., Freeman, C. B., and Novikovs, L. (1980). Characterization of lymphoma-derived cell lines: comparison of cell lines positive and negative for Epstein-Barr virus nuclear antigen. I. Physical, cytogenetic, and growth characteristics. J Natl Cancer Inst 64, 465-76.
Monni, O., Joensuu, H., Franssila, K., Klefstrom, J., Alitalo, K., and Knuutila, S. (1997). BCL2 overexpression associated with chromosomal amplification in diffuse large B-cell lymphoma. Blood 90, 1168-74.
Neri, A., Barriga, F., Knowles, D. M., Magrath, I. T., and Dalla-Favera, R. (1988). Different regions of the immunoglobulin heavy-chain locus are involved in chromosomal translocations in distinct pathogenetic forms of Burkitt lymphoma. Proc Natl Acad Sci U S A 85, 2748-52.
Neri, A., Chang, C. C., Lombardi, L., Salina, M., Corradini, P., Maiolo, A. T., Chaganti, R. S., and Dalla-Favera, R. (1991). B cell lymphoma-associated chromosomal translocation involves candidate oncogene lyt-10, homologous to NF-kappa B p50. Cell 67, 1075-87.
Newman, P. J., Berndt, M. C., Gorski, J., White, G. C. d., Lyman, S., Paddock, C., and Muller, W. A. (1990). PECAM-1 (CD31) cloning and relation to adhesion molecules of the immunoglobulin gene superfamily. Science 247, 1219-22.
Offit, K., Louie, D. C., Parsa, N. Z., Roy, P., Leung, D., Lo Coco, F., Zelenetz, A., Dalla-Favera, R., Chaganti, R. S. (1995). BCL6 gene rearrangement and other cytogenetic abnormalities in diffuse large cell lymphoma. Leuk Lymphoma 20, 85-9.
Pelicci, P. G., Knowles, D. M. d., Magrath, I., and Dalla-Favera, R. (1986). Chromosomal breakpoints and structural alterations of the c-myc locus differ in endemic and sporadic forms of Burkitt lymphoma. Proc Natl Acad Sci U S A 83, 2984-8.
Polito, P., Cilia, A. M., Gloghini, A., Cozzi, M., Perin, T., De Paoli, P., Gaidano, G., and Carbone, A. (1995). High frequency of EBV association with non-random abnormalities of the chromosome region 1q21-25 in AIDS-related Burkitt's lymphoma-derived cell lines. Int J Cancer 61, 370-4.
Pruneri, G., Fabris, S., Baldini, L., Carboni, N., Zagano, S., Colombi, M. A., Ciceri, G., Lombardi, L., Rocchi, M., Buffa, R., Maiolo, A. T., Neri, A. (2000). Immunohistochemical analysis of cyclin D1 shows deregulated expression in multiple myeloma with the t(11;14). Am J Pathol 156, 1505-13.
Qiu, W. Q., de Bruin, D., Brownstein, B. H., Pearse, R., Ravetch, J. V. (1990). Organization of the human and mouse low-affinity Fc gamma R genes: duplication and recombination. Science 248, 732-5.
Rao, P. H., Houldsworth, J., Dyomina, K., Parsa, N. Z., Cigudosa, J. C., Louie, D. C., Popplewell, L., Offit, K., Jhanwar, S. C., and Chaganti, R. S. (1998). Chromosomal and gene amplification in diffuse large B-cell lymphoma. Blood 92, 234-40.
Rao, P. H., Murty, V. V., Gaidano, G., Hauptschein, R., Dalla-Favera, R., and Chaganti, R. S. (1993). Subregional localization of 20 single-copy loci to chromosome 6 by fluorescence in situ hybridization. Genomics 16, 426-30.
Ravetch, J. V., and Lanier, L. L. (2000). Immune inhibitory receptors [In Process Citation]. Science 290, 84-9.
Reth, M. (1989). Antigen receptor tail clue [letter]. Nature 338, 383-4.
Reth, M. (1992). Antigen receptors on B lymphocytes. Annu Rev Immunol 10, 97-121.
Richelda, R., Ronchetti, D., Baldini, L., Cro, L., Viggiano, L., Marzella, R., Rocchi, M., Otsuki, T., Lombardi, L., Maiolo, A. T., Neri, A. (1997). A novel chromosomal translocation t(4; 14)(p16.3; q32) in multiple myeloma involves the fibroblast growth-factor receptor 3 gene [see comments]. Blood 90, 4062-70.
Riley, J., Butler, R., Ogilvie, D., Finniear, R., Jenner, D., Powell, S., Anand, R., Smith, J. C., and Markham, A. F. (1990). A novel, rapid method for the isolation of terminal sequences from yeast artificial chromosome (YAC) clones. Nucleic Acids Res 18, 2887-90.
Ronchetti, D., Finelli, P., Richelda, R., Baldini, L., Rocchi, M., Viggiano, L., Cuneo, A., Bogni, S., Fabris, S., Lombardi, L., Maiolo, A. T., and Neri, A. (1999). Molecular analysis of 11q13 breakpoints in multiple myeloma. Blood 93, 1330-7.
Rosenberg, C. L., Wong, E., Petty, E. M., Bale, A. E., Tsujimoto, Y., Harris, N. L., and Arnold, A. (1991). PRAD1, a candidate BCL1 oncogene: mapping and expression in centrocytic lymphoma. Proc Natl Acad Sci U S A 88, 9638-42.
Sawyer, J. R., Tricot, G., Mattox, S., Jagannath, S., and Barlogie, B. (1998). Jumping translocations of chromosome 1q in multiple myeloma: evidence for a mechanism involving decondensation of pericentromeric heterochromatin. Blood 91, 1732-41.
Sawyer, J. R., Waldron, J. A., Jagannath, S., Barlogie, B. (1995). Cytogenetic findings in 200 patients with multiple myeloma. Cancer Genet Cytogenet 82, 41-9.
Shou, Y., Martelli, M. L., Gabrea, A., Qi, Y., Brents, L. A., Roschke, A., Dewald, G., Kirsch, I. R., Bergsagel, P. L., and Kuehl, W. M. (2000). Diverse karyotypic abnormalities of the c-myc locus associated with c- myc dysregulation and tumor progression in multiple myeloma. Proc Natl Acad Sci U S A 97, 228-33.
Stockinger, H., Gadd, S. J., Eher, R., Majdic, O., Schreiber, W., Kasinrerk, W., Strass, B., Schnabl, E., and Knapp, W. (1990). Molecular characterization and functional analysis of the leukocyte surface protein CD31. J Immunol 145, 3889-97.
Swerdlow, S. H., Yang, W. I., Zukerberg, L. R., Harris, N. L., Arnold, A., Williams, M. E. (1995). Expression of cyclin D1 protein in centrocytic/mantle cell lymphomas with and without rearrangement of the BCL1/cyclin D1 gene. Hum Pathol 26, 999-1004.
Tagawa, S., Doi, S., Taniwaki, M., Abe, T., Kanayama, Y., Nojima, J., Matsubara, K., and Kitani, T. (1990). Amylase-producing plasmacytoma cell lines, AD3 and FR4, with der(14)t(8;14) and dic(8)t(1;8) established from ascites. Leukemia 4, 600-5.
Taub, R., Kirsch, I., Morton, C., Lenoir, G., Swan, D., Tronick, S., Aaronson, S., Leder, P. (1982). Translocation of the c-myc gene into the immunoglobulin heavy chain locus in human Burkitt lymphoma and murine plasmacytoma cells. Proc Natl Acad Sci U S A 79, 7837-41.
Thompson, J. D., Higgins, D. G., Gibson, T. J. CLUSTAL W: improving the sensitivity of progressive multiple sequence alignment through sequence weighting, position-specific gap penalties and weight matrix choice. Nucleic Acids Res 22, 4673-80.
Tusnady, G. E., Simon, I. (1998). Principles governing amino acid composition of integral membrane proteins: application to topology prediction. J Mol Bio 283, 489-506.
Unkeless, J. C., and Jin, J. (1997). Inhibitory receptors, ITIM sequences and phosphatases. Curr Opin Immunol 9, 338-43.
von Heijne, G. (1986). A new method for predicting signal sequence cleavage sites. Nucleic Acids Res 14, 4683-90.
Whang-Peng, J., Knutsen, T., Jaffe, E. S., Steinberg, S. M., Raffeld, M., Zhao, W. P., Duffey, P., Condron, K., Yano, T., Longo, D. L. (1995). Sequential analysis of 43 patients with non-Hodgkin's lymphoma: clinical correlations with cytogenetic, histologic, immunophenotyping, and molecular studies. Blood 85, 203-16.
Willis, T. G., Zalcberg, I. R., Coignet, L. J., Wlodarska, I., Stul, M., Jadayel, D. M., Bastard, C., Treleaven, J. G., Catovsky, D., Silva, M. L., and Dyer, M. J. (1998). Molecular cloning of translocation tt(1;14)(q21;q32) defines a novel gene (BCL9) at chromosome 1q21. Blood 91, 1873-81.
Ye, B. H., Lista, F., Lo Coco, F., Knowles, D. M., Offit, K., Chaganti, R. S., and Dalla-Favera, R. (1993). Alterations of a zinc finger-encoding gene, BCL-6, in diffuse large- cell lymphoma. Science 262, 747-50.
Yu, L. M., Peng, C., Starnes, S. M., Liou, R. S., and Chang, T. W. (1990). Two isoforms of human membrane-bound alpha Ig resulting from alternative mRNA splicing in the membrane segment. J Immunol 145, 3932-6.
Zhang, X. G., Gaillard, J. P., Robillard, N., Lu, Z. Y., Gu, Z. J., Jourdan, M., Boiron, J. M., Bataille, R., and Klein, B. (1994). Reproducible obtaining of human myeloma cell lines as a model for tumor stem cell study in human multiple myeloma. Blood 83, 3654-63.

### Third Series of Experiments

Chromosome 1q21 is frequently altered by translocations and duplications in several types of B cell malignancy, including multiple myeloma, Burkitt lymphoma, marginal zone lymphomas, and follicular lymphoma. To identify the genes involved in these aberrations, cloned was the chromosomal breakpoint of a t(1;14)(q21;q32) in the myeloma cell line FR4. A 300kb region spanning the breakpoint contains at least five highly related adjacent genes which encode surface receptor molecules that are members of the immunoglobulin gene superfamily, and thus called IRTA (Immunoglobulin Receptor Translocation Associated). The various IRTA molecules have from three to nine extracellular immunoglobulin superfamily domains and are related to the Fc gamma receptors. They have transmembrane and cytoplasmic domains containing ITIM-like and ITAM-like (ITRA-1, IRTA-3, IRTA-4) signaling motifs. In situ hybridization experiments show that all IRTA genes are expresed in the B cell lineage with distinct developmental stage-specific patterns: IRTA-1 is expressed in a marginal B cell pattern. IRTA-2 is expressed in centrocytes and more mature B cells. As a result of the translocation in FR4, IRTA-1 is broken and produces a fusion transcript with the immunoglobulin locus. The IRTA-2 gene, normally silent in centroblasts, is overexpressed in multiple myeloma and in Burkitt lymphoma cell lines carrying 1q21 abnormalities. The data here suggests that IRTA genes are novel B cell regulatory molecules that may also have a role in lymphomagenesis.

## Claims

1. A nucleic acid molecule which encodes immunoglobulin receptor, Immunoglobulin superfamily Receptor Translocation Associated, IRTA2 protein comprising the amino acid sequence set forth in Figures 18-B-1 to 18-B-3.

2. A nucleic acid molecule of claim 1, wherein the nucleic acid molecule is DNA.

3. The DNA molecule of claim 1, wherein the DNA is cDNA.

4. The DNA molecule of claim 1, wherein the DNA is genomic DNA.

5. The nucleic acid molecule of claim 1, wherein the nucleic acid molecule is an RNA molecule.

6. The DNA molecule of claim 2, wherein the DNA molecule is cDNA having the nucleotide sequence set forth in Figure 18-B-1 to 18-B-3.

7. The nucleic acid molecule of claim 1 operatively linked to a promoter of DNA transcription.

8. The nucleic acid molecule of claim 7, wherein the promoter comprises a bacterial, yeast, insect, plant or mammalian promoter.

9. A vector comprising the nucleic acid molecule of claim 7.

10. The vector of claim 9, wherein the vector is a plasmid.

11. A host cell comprising the vector of claim 10.

12. The host cell of claim 11, wherein the cell is selected from the group consisting of a bacterial cell, a plant cell, an insect cell and a mammalian cell.

13. A method for detecting a B cell malignancy or a type of B cell malignancy in a sample from a subject wherein the B cell malignancy comprises a 1q21 chromosomal rearrangement which comprises:
a) contacting a sample of RNA obtained from the subject with a nucleic acid molecule of at least 15 contiguous nucleotides capable of specifically hybridizing with a unique sequence included within the sequence of an isolated RNA encoding human IRTA2 protein under conditions permitting hybridization of the RNA of step (a) with the nucleic acid molecule capable of specifically hybridizing with a unique sequence included with the sequence of an isolated RNA encoding human IRTA2 protein, wherein the nucleic acid molecule is labeled with a detectable marker; and
b) detecting any hybridization in step (a), wherein detection of hybridization indicates presence of B cell malignancy or a type of B cell malignancy in the sample
wherein the human IRTA2 protein comprises the amino acid sequence set forth in Figure 18-B-1 to 18-B-3.

14. The method of claim 13, wherein the detectable marker is a radioactive isotope, enzyme, dye, biotin, a fluorescent label or a chemiluminiescent label.

15. The method of claim 13, wherein the B cell malignancy is selected from the group consisting of B cell lymphoma, multiple myeloma, Burkitt's lymphoma, marginal zone lymphoma, diffuse large cell lymphoma and follicular lymphoma cells.

16. The method of claim 15, wherein the B cell lymphoma is Mucosa-Associated Lymphoid Tissue B cell lymphoma (MALT).

17. The method of claim 15, wherein the B cell lymphoma is non-Hodgkin's lymphoma.

18. An antisense oligonucleotide having a sequence capable of specifically hybridizing to an mRNA molecule encoding a human IRTA2 protein so as to prevent overexpression of the mRNA molecule, wherein the IRTA2-b protein comprises the amino acid sequence set forth in Figures 18-B-1 to 18-B-3.

19. An antibody directed to a purified human IRTA2 protein, comprising the amino acid sequence set forth in Figures 18-B-1 to 18-B-3.

20. The antibody of claim 19, wherein the antibody is a monoclonal antibody or a polyclonal antibody.

21. The antibody of claim 20, wherein the monoclonal antibody is a murine monoclonal antibody or a humanized monoclonal antibody.

22. The antibody of claim 19, wherein the antibody is conjugated to a therapeutic agent, wherein the therapeutic agent is selected from the group consisting of a radioisotope, a toxin, a toxoid, or a chemotherapeutic agent.

23. A pharmaceutical composition comprising an amount of the antibody of claim 19 effective to bind to cancer cells expressing an IRTA2 protein so as to prevent growth of the cancer cells and a pharmaceutically acceptable carrier.

24. The pharmaceutical composition of claim 23, wherein the cancer cells are selected from the group consisting of B cell lymphoma, a mantle cell lymphoma, multiple myeloma, Burkitt's lymphoma, marginal zone lymphoma, diffuse large cell lymphoma and follicular lymphoma cells.

25. The pharmaceutical composition of claim 24, wherein the B cell lymphoma cells are Mucosa-Associated-Lymphoid Tissue B cell lymphoma (MALT) cells.

26. The pharmaceutical composition of claim 24, wherein the B cell lymphoma cells are non-Hodgkin's lymphoma cells.

27. A pharmaceutical composition comprising an amount of the oligonucleotide of claim 18 effective to prevent overexpression of a human IRTA2 protein and a pharmaceutically acceptable carrier.

28. A method of diagnosing a B cell malignancy which comprises a 1q21 chromosomal rearrangement in a sample from a subject which comprises:
a) contacting a sample obtained from the subject with the antibody of claim 19 capable of specifically binding with a human IRTA2 protein on a cell surface of a cancer cell under conditions permitting binding of the antibody with human IRTA2 protein on the cell surface of the cancer cell, wherein the antibody is labeled with a detectable marker; and
b) detecting any binding in step (a), wherein detecting of binding indicates a diagnosis of B cell malignancy in the sample.

29. The method of claim 28, wherein the B cell malignancy is selected from the group consisting of B cell lymphoma, multiple myeloma, Burkitt's lymphoma, mantle cell lymphoma, marginal zone lymphoma, diffuse large cell lymphoma and follicular lymphoma.

30. The method of claim 29, wherein the B cell lymphoma is Mucosa-Associated-Lymphoid Tissue B cell lymphoma (MALT).

31. The method of claim 29, wherein the B cell lymphoma is non-Hodgkin's lymphoma.

32. Use of an amount of anti-IRTA2 antibody effective to bind to cancer cells expressing a human IRTA2 protein so as to prevent growth of the cancer cells for the preparation of a pharmaceutical composition for treating a subject having a B cell cancer, wherein the human IRTA2 protein comprises the amino acid sequence set forth in Figures 18-B-1 to 18-B-3.

33. The use of claim 32, wherein the anti-IRTA2 antibody is a monoclonal antibody.

34. The use of claim 33, wherein the monoclonal antibody is a murine monoclonal antibody or a humanized monoclonal antibody.

35. The use of claim 32, wherein the anti-IRTA2 antibody is a polyclonal antibody.

36. The use of claim 32, wherein the B cell cancer is selected from the group consisting of B cell lymphoma, multiple myeloma, mantle cell lymphoma, Burkitt's lymphoma, marginal zone lymphoma, diffuse large cell lymphoma and follicular lymphoma.

37. The use of claim 36, wherein the B cell lymphoma is a Mucosa-Associated-Lymphoid Tissue B cell lymphoma (MALT).

38. The use of claim 36, wherein the B cell lymphoma is a non-Hodgkin's lymphoma.

39. Use of an amount of the oligonucleotide of claim 18 effective to prevent overexpression of human IRTA2 protein, so as to arrest cell growth or induce cell death of cancer cells expressing IRTA2 protein(s) for the preparation of a pharmaceutical composition for treating a subject having a B cell cancer.

40. The use of claim 39, wherein the B cell cancer is selected from the group consisting of B cell lymphoma, mantle cell lymphoma, multiple myeloma, Burkitt's lymphoma, marginal zone lymphoma, diffuse large cell lymphoma and follicular lymphoma.

41. The use of claim 40, wherein the B cell lymphoma is Mucosa-Associated-Lymphoid Tissue B cell lymphoma (MALT).

42. The use of claim 40, wherein the B cell lymphoma is non-Hodgkin's lymphoma.

## Patentansprüche

1. Nucleinsäuremolekül, das einen Immunglobulinrezeptor, das Immunglobulin-Superfamilie-Rezeptor-Translokation-assoziierte IRTA2-Protein, codiert, das die in Figuren 18-B-1 bis 18-B-3 gezeigte Aminosäuresequenz umfasst.

2. Nucleinsäuremolekül nach Anspruch 1, wobei das Nucleinsäuremolekül DNA ist.

3. DNA-Molekül nach Anspruch 1, wobei die DNA cDNA ist.

4. DNA-Molekül nach Anspruch 1, wobei die DNA genomische DNA ist.

5. Nucleinsäuremolekül nach Anspruch 1, wobei das Nucleinsäuremolekül ein RNA-Molekül ist.

6. DNA-Molekül nach Anspruch 2, wobei das DNA-Molekül cDNA ist, die die in Figuren 18-B-1 bis 18-B-3 gezeigte Nucleotidsequenz hat.

7. Nucleinsäuremolekül nach Anspruch 1, das mit einem Promoter für DNA-Transkription operativ verknüpft ist.

8. Nucleinsäuremolekül nach Anspruch 7, wobei der Promoter einen Bakterien-, Hefe-, Insekten-, Pflanzen- oder Säuger-Promoter umfasst.

9. Vektor, der das Nucleinsäuremolekül nach Anspruch 7 umfasst.

10. Vektor nach Anspruch 9, wobei der Vektor ein Plasmid ist.

11. Wirtszelle, die den Vektor nach Anspruch 10 umfasst.

12. Wirtszelle nach Anspruch 11, wobei die Zelle ausgewählt ist aus der Gruppe bestehend aus einer bakteriellen Zelle, einer Pflanzenzelle, einer Insektenzelle und einer Säugerzelle.

13. Verfahren zum Nachweisen eines B-Zell-Malignoms oder einer Art des B-Zell-Malignoms in einer Probe eines Individuums, wobei das B-Zell-Malignom ein Rearrangement des Chromosoms 1q21 umfasst, umfassend:
(a) Inkontaktbringen einer vom Individuum erhaltenen RNA-Probe mit einem Nucleinsäuremolekül aus mindestens 15 fortlaufenden Nucleotiden, die in der Lage sind, spezifisch mit einer einmaligen Sequenz zu hybridisieren, die innerhalb der Sequenz einer isolierten RNA eingeschlossen ist, die ein humanes IRTA2-Protein codiert, unter Bedingungen, die die Hybridisierung der RNA aus Schritt (a) mit dem Nucleinsäuremolekül ermöglichen, das in der Lage ist, spezifisch mit einer einmaligen Sequenz zu hybridisieren, die innerhalb der Sequenz einer isolierten RNA eingeschlossen ist, die ein humanes IRTA2-Protein codiert, wobei das Nucleinsäuremolekül mit einem nachweisbaren Marker markiert ist; und
(b) Nachweisen einer Hybridisierung in Schritt (a), wobei der Nachweis der Hybridisierung auf das Vorhandensein eines B-Zell-Malignoms oder einer Art des B-Zell-Malignoms in der Probe hinweist,
wobei das humane IRTA2-Protein die in Figuren 18-B-1 bis 18-B-3 gezeigte Aminosäuresequenz umfasst.

14. Verfahren nach Anspruch 13, wobei der nachweisbare Marker ein radioaktives Isotop, Enzym, Farbstoff, Biotin, ein fluoreszierender Marker oder ein chemilumineszierender Marker ist.

15. Verfahren nach Anspruch 13, wobei das B-Zell-Malignom ausgewählt ist aus der Gruppe bestehend aus B-Zell-Lymphom, multiplem Myelom, Burkitt-Lymphom, Marginalzonen-Lymphom, diffusem großzelligem Lymphom und follikulären Lymphomzellen.

16. Verfahren nach Anspruch 15, wobei das B-Zell-Lymphom B-Zell-Lymphom des Mukosa-assoziierten lymphatischen Gewebes (MALT) ist.

17. Verfahren nach Anspruch 15, wobei das B-Zell-Lymphom Non-Hodgkin-Lymphom ist.

18. Antisense-Oligonucleotid, das eine Sequenz hat, die in der Lage ist, an ein mRNA-Molekül spezifisch zu hybridisieren, das ein humanes IRTA2-Protein codiert, um eine Überexpression des mRNA-Moleküls zu verhindern, wobei das IRTA2-b-Protein die in Figuren 18-B-1 bis 18-B-3 gezeigte Aminosäuresequenz umfasst.

19. Antikörper, der auf ein aufgereinigtes humanes IRTA2-Protein gerichtet ist, das die in Figuren 18-B-1 bis 18-B-3 gezeigte Aminosäuresequenz umfasst.

20. Antikörper nach Anspruch 19, wobei der Antikörper ein monoclonaler Antikörper oder ein polyclonaler Antikörper ist.

21. Antikörper nach Anspruch 20, wobei der monoclonale Antikörper ein muriner monoclonaler Antikörper oder ein humanisierter monoclonaler Antikörper ist.

22. Antikörper nach Anspruch 19, wobei der Antikörper an ein therapeutisches Agens konjugiert ist, wobei das therapeutische Agens ausgewählt ist aus der Gruppe bestehend aus einem Radioisotop, einem Toxin, einem Toxoid oder einem chemotherapeutischen Agens.

23. Arzneimittel, das eine Menge des Antikörpers nach Anspruch 19, die wirksam ist, an Krebszellen zu binden, die ein IRTA2-Protein exprimieren, um das Wachstum der Krebszellen zu verhindern, und einen pharmazeutisch verträglichen Träger umfasst.

24. Arzneimittel nach Anspruch 23, wobei die Krebszellen ausgewählt sind aus der Gruppe bestehend aus B-Zell-Lymphom, einem Mantelzell-Lymphom, multiplem Myelom, Burkitt-Lymphom, Marginalzonen-Lymphom, diffusem großzelligem Lymphom und follikulären Lymphomzellen.

25. Arzneimittel nach Anspruch 24, wobei die B-Zell-Lymphomzellen B-Zell-Lymphomzellen des Mukosa-assoziierten lymphatischen Gewebes (MALT) sind.

26. Arzneimittel nach Anspruch 24, wobei die B-Zell-Lymphomzellen Non-Hodgkin-Lymphomzellen sind.

27. Arzneimittel, das eine Menge des Oligonucleotids nach Anspruch 18, die wirksam ist, Überexpression eines humanen IRTA2-Proteins zu verhindern, und einen pharmazeutisch verträglichen Träger umfasst.

28. Verfahren zum Diagnostizieren eines B-Zell-Malignoms, das ein Rearrangement des Chromosoms 1q21 in einer Probe von einem Individuum umfasst, umfassend:
(a) Inkontaktbringen einer vom Individuum erhaltenen Probe mit dem Antikörper nach Anspruch 19, der in der Lage ist, mit einem humanen IRTA2-Protein auf einer Zelloberfläche einer Krebszelle spezifisch zu binden, unter Bedingungen, die es dem Antikörper ermöglichen, mit dem humanen IRTA2-Protein auf der Zelloberfläche der Krebszelle zu binden, wobei der Antikörper mit einem nachweisbaren Marker markiert ist; und
(b) Nachweisen einer Bindung in Schritt (a), wobei der Nachweis der Bindung auf eine Diagnose eines B-Zell-Malignoms in der Probe hinweist.

29. Verfahren nach Anspruch 28, wobei das B-Zell-Malignom ausgewählt ist aus der Gruppe bestehend aus B-Zell-Lymphom, multiplem Myelom, Burkitt-Lymphom, Mantelzell-Lymphom, Marginalzonen-Lymphom, diffusem großzelligem Lymphom und follikulärem Lymphom.

30. Verfahren nach Anspruch 29, wobei das B-Zell-Lymphom B-Zell-Lymphom des Mukosa-assoziierten lymphatischen Gewebes (MALT) ist.

31. Verfahren nach Anspruch 29, wobei das B-Zell-Lymphom Non-Hodgkin-Lymphom ist.

32. Verwendung einer Menge eines Anti-IRTA2-Antikörpers, die wirksam ist, an Krebszellen zu binden, die ein humanes IRTA2-Protein exprimieren, um Wachstum der Krebszellen zu verhindern, zur Herstellung eines Arzneimittels zur Behandlung eines Individuums, das einen B-Zell-Krebs hat, wobei das humane IRTA2-Protein die in Figuren 18-B-1 bis 18-B-3 gezeigte Aminosäuresequenz umfasst.

33. Verwendung nach Anspruch 32, wobei der Anti-IRTA2-Antikörper ein monoclonaler Antikörper ist.

34. Verwendung nach Anspruch 33, wobei der monoclonale Antikörper ein muriner monoclonaler Antikörper oder ein humanisierter monoclonaler Antikörper ist.

35. Verwendung nach Anspruch 32, wobei der Anti-IRTA2-Antikörper ein polyclonaler Antikörper ist.

36. Verwendung nach Anspruch 32, wobei der B-Zell-Krebs ausgewählt ist aus der Gruppe bestehend aus B-Zell-Lymphom, multiplem Myelom, Mantelzell-Lymphom, Burkitt-Lymphom, Marginalzonen-Lymphom, diffusem großzelligem Lymphom und follikulärem Lymphom.

37. Verwendung nach Anspruch 36, wobei das B-Zell-Lymphom B-Zell-Lymphom des Mukosa-assoziierten lymphatischen Gewebes (MALT) ist.

38. Verwendung nach Anspruch 36, wobei das B-Zell-Lymphom Non-Hodgkin-Lymphom ist.

39. Verwendung einer Menge des Oligonucleotids nach Anspruch 18, die wirksam ist, Überexpression des humanen IRTA2-Proteins zu verhindern, um Zellwachstum von Krebszellen zu arretieren oder Zelltod von Krebszellen zu induzieren, die IRTA2-Protein(e) exprimieren, zur Herstellung eines Arzneimittels zur Behandlung eines Individuums mit B-Zell-Krebs.

40. Verwendung nach Anspruch 39, wobei der B-Zell-Krebs ausgewählt ist aus der Gruppe bestehend aus B-Zell-Lymphom, Mantelzell-Lymphom, multiplem Myelom, Burkitt-Lymphom, Marginalzonen-Lymphom, diffusem großzelligem Lymphom und follikulärem Lymphom.

41. Verwendung nach Anspruch 40, wobei das B-Zell-Lymphom B-Zell-Lymphom des Mukosa-assoziierten lymphatischen Gewebes (MALT) ist.

42. Verwendung nach Anspruch 40, wobei das B-Zell-Lymphom Non-Hodgkin-Lymphom ist.

## Revendications

1. Molécule d'acide nucléique qui code pour une protéine IRTA2, récepteur d'immunoglobuline de la superfamille des immunoglobulines de translocation associée, comprenant la séquence aminoacide présentée aux figures 18-B-1 à 18-B-3.

2. Molécule d'acide nucléique selon la revendication 1, où la molécule d'acide nucléique est un ADN.

3. Molécule d'ADN selon la revendication 1, où l'ADN est un ADNc.

4. Molécule d'ADN selon la revendication 1, où l'ADN est un ADN génomique.

5. Molécule d'acide nucléique selon la revendication 1, où la molécule d'acide nucléique est une molécule de RNA.

6. Molécule d'ADN selon la revendication 2, où la molécule d'ADN est un ADNc ayant la séquence nucléotidique présentée aux figures 18-B-1 à 18-B-3.

7. Molécule d'acide nucléique selon la revendication 1, liée de manière opérationnelle à un promoteur de la transcription d'ADN.

8. Molécule d'acide nucléique selon la revendication 7, où le promoteur comprend un promoteur de bactérie, de levure, d'insecte, de végétal ou de mammifère.

9. Vecteur comprenant la molécule d'acide nucléique selon la revendication 7.

10. Vecteur selon la revendication 9, où le vecteur est un plasmide.

11. Cellule hôte comprenant le vecteur selon la revendication 10.

12. Cellule hôte selon la revendication 11, où la cellule est choisie dans le groupe constitué d'une cellule bactérienne, une cellule végétale, une cellule d'insecte et une cellule de mammifère.

13. Méthode de détection d'une malignité à cellules B ou d'un type de malignité à cellules B dans un échantillon provenant d'un sujet où la malignité à cellules B comprend un réarrangement chromosomique 1q21 qui comprend :
a) la mise en contact d'un échantillon de RNA obtenu du sujet avec une molécule d'acide nucléique d'au moins 15 nucléotides contigus capable de s'hybrider spécifiquement avec une séquence unique incluse dans la séquence d'un RNA isolé codant pour une protéine humaine IRTA2 dans des conditions permettant l'hybridation du RNA de l'étape (a) avec la molécule d'acide nucléique capable de s'hybrider spécifiquement avec une séquence unique incluse avec la séquence d'un RNA isolé codant pour une protéine humaine IRTA2, où la molécule d'acide nucléique est marquée avec un marqueur détectable ; et
b) la détection de toute hybridation dans l'étape (a), où la détection d'hybridation indique la présence d'une malignité à cellules B ou d'un type de malignité à cellules B dans l'échantillon
où la protéine IRTA2 humaine comprend la séquence aminoacide présentée aux figures 18-b-1 à 18-B-3.

14. Méthode selon la revendication 13, où le marqueur détectable est un isotope radioactif, une enzyme, un colorant, une biotine, un marqueur fluorescent ou un marqueur chimioluminescent.

15. Méthode selon la revendication 13, où la malignité à cellules B est choisie dans le groupe constitué du lymphome à cellules B, du myélome multiple, du lymphome de Burkitt, du lymphome de la zone marginale, du lymphome diffus à grandes cellules et de cellules de lymphome folliculaire.

16. Méthode selon la revendication 15, où le lymphome à cellules B est un lymphome à cellules B du tissu lymphoïde associé aux muqueuses (MALT).

17. Méthode selon la revendication 15, où le lymphome à cellules B est un lymphome non-hodgkinien.

18. Oligonucléotide anti-sens ayant une séquence capable de s'hybrider spécifiquement avec une molécule d'ARNm codant pour une protéine IRTA2 humaine de manière à empêcher la surexpression de la molécule d'ARNm, où la protéine IRTA2-b comprend la séquence aminoacide présentée aux figures 18-B-1 à 18-B-3.

19. Anticorps dirigé contre une protéine IRTA2 humaine purifiée, comprenant la séquence aminoacide présentée aux figures 18-B-1 à 18-B-3.

20. Anticorps selon la revendication 19, où l'anticorps est un anticorps monoclonal ou un anticorps polyclonal.

21. Anticorps selon la revendication 20, où l'anticorps monoclonal est un anticorps murin monoclonal ou un anticorps monoclonal humanisé.

22. Anticorps selon la revendication 19, où l'anticorps est conjugué à un agent thérapeutique, où l'agent thérapeutique est choisi dans le groupe constitué d'un radio-isotope, une toxine, un toxoïde, ou un agent chimiothérapeutique.

23. Composition pharmaceutique comprenant une quantité d'anticorps selon la revendication 19 efficace pour se lier à des cellules cancéreuses exprimant une protéine IRTA2 de manière à empêcher la croissance des cellules cancéreuses et un vecteur pharmaceutiquement acceptable.

24. Composition pharmaceutique selon la revendication 23, où les cellules cancéreuses sont choisies dans le groupe constitué du lymphome à cellules B, du lymphome à cellules du manteau, du myélome multiple, du lymphome de Burkitt, du lymphome de la zone marginale, du lymphome diffus à grandes cellules et de cellules de lymphome folliculaire.

25. Composition pharmaceutique selon la revendication 24, où les cellules du lymphome à cellules B sont des cellules de lymphome à cellules B du tissu lymphoïde associé aux muqueuses (MALT).

26. Composition pharmaceutique selon la revendication 24, où les cellules du lymphome à cellules B sont des cellules de lymphome non-hodgkinien.

27. Composition pharmaceutique comprenant une quantité de l'oligonucléotide selon la revendication 18 efficace pour empêcher la surexpression d'une protéine IRTA2 humaine et un vecteur pharmaceutiquement acceptable.

28. Méthode de diagnostic d'une malignité à cellules B comprenant un réarrangement chromosomique 1q21 dans un échantillon provenant d'un sujet qui comprend :
a) la mise en contact d'un échantillon obtenu du sujet avec l'anticorps selon la revendication 19 capable de se lier spécifiquement avec une protéine IRTA2 humaine sur une surface cellulaire d'une cellule cancéreuse dans des conditions permettant la liaison de l'anticorps avec la protéine IRTA2 humaine sur la surface cellulaire de la cellule cancéreuse, où l'anticorps est marqué avec un marqueur détectable ; et
b) la détection de toute liaison dans l'étape (a), où la détection de liaison indique un diagnostic de la malignité à cellules B dans l'échantillon.

29. Méthode selon la revendication 28, où la malignité à cellules B est choisie dans le groupe constitué du lymphome à cellules B, du myélome multiple, du lymphome de Burkitt, du lymphome à cellules du manteau, du lymphome de la zone marginale, du lymphome diffus à grandes cellules et du lymphome folliculaire.

30. Méthode selon la revendication 29, où le lymphoma à cellules B est un lymphome à cellules B du tissu lymphoïde associé aux muqueuses (MALT).

31. Méthode selon la revendication 29, où le lymphome à cellules B est un lymphome non-hodgkinien.

32. Utilisation d'une quantité d'anticorps anti-IRTA2 efficace pour se lier à des cellules cancéreuses exprimant une protéine IRTA2 de manière à empêcher la croissance des cellules cancéreuses pour la préparation d'une composition pharmaceutique pour le traitement d'un patient ayant un cancer à cellules B, où la protéine IRTA2 humaine comprend la séquence aminoacide présentée aux figures 18-B-1 à 18-B-3.

33. Utilisation selon la revendication 32, où l'anticorps anti-IRTA2 est un anticorps monoclonal.

34. Utilisation selon la revendication 33, où l'anticorps monoclonal est un anticorps monoclonal murin ou un anticorps monoclonal humanisé.

35. Utilisation selon la revendication 32, où l'anticorps anti-IRTA2 est un anticorps polyclonal.

36. Utilisation selon la revendication 32, où le cancer à cellules B est choisi dans le groupe constitué du lymphome à cellules B, du myélome multiple, du lymphome à cellules du manteau, du lymphome de Burkitt, du lymphome de la zone marginale, du lymphome diffus à grandes cellules et du lymphome folliculaire.

37. Utilisation selon la revendication 36, où le lymphome à cellules B est un lymphome à cellules B du tissu lymphoïde associé aux muqueuses (MALT).

38. Utilisation selon la revendication 36, où le lymphome à cellule B est un lymphome non-hodgkinien.

39. Utilisation d'une quantité de l'oligonucléotide selon la revendication 18 efficace pour empêcher la surexpression de la protéine IRTA2 humaine, de façon à arrêter la croissance des cellules ou à induire la mort cellulaire des cellules cancéreuses exprimant la / les protéine(s) IRTA2 pour la préparation d'une composition pharmaceutique pour le traitement d'un sujet ayant un cancer à cellules B.

40. Utilisation selon la revendication 39, où le cancer à cellules B est choisi dans le groupe constitué du lymphome à cellules B, du lymphome à cellules du manteau, du myélome multiple, du lymphome de Burkitt, du lymphome de la zone marginale, du lymphome diffus à grandes cellules et du lymphome folliculaire.

41. Utilisation selon la revendication 40, où le lymphome à cellules B est un lymphome à cellules B du tissu lymphoïde associé aux muqueuses (MALT).

42. Utilisation selon la revendication 40, où le lymphome à cellules B est un lymphome non-hodgkinien.
